Europäisches Patentamt

(19) European Patent Office

Office européen des brevets

(11) EP 0 789 076 A2

# EUROPEAN PATENT APPLICATION

(43) Date of publication:
13.08.1997 Bulletin 1997/33

(21) Application number: 97101767.8

(22) Date of filing: 05.02.1997

(51) Int. Cl.$^6$: **C12N 15/12**, C07K 14/72, C12P 21/02, G01N 33/566, C07K 16/28

(84) Designated Contracting States:
AT BE CH DE DK ES FI FR GB GR IE IT LI LU NL PT SE

(30) Priority: 07.02.1996 JP 21562/96

(71) Applicant:
TAKEDA CHEMICAL INDUSTRIES, LTD.
Chuo-ku, Osaka 541 (JP)

(72) Inventors:
• Hinuma, Shuji
Tsukuba, Ibaraki 305 (JP)
• Sakamoto, Junichi
Toyonaka, Osaka 565 (JP)
• Hosoya, Masaki
Tsuchiura, Ibaraki 300 (JP)

(74) Representative: von Kreisler, Alek, Dipl.-Chem. et al
Patentanwälte,
von Kreisler-Selting-Werner,
Bahnhofsvorplatz 1 (Deichmannhaus)
50667 Köln (DE)

Remarks:
The applicant has subsequently filed a sequence listing and declared, that it includes no new matter.

(54) **G protein coupled receptor proteins, their production and use**

(57) This invention relates to a human amygdaloid nucleus-derived G protein coupled receptor protein or a salt thereof, a partial peptide of the G protein coupled receptor protein, DNA encoding the G protein coupled receptor protein, a method of producing the G protein coupled receptor protein, a method of identifying a ligand to the G protein coupled receptor protein, a method or kit for screening out compounds capable of changing the binding activity of a ligand to the G protein coupled receptor protein, a compound or a salt thereof obtained by the screening method or by using the screening kit, a pharmaceutical composition comprising the compound or a salt thereof, and an antibody against the G protein coupled receptor protein or a partial peptide. The G protein coupled receptor protein and DNA encoding the protein can be used (1) identifying a ligand to the G protein coupled receptor protein of the present invention, (2) acquiring antibodies and antisera, (3) constructing the expression system for the recombinant receptor protein, (4) developing receptor binding assay systems using the expression system, and screening out candidate drug substances, (5) drug designing based on comparison with structurally similar ligands and receptors, (6) construction of probes and PCR primers for gene diagnosis, and (7) gene therapy, among others. In particular, elucidation of the structure and properties of the G protein coupled receptor will result in development of a unique drug acting on these systems.

EP 0 789 076 A2

SEQUENCE LISTING

(1) GENERAL INFORMATION:

    (i) APPLICANT:
        (A) NAME: Takeda Chemical Industries, Ltd.
        (B) STREET: 1-1, Doshomachi 4-chome, Chuo-ku
        (C) CITY: Osaka-shi
        (D) STATE: Osaka
        (E) COUNTRY: Japan
        (F) POSTAL CODE (ZIP): 541

    (ii) TITLE OF INVENTION: G Protein Coupled Receptor Proteins, Their
        Production And Use

    (iii) NUMBER OF SEQUENCES: 19

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)

    (v) CURRENT APPLICATION DATA:
        APPLICATION NUMBER: EP 97101767.8

(2) INFORMATION FOR SEQ ID NO:1:
    (i) SEQUENCE CHARACTERISTICS:
        (A) LENGTH:    423
        (B) TYPE:     Amino acid
        (C) TOPOLOGY:  Linear
    (ii)  MOLECULE TYPE: Peptide
    (iii)  SEQUENCE DESCRIPTION: SEQ ID NO:1:

```
Met Val Pro His Leu Leu Leu Leu Cys Leu Leu Pro Leu Val Arg Ala
1               5                   10                  15
Thr Glu Pro His Glu Gly Arg Ala Asp Glu Gln Ser Ala Glu Ala Ala
            20                  25                  30
Leu Ala Val Pro Asn Ala Ser His Phe Phe Ser Trp Asn Asn Tyr Thr
        35                  40                  45
Phe Ser Asp Trp Gln Asn Phe Val Gly Arg Arg Arg Tyr Gly Ala Glu
    50                  55                  60
Ser Gln Asn Pro Thr Val Lys Ala Leu Leu Ile Val Ala Tyr Ser Phe
65                  70                  75                  80
Ile Ile Val Phe Ser Leu Phe Gly Asn Val Leu Val Cys His Val Ile
            85                  90                  95
Phe Lys Asn Gln Arg Met His Ser Ala Thr Ser Leu Phe Ile Val Asn
            100                 105                 110
Leu Ala Val Ala Asp Ile Met Ile Thr Leu Leu Asn Thr Pro Phe Thr
        115                 120                 125
Leu Val Arg Phe Val Asn Ser Thr Trp Ile Phe Gly Lys Gly Met Cys
    130                 135                 140
His Val Ser Arg Phe Ala Gln Tyr Cys Ser Leu His Val Ser Ala Leu
145                 150                 155                 160
Thr Leu Thr Ala Ile Ala Val Asp Arg His Gln Val Ile Met His Pro
            165                 170                 175
Leu Lys Pro Arg Ile Ser Ile Thr Lys Gly Val Ile Tyr Ile Ala Val
            180                 185                 190
Ile Trp Thr Met Ala Thr Phe Phe Ser Leu Pro His Ala Ile Cys Gln
    195                 200                 205
Lys Leu Phe Thr Phe Lys Tyr Ser Glu Asp Ile Val Arg Ser Leu Cys
    210                 215                 220
Leu Pro Asp Phe Pro Glu Pro Ala Asp Leu Phe Trp Lys Tyr Leu Asp
225                 230                 235                 240
Leu Ala Thr Phe Ile Leu Leu Tyr Ile Leu Pro Leu Leu Ile Ile Ser
            245                 250                 255
```

```
Val Ala Tyr Ala Arg Val Ala Lys Lys Leu Trp Leu Cys Asn Met Ile
            260                 265                 270
Gly Asp Val Thr Thr Glu Gln Tyr Phe Ala Leu Arg Arg Lys Lys Lys
            275                 280                 285
Lys Thr Ile Lys Met Leu Met Leu Val Val Val Leu Phe Ala Leu Cys
    290                 295                 300
Trp Phe Pro Leu Asn Cys Tyr Val Leu Leu Leu Ser Ser Lys Val Ile
305                 310                 315                 320
Arg Thr Asn Asn Ala Leu Tyr Phe Ala Phe His Trp Phe Ala Met Ser
                325                 330                 335
Ser Thr Cys Tyr Asn Pro Phe Ile Tyr Cys Trp Leu Asn Glu Asn Phe
                340                 345                 350
Arg Ile Glu Leu Lys Ala Leu Leu Ser Met Cys Gln Arg Pro Pro Lys
            355                 360                 365
Pro Gln Glu Asp Arg Pro Pro Ser Pro Val Pro Ser Phe Arg Val Ala
            370                 375                 380
Trp Thr Glu Lys Asn Asp Gly Gln Arg Ala Pro Leu Ala Asn Asn Leu
385                 390                 395                 400
Leu Pro Thr Ser Gln Leu Gln Ser Gly Lys Thr Asp Leu Ser Ser Val
                405                 410                 415
Glu Pro Ile Val Thr Met Ser
                420
```

(2) INFORMATION FOR SEQ ID NO:2:
    (i)    SEQUENCE CHARACTERISTICS:
        (A)  LENGTH:    1272
        (B)  TYPE:      Nucleic acid
        (C)  STRANDENESS:    Double
        (C)  TOPOLOGY:  Linear
    (ii)   MOLECULE TYPE: cDNA
    (xi)   FEATURE
        (C)  IDENTIFICATION METHOD: S
    (X)    SEQUENCE DESCRIPTION: SEQ ID NO:2:

```
ATGGTCCCTC ACCTCTTGCT GCTCTGTCTC CTCCCCTTGG TGCGAGCCAC CGAGCCCCAC   60
GAGGGCCGGG CCGACGAGCA GAGCGCGGAG GCGGCCCTGG CCGTGCCCAA TGCCTCGCAC  120
TTCTTCTCTT GGAACAACTA CACCTTCTCC GACTGGCAGA ACTTTGTGGG CAGGAGGCGC  180
TACGGCGCTG AGTCCCAGAA CCCCACGGTG AAAGCCCTGC TCATTGTGGC TTACTCCTTC  240
ATCATTGTCT TCTCACTCTT TGGCAACGTC CTGGTCTGTC ATGTCATCTT CAAGAACCAG  300
CGAATGCACT CGGCCACCAG CCTCTTCATC GTCAACCTGG CAGTTGCCGA CATAATGATC  360
ACGCTGCTCA ACACCCCCTT CACTTTGGTT CGCTTTGTGA ACAGCACATG GATATTTGGG  420
AAGGGCATGT GCCATGTCAG CCGCTTTGCC CAGTACTGCT CACTGCACGT CTCAGCACTG  480
ACACTGACAG CCATTGCGGT GGATCGCCAC CAGGTCATCA TGCACCCCTT GAAACCCCGG  540
ATCTCAATCA CAAAGGGTGT CATCTACATC GCTGTCATCT GGACCATGGC TACGTTCTTT  600
TCACTCCCAC ATGCTATCTG CCAGAAATTA TTTACCTTCA AATACAGTGA GGACATTGTG  660
CGCTCCCTCT GCCTGCCAGA CTTCCCTGAG CCAGCTGACC TCTTCTGGAA GTACCTGGAC  720
TTGGCCACCT TCATCCTGCT CTACATCCTG CCCCTCCTCA TCATCTCTGT GGCCTACGCT  780
CGTGTGGCCA AGAAACTGTG GCTGTGTAAT ATGATTGGCG ATGTGACCAC AGAGCAGTAC  840
TTTGCCCTGC GGCGCAAAAA GAAGAAGACC ATCAAGATGT TGATGCTGGT GGTAGTCCTC  900
TTTGCCCTCT GCTGGTTCCC CCTCAACTGC TACGTCCTCC TCCTGTCCAG CAAGGTCATC  960
CGCACCAACA ATGCCCTCTA CTTTGCCTTC CACTGGTTTG CCATGAGCAG CACCTGCTAT 1020
AACCCCTTCA TATACTGCTG GCTGAACGAG AACTTCAGGA TTGAGCTAAA GGCATTACTG 1080
AGCATGTGTC AAAGACCTCC CAAGCCTCAG GAGGACAGGC CACCCTCCCC AGTTCCTTCC 1140
TTCAGGGTGG CCTGGACAGA GAAGAATGAT GGCCAGAGGG CTCCCCTTGC CAATAACCTC 1200
CTGCCCACCT CCCAACTCCA GTCTGGGAAG ACAGACCTGT CATCTGTGGA ACCCATTGTG 1260
ACGATGAGTT AG                                                     1272
```

(2) INFORMATION FOR SEQ ID NO:3:
    (i)    SEQUENCE CHARACTERISTICS:
        (A)  LENGTH:    70
        (B)  TYPE:      Amino acid
        (C)  TOPOLOGY:  Linear
    (ii)   MOLECULE TYPE: Peptide
    (xi)   SEQUENCE DESCRIPTION: SEQ ID NO:3:

```
Val Cys His Val Ile Phe Lys Asn Gln Arg Met His Ser Ala Thr Ser
  1             5                  10                  15
```

```
Leu Phe Ile Val Asn Leu Ala Val Ala Asp Ile Met Ile Thr Leu Leu
            20                  25                  30
Asn Thr Pro Phe Thr Leu Val Arg Phe Val Asn Ser Thr Trp Ile Phe
        35                  40                  45
Gly Lys Gly Met Cys His Val Ser Arg Phe Ala Gln Tyr Cys Ser Leu
    50                  55                  60
His Val Ser Ala Leu Thr
65                  70
```

(2) INFORMATION FOR SEQ ID NO:4:
    (i)    SEQUENCE CHARACTERISTICS:
        (A) LENGTH:    71
        (B) TYPE:    Amino acid
        (C) TOPOLOGY:  Linear
    (ii)   MOLECULE TYPE: Peptide
    (xi)   SEQUENCE DESCRIPTION: SEQ ID NO:4:

```
Glu Pro Ala Asp Leu Phe Trp Lys Asn Leu Asp Leu Pro Thr Phe Ile
1               5                   10                  15
Leu Leu Asn Ile Leu Pro Leu Leu Ile Ile Ser Val Ala Tyr Val Arg
            20                  25                  30
Val Thr Lys Lys Leu Trp Leu Cys Asn Met Ile Val Asp Val Thr Thr
        35                  40                  45
Glu Gln Tyr Phe Ala Leu Arg Pro Lys Lys Lys Lys Thr Ile Lys Met
    50                  55                  60
Leu Met Leu Val Val Val Leu
65                  70
```

(2) INFORMATION FOR SEQ ID NO:5:
    (i)    SEQUENCE CHARACTERISTICS:
        (A) LENGTH:    210
        (B) TYPE:    Nucleic acid
        (C) STRANDENESS:    Double
        (C) TOPOLOGY:  Linear
    (ii)   MOLECULE TYPE: cDNA
    (xi)   FEATURE
        (C) IDENTIFICATION METHOD: S
    (X)    SEQUENCE DESCRIPTION: SEQ ID NO:5:

```
GTCTGTCATG TCATCTTCAA GAACCAGCGA ATGCACTCGG CCACCAGCCT CTTCATCGTC    60
AACCTGGCAG TTGCCGACAT AATGATCACG CTGCTCAACA CCCCCTTCAC TTTGGTTCGC   120
TTTGTGAACA GCACATGGAT ATTTGGGAAG GGCATGTGCC ATGTCAGCCG CTTTGCCCAG   180
TACTGCTCAC TGCACGTCTC AGCACTGACA                                    210
```

(2) INFORMATION FOR SEQ ID NO:6:
    (i)    SEQUENCE CHARACTERISTICS:
        (A) LENGTH:    213
        (B) TYPE:    Nucleic acid
        (C) STRANDENESS:    Double
        (C) TOPOLOGY:  Linear
    (ii)   MOLECULE TYPE: cDNA
    (xi)   FEATURE
        (C) IDENTIFICATION METHOD: S
    (X)    SEQUENCE DESCRIPTION: SEQ ID NO:6:

```
GAGCCAGCTG ACCTCTTCTG GAAGAACCTG GACTTGCCCA CCTTCATCCT GCTCAACATC    60
CTGCCCCTCC TCATCATCTC TGTGGCCTAC GTTCGTGTGA CCAAGAAACT GTGGCTGTGT   120
AATATGATTG TCGATGTGAC CACAGAGCAG TACTTTGCCC TGCGGCCCAA AAAGAAGAAG   180
ACCATCAAGA TGTTGATGCT GGTGGTAGTC CTC                                213
```

(2) INFORMATION FOR SEQ ID NO:7:
    (i)    SEQUENCE CHARACTERISTICS:
        (A) LENGTH:    25
        (B) TYPE:    Nucleic acid
        (C) STRANDEDNESS:  Single
        (C) TOPOLOGY:  Linear

```
        (ii)   MOLECULE TYPE:          Other nucleic acid Synthetic
                                        DNA
        (xi)   SEQUENCE DESCRIPTION: SEQ ID NO:7:

CGTGGSCMTS STGGGCAACN YCCTG            25

(2) INFORMATION FOR SEQ ID NO:8:
        (i)    SEQUENCE CHARACTERISTICS:
               (A) LENGTH:             27
               (B) TYPE:               Nucleic acid
               (C) STRANDEDNESS:       Single
               (C) TOPOLOGY:           Linear
        (ii)   MOLECULE TYPE:          Other nucleic acid Synthetic
                                        DNA
        (xi)   SEQUENCE DESCRIPTION: SEQ ID NO:8:

GTNGWRRGGC ANCCAGCAGA KGGCAAA         27

(2) INFORMATION FOR SEQ ID NO:9:
        (i)    SEQUENCE CHARACTERISTICS:
               (A) LENGTH:             25
               (B) TYPE:               Nucleic acid
               (C) STRANDEDNESS:       Single
               (C) TOPOLOGY:           Linear
        (ii)   MOLECULE TYPE:          Other nucleic acid Synthetic
                                        DNA
        (xi)   SEQUENCE DESCRIPTION: SEQ ID NO:9:

ATATGGGGAT TGGTGGCGAC GACTC           25

(2) INFORMATION FOR SEQ ID NO:10:
        (i)    SEQUENCE CHARACTERISTICS:
               (A) LENGTH:             25
               (B) TYPE:               Nucleic acid
               (C) STRANDEDNESS:       Single
               (C) TOPOLOGY:           Linear
        (ii)   MOLECULE TYPE:          Other nucleic acid Synthetic
                                        DNA
        (xi)   SEQUENCE DESCRIPTION: SEQ ID NO:10:

CAACATCAGC CGCTACAGTC AACAG           25

(2) INFORMATION FOR SEQ ID NO:11:
        (i)    SEQUENCE CHARACTERISTICS:
               (A) LENGTH:             25
               (B) TYPE:               Nucleic acid
               (C) STRANDEDNESS:       Single
               (C) TOPOLOGY:           Linear
        (ii)   MOLECULE TYPE:          Other nucleic acid Synthetic
                                        DNA
        (xi)   SEQUENCE DESCRIPTION: SEQ ID NO:11:

GCCCGGTTAT TATTATTTTT GACAC           25

(2) INFORMATION FOR SEQ ID NO:12:
        (i)    SEQUENCE CHARACTERISTICS:
               (A) LENGTH:             25
               (B) TYPE:               Nucleic acid
               (C) STRANDEDNESS:       Single
               (C) TOPOLOGY:           Linear
        (ii)   MOLECULE TYPE:          Other nucleic acid Synthetic
                                        DNA
        (xi)   SEQUENCE DESCRIPTION: SEQ ID NO:12:

TTCCTTACGC GAAATACGGG CAGAC           25
```

(2) INFORMATION FOR SEQ ID NO:13:
    (i)   SEQUENCE CHARACTERISTICS:
        (A) LENGTH:         31
        (B) TYPE:           Nucleic acid
        (C) STRANDEDNESS:   Single
        (C) TOPOLOGY:      Linear
    (ii)  MOLECULE TYPE:     Other nucleic acid Synthetic
                           DNA
    (xi)  SEQUENCE DESCRIPTION: SEQ ID NO:13:

CGGCCACCAG CCTCTTCATC GTCAACCTGG C     31

(2) INFORMATION FOR SEQ ID NO:14:
    (i)   SEQUENCE CHARACTERISTICS:
        (A) LENGTH:         30
        (B) TYPE:           Nucleic acid
        (C) STRANDEDNESS:   Single
        (C) TOPOLOGY:      Linear
    (ii)  MOLECULE TYPE:     Other nucleic acid Synthetic
                           DNA
    (xi)  SEQUENCE DESCRIPTION: SEQ ID NO:14:

GGCAACCAGC AGAGGGCAAA GAGGACTACC     30

(2) INFORMATION FOR SEQ ID NO:15:
    (i)   SEQUENCE CHARACTERISTICS:
        (A) LENGTH:         30
        (B) TYPE:           Nucleic acid
        (C) STRANDEDNESS:   Single
        (C) TOPOLOGY:      Linear
    (ii)  MOLECULE TYPE:     Other nucleic acid Synthetic
                           DNA
    (xi)  SEQUENCE DESCRIPTION: SEQ ID NO:15:

TTCATCCTGC TCTACATCCT GCCCCTCCTC     30

(2) INFORMATION FOR SEQ ID NO:16:
    (i)   SEQUENCE CHARACTERISTICS:
        (A) LENGTH:         30
        (B) TYPE:           Nucleic acid
        (C) STRANDEDNESS:   Single
        (C) TOPOLOGY:      Linear
    (ii)  MOLECULE TYPE:     Other nucleic acid Synthetic
                           DNA
    (xi)  SEQUENCE DESCRIPTION: SEQ ID NO:16:

GCCCTGCGGC GCAAAAAGAA GAAGACCATC     30

(2) INFORMATION FOR SEQ ID NO:17:
    (i)   SEQUENCE CHARACTERISTICS:
        (A) LENGTH:         30
        (B) TYPE:           Nucleic acid
        (C) STRANDEDNESS:   Single
        (C) TOPOLOGY:      Linear
    (ii)  MOLECULE TYPE:     Other nucleic acid Synthetic
                           DNA
    (xi)  SEQUENCE DESCRIPTION: SEQ ID NO:17:

GCTGGTGGTA GTCCTCTTTG CCCTCTGCTG     30

(2) INFORMATION FOR SEQ ID NO:18:
    (i)   SEQUENCE CHARACTERISTICS:
        (A) LENGTH:         33
        (B) TYPE:           Nucleic acid
        (C) STRANDEDNESS:   Single
        (C) TOPOLOGY:      Linear

```
    (ii)   MOLECULE TYPE:        Other nucleic acid Synthetic
                                  DNA
    (xi)   SEQUENCE DESCRIPTION: SEQ ID NO:18:

GGAGTCGACC AGGGGAGGGG TGGCTCCTGC AAA       33

(2) INFORMATION FOR SEQ ID NO:19:
    (i)    SEQUENCE CHARACTERISTICS:
           (A) LENGTH:           33
           (B) TYPE:             Nucleic acid
           (C) STRANDEDNESS:     Single
           (C) TOPOLOGY:         Linear
    (ii)   MOLECULE TYPE:        Other nucleic acid Synthetic
                                  DNA
    (xi)   SEQUENCE DESCRIPTION: SEQ ID NO:19:

CCCGTCGACC CCCTCCCACT CCCTCTTCCC AAC       33
```

GCCCGGTTAT TATTATTTTT GACAC     25

(2) INFORMATION FOR SEQ ID NO:12:
   (i)   SEQUENCE CHARACTERISTICS:
      (A) LENGTH:          25
      (B) TYPE:            Nucleic acid
      (C) STRANDEDNESS:    Single
      (C) TOPOLOGY:        Linear
   (ii)  MOLECULE TYPE:      Other nucleic acid
                         Synthetic DNA
   (xi)  SEQUENCE DESCRIPTION: SEQ ID NO:12:

TTCCTTACGC GAAATACGGG CAGAC     25

(2) INFORMATION FOR SEQ ID NO:13:
   (i)   SEQUENCE CHARACTERISTICS:
      (A) LENGTH:          31
      (B) TYPE:            Nucleic acid
      (C) STRANDEDNESS:    Single
      (C) TOPOLOGY:        Linear
   (ii)  MOLECULE TYPE:      Other nucleic acid
                         Synthetic DNA
   (xi)  SEQUENCE DESCRIPTION: SEQ ID NO:13:

CGGCCACCAG CCTCTTCATC GTCAACCTGG C     31

(2) INFORMATION FOR SEQ ID NO:14:
   (i)   SEQUENCE CHARACTERISTICS:
      (A) LENGTH:          30
      (B) TYPE:            Nucleic acid
      (C) STRANDEDNESS:    Single
      (C) TOPOLOGY:        Linear
   (ii)  MOLECULE TYPE:      Other nucleic acid
                         Synthetic DNA
   (xi)  SEQUENCE DESCRIPTION: SEQ ID NO:14:

GGCAACCAGC AGAGGGCAAA GAGGACTACC    30


(2) INFORMATION FOR SEQ ID NO:15:
    (i)   SEQUENCE CHARACTERISTICS:
          (A) LENGTH:           30
          (B) TYPE:             Nucleic acid
          (C) STRANDEDNESS:     Single
          (C) TOPOLOGY:         Linear
    (ii)  MOLECULE TYPE:        Other nucleic acid
                                Synthetic DNA
    (xi)  SEQUENCE DESCRIPTION: SEQ ID NO:15:


TTCATCCTGC TCTACATCCT GCCCCTCCTC    30


(2) INFORMATION FOR SEQ ID NO:16:
    (i)   SEQUENCE CHARACTERISTICS:
          (A) LENGTH:           30
          (B) TYPE:             Nucleic acid
          (C) STRANDEDNESS:     Single
          (C) TOPOLOGY:         Linear
    (ii)  MOLECULE TYPE:        Other nucleic acid
                                Synthetic DNA
    (xi)  SEQUENCE DESCRIPTION: SEQ ID NO:16:


GCCCTGCGGC GCAAAAAGAA GAAGACCATC    30


(2) INFORMATION FOR SEQ ID NO:17:
    (i)   SEQUENCE CHARACTERISTICS:
          (A) LENGTH:           30
          (B) TYPE:             Nucleic acid
          (C) STRANDEDNESS:     Single
          (C) TOPOLOGY:         Linear
    (ii)  MOLECULE TYPE:        Other nucleic acid
                                Synthetic DNA
    (xi)  SEQUENCE DESCRIPTION: SEQ ID NO:17:

```
GCTGGTGGTA GTCCTCTTTG CCCTCTGCTG       30
```

(2) INFORMATION FOR SEQ ID NO:18:

    (i)   SEQUENCE CHARACTERISTICS:

        (A) LENGTH:        33

        (B) TYPE:        Nucleic acid

        (C) STRANDEDNESS:  Single

        (C) TOPOLOGY:    Linear

    (ii) MOLECULE TYPE:    Other nucleic acid

                          Synthetic DNA

    (xi)  SEQUENCE DESCRIPTION: SEQ ID NO:18:

```
GGAGTCGACC AGGGGAGGGG TGGCTCCTGC AAA       33
```

(2) INFORMATION FOR SEQ ID NO:19:

    (i)   SEQUENCE CHARACTERISTICS:

        (A) LENGTH:        33

        (B) TYPE:        Nucleic acid

        (C) STRANDEDNESS:  Single

        (C) TOPOLOGY:    Linear

    (ii) MOLECULE TYPE:    Other nucleic acid

                          Synthetic DNA

    (xi)  SEQUENCE DESCRIPTION: SEQ ID NO:19:

```
CCCGTCGACC CCCTCCCACT CCCTCTTCCC AAC       33
```

## Description

[Field of the Invention]

The present invention relates to a novel human amygdaloid nucleus-derived G protein coupled receptor protein, a DNA encoding said G protein coupled receptor protein, a method of producing said G protein coupled receptor protein, and uses of said protein and DNA.

[Background of the Invention]

Many bioactive substances such as hormones and neurotransmitters modulate biological functions through receptor proteins present on the cell surface or within the cell. The interactions of those bioactive substances (hereinafter sometimes referred to also as ligands in contrast with receptors) with receptor proteins are very specific and thereby determine the target cell or organ, pharmacological action, and intensity and duration thereof, for instance, of each individual bioactive substance. Therefore, identifying substances that interact with receptor proteins is one of the important means of drug development. In particular, novel substances acting on receptor proteins, if discovered, might be expected to have very unique pharmacological activities.

Among various receptor proteins, there is a group of proteins that engage in intracellular signal transduction through activation of guanine nucleotide-binding proteins (hereinafter sometimes referred to briefly as G proteins). The proteins of said group are called G protein coupled receptor proteins. Their structural characteristics are such that they have seven highly hydrophobic domains presumably penetrating the cell membrane (transmembrane domains) and show a very high level of similarity in amino acid sequence with one another. Therefore, even if the substances to which such proteins bind are unknown, the proteins are designated receptor proteins from the structural viewpoint. Therefore, a number of DNAs encoding novel G protein coupled receptor proteins have been isolated by the technique of polymerase chain reaction (hereinafter referred to briefly as PCR) utilizing similarities in sequence among G protein coupled receptor proteins, or hybridization with known receptor cDNAs, or random sequencing of cDNAs, for instance. Generally, receptors structurally close to one another tend to bind to ligands similar in structure to one another. From such viewpoints, for some novel receptor proteins, the ligands to which they bind have so far been identified. For some other novel receptor proteins, however, such substances still remain to be determined despite the expectation that said proteins should bind to ligands similar in structure to known ligands. Furthermore, there are many receptors showing an insufficient similarity for predicting the relevant ligands and such receptors are grouped into the category of orphan receptors. Although the ligands to which they bind are unknown, the expression patterns or localizations of those orphan receptors can be determined by in situ hybridization or Northern blotting. For some orphan receptors for which the physiological functions in which they are involved have already been predicted to a certain extent, it is all the more desired that the ligands to which they bind should be determined.

The glucocorticoid-induced receptor (GIR), one of such orphan receptors, was isolated from the mouse T lymphocytic cell line WEHI-7TG treated with a glucocorticoid and forskolin [Harrigan, M. T. et al., Molecular Endocrinology, 5, 1331-1338 (1991)]. It is well known that treatment of T lymphocytes with a glucocorticoid induces breakdown of the metabolic system of said cells, leading to cell death. On that occasion, expression of specific genes is induced. GIR is one of such genes and is thought to be a receptor involved in the modulation of the immune system. Therefore, searching for ligands to GIR might possibly be further research and development of novel immunomodulators. Recently, the present inventors obtained a novel cDNA fragment very close to the cDNA encoding said receptor from a human brain amygdaloid nucleus-derived cDNA by the PCR method. Based on the identity of the partially analyzed amino acid sequence, it is estimated that this receptor is a human counterpart of the receptor GIR known in mice. The structure of the human counterpart of GIR has so far been quite unknown.

According to recent findings, factors acting on both the immune system and central nervous system, for example interleukin 6, are also known. In view of this, it is anticipated that said orphan receptor and the ligand thereto are playing an important role also in the control of some or other function of the central nervous system. From the viewpoint of drug development, it is desirable to obtain a human counterpart of a receptor protein and use it in developing each drug substance. For instance, a compound may produce quite different effects from one animal species to another and it is not rare that this is caused by species differences of the receptor itself. Therefore, a human counterpart receptor gene is particularly needed in research and development of a drug for human use.

[Summary of the Invention]

The present invention is to provide a novel human amygdaloid nucleus-derived G protein coupled receptor protein, a DNA comprising a DNA encoding said G protein coupled receptor protein, a method of producing said G protein coupled receptor protein, and uses of said protein and DNA.

Although the present inventors previously obtained a cDNA sequence encoding a fragment of human amygdaloid nucleus-derived receptor protein and analyzed that partial nucleotide sequence thereof, it was still necessary to obtain a cDNA having the full-length open reading frame so that they could identify the ligand binding to the receptor protein encoded by said cDNA.

For example, the use of a cDNA having such full-length open reading frame makes it possible to produce said receptor protein with good efficiency. The use of said receptor protein expressed by an appropriate means makes it possible to screen out a ligand to said receptor protein from among compounds occurring in vivo or other natural or nonnatural compounds, using the results of a receptor binding experiment or intracellular second messenger measurement as indices. Furthermore, it becomes possible to screen out compounds capable of changing the ligand-to-receptor protein binding activity.

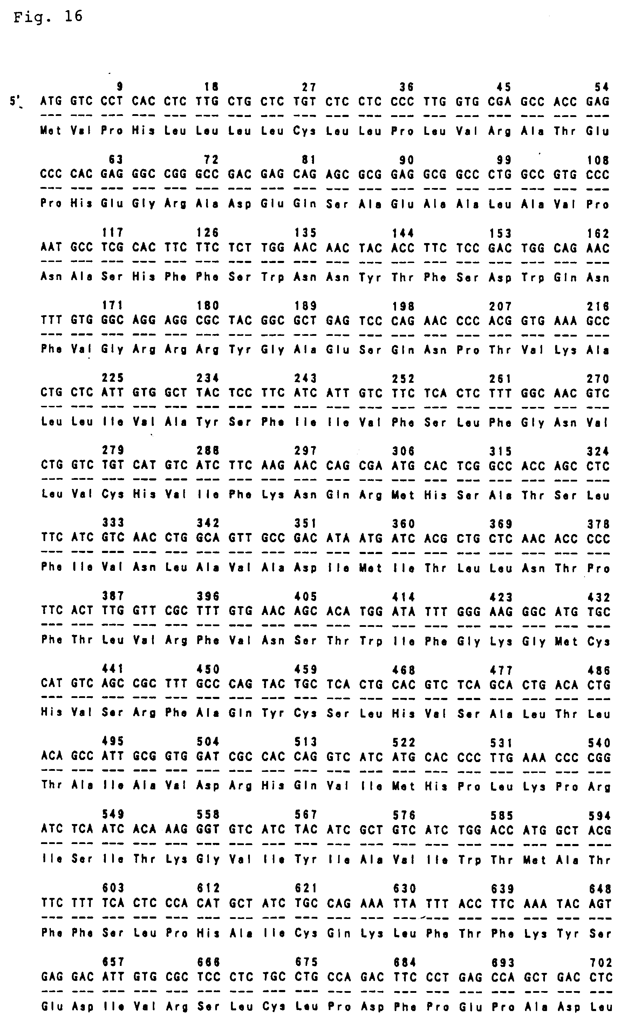

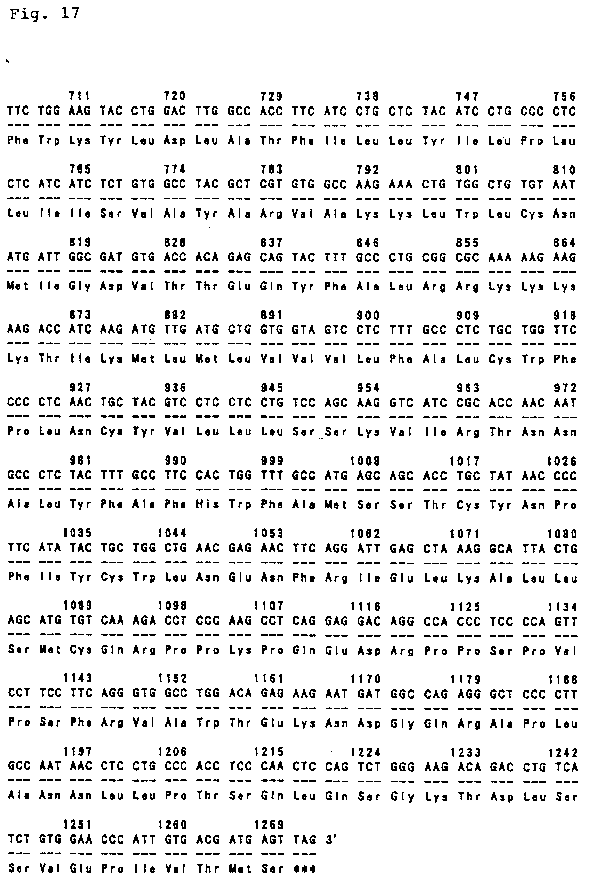

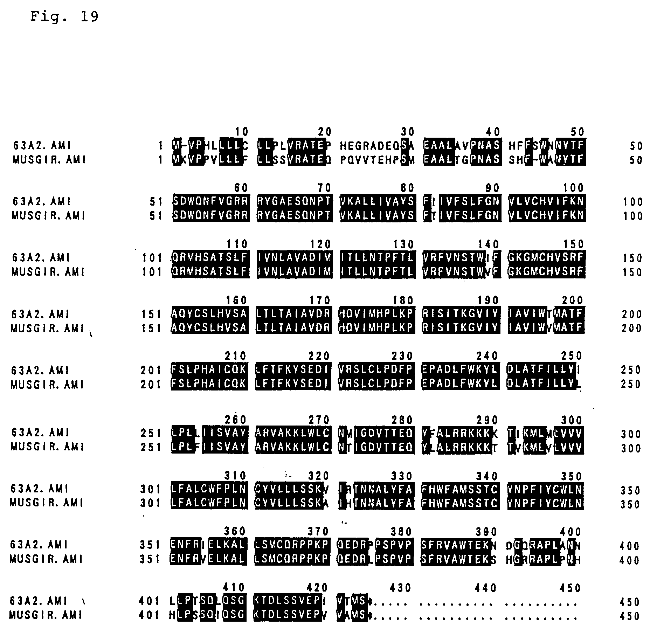

More concretely, to reveal the complete primary structure of the human amygdaloid nucleus-derived receptor obtained by the PCR using DNA primers designed and prepared based on the sequences common to G protein coupled proteins and to search for ligands to the receptor in question following construction of an appropriate expression system, the nucleotide sequences of the 5' and 3' terminal portion of the open reading frame for said receptor were analyzed by RACE (rapid amplification of cDNA ends) and the full-length open reading frame was cloned by the PCR technique. As a result, a cDNA having the nucleotide sequence represented by SEQ ID NO:2 and the whole amino acid sequence encoded thereby was determined (SEQ ID NO:1). In particular, of the sequence shown in Fig. 16 and Fig. 17, the section encoding the first amino acid residue (Met) to the 91st one (Leu) and the section encoding the 301st amino acid residue (Phe) to the translation termination codon were made clear for the first time by the present invention. As for the section encoding the 92nd (Val) to the 300th amino acid residue (Leu), a partial sequence thereof has been determined through the analysis of p63A2 but the whole sequence of this region has not been definitely determined. The present invention has now revealed the precise sequence of this section. A comparison in total amino acid sequence with the murine receptor GIR revealed a very high level of identity, namely 85.8%, although the N-terminal and C-terminal portions show differences in sequence which are presumably due to the difference in species [Fig. 19].

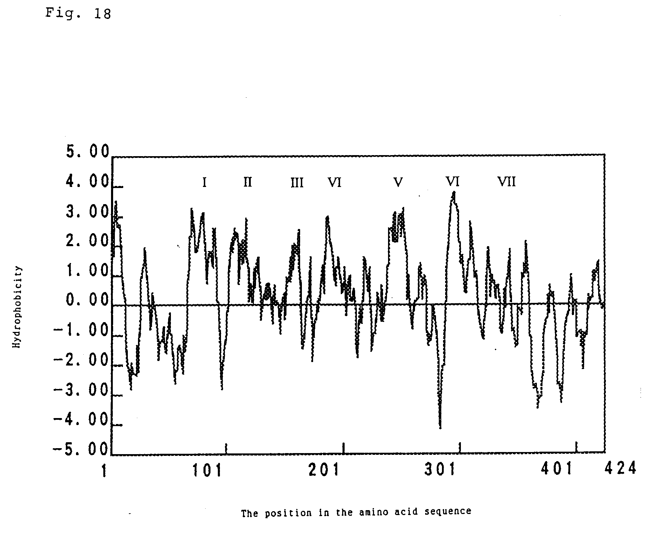

The structure of the full-length open reading frame thus revealed suggests even more storongly that the protein encoded thereby falls under the category of the so-called G protein coupled receptor proteins having seven hydrophobic amino acid clusters [Fig. 18].

Furthermore, the fact that the cDNA encoding said receptor is amplified by PCR from a human hypothalamic cDNA library and a human whole brain poly(A)$^+$ RNA strongly suggests that said receptor is functioning in a relatively wide area of the central nervous system.

When the cDNA of the present invention which has the full-length open reading frame is used, the receptor protein can be produced. When said receptor protein expressed by any appropriate means is used, a ligand to said receptor protein can be screened out from among compounds occurring in vivo or other natural or nonnatural compounds, with the results of a receptor binding experiment or intracellular second messenger measurement as indices. Furthermore, it becomes possible to screen out compounds capable of changing the binding activity of the G protein coupled receptor protein with a ligand, for example the compounds capable of inhibiting the binding activity.

Thus, the present invention provides:

(1) a G protein coupled receptor protein which comprises an amino acid sequence represented by SEQ ID NO:1 or its substantial equivalent thereto, or a salt thereof;

(2) a partial peptide of the G protein coupled receptor protein as described in (1), or a salt thereof. For example, a fragment of at least 16 amino acids;

(3) a DNA which comprises a nucleotide sequence coding for the G protein coupled receptor protein as described in (1) or the partial peptide as described in (2);

(4) the DNA as described in (3), which comprises the nucleotide sequence represented by SEQ ID NO:2;

(5) a recombinant vector comprising the DNA as described in (3);

(6) a transformant carrying the DNA as described in (3) or the recombinant vector as described in (5);

(7) a process for producing the G protein coupled receptor protein or a salt thereof as described in (1) which comprises culturing the transformant as described in (6);

(8) a method for determining a ligand to the G protein coupled receptor protein as described in (1), which comprises contacting (i) the G protein coupled receptor protein or a salt thereof as described in (1) or the partial peptide or a salt thereof as described in (2), with (ii) a sample to be tested;

(9) a screening method for a compound capable of changing the binding activity of the G protein coupled receptor protein as described in (1) with a ligand, or a salt thereof, which comprises making a comparison between: (i) at least one case where said ligand is contacted with the G protein coupled receptor protein or a salt thereof as described in (1) or the partial peptide or a salt thereof as described in (2), and (ii) at least one case where said ligand together with a sample to be tested is contacted with the G protein coupled receptor protein or a salt thereof as described in (2) or the partial peptide or a salt thereof as described in (2);

(10) a kit for the screening of a compound capable of changing the binding activity of the G protein coupled receptor protein as described in (1) with a ligand, or a salt thereof, which comprises the G protein coupled receptor protein or a salt thereof as described in (1), or the partial peptide or a salt thereof as described in (2);

(11) a compound capable of changing the binding activity of the G protein coupled receptor protein as described in (1) with a ligand, or a salt thereof, which is obtained by the screening method as described in (9) or by using the kit for the screening as described in (10);

(12) an antibody against the G protein coupled receptor protein or a salt thereof as described in (1) or the partial peptide or a salt thereof as described in (2).

More specifically, it further provides:

(13) the method for determining a ligand as described in (8) in which the ligand is one of the following: angiotensin, bombesin, cannabinoids, cholecystokinin, glutamine, serotonine, melatonin, neuropeptide Y, opioids, purine, vasopressin, oxytocin, VIPs (vasoactive intestinal and related peptides), somatostatin, dopamine, motilin, amyrin, bradykinin, CGRP (calcitonin gene related peptide), leukotrienes, pancreastatin, prostanoids, thromboxane, adenosine, adrenaline, $\alpha$- and $\beta$-chemokines (e.g. IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP1$\beta$, RANTES, etc.), endothelin, enterogastrin, histamine, neurotensin, TRH, pancreatic polypeptides and galanin;

(14) a screening method for a compound capable of changing the binding activity of the G protein coupled receptor protein as described in (1) with a ligand or a salt thereof, which comprises measuring amounts of a labeled ligand bound to said G protein coupled receptor protein in at least two cases: (i) where the labeled ligand is contacted with the G protein coupled receptor protein or a salt thereof as described in (1) or the partial peptide or a salt thereof as described in (2), and (ii) where the labeled ligand together with a compound to be tested is contacted with the G protein coupled receptor protein or a salt thereof as described in (1) or the partial peptide or a salt thereof as described in (2), and comparing the measured amounts of the labeled ligand;

(15) a screening method for a compound capable of changing the binding activity of the G protein coupled receptor protein as described in (1) with a ligand or a salt thereof, which comprises measuring amounts of a labeled ligand bound to a cell comprising said G protein coupled receptor protein in at least two cases: (i) where the labeled ligand is contacted with the said cell, and (ii) where the labeled ligand together with a compound to be tested is contacted with the said cell, and comparing the amounts of the labeled ligand measured;

(16) a screening method for a compound capable of changing the binding activity of the G protein coupled receptor protein as described in (1) with a ligand or a salt thereof, which comprises measuring amounts of a labeled ligand bound to a membrane fraction of a cell comprising said G protein coupled receptor protein in at least two cases; (i) where the labeled ligand is contacted with said membrane fraction, and (ii) where the labeled ligand together with a compound to be tested is contacted with the membrane fraction, and comparing the amounts of the labeled ligand measured;

(17) a screening method for a compound capable of changing the binding activity of the G protein coupled receptor protein as described in (1) with a ligand or a salt thereof, which comprises measuring amounts of a labeled ligand bound to said G protein coupled receptor protein in at least two cases; (i) where the labeled ligand is contacted with the G protein coupled receptor protein as described in (1) which is expressed on the cell membrane of the transformant as described in (6) upon culturing said transformant, and (ii) where the labeled ligand together with a compound to be tested is contacted with the G protein coupled receptor protein as described in (1) which is expressed on the cell membrane of the transformant as described in (6) upon culturing said transformant, and comparing the amounts of the labeled ligand measured;

(18) a screening method for a compound capable of changing the binding activity of the G protein coupled receptor protein as described in (1) with a ligand or a salt thereof, which comprises measuring G protein coupled receptor protein-mediated cell-stimulating activities in at least two cases: (i) where a compound capable of activating the G protein coupled receptor protein as described in (1) is contacted with the said cell comprising said G protein coupled receptor protein as described in (1), and (ii) where the compound capable of activating the G protein coupled receptor protein as described in (1) together with a compound to be tested is contacted with the cell comprising the G protein coupled receptor protein, and comparing the cell-stimulating activities measured;

(19) a screening method for a compound capable of changing the binding activity of the G protein coupled receptor protein as described in (1) with a ligand or a salt thereof, which comprises measuring G protein coupled receptor protein-mediated cell-stimulating activities in at least two cases: (i) where a compound capable of activating the G protein coupled receptor protein as described in (1) is contacted with the G protein coupled receptor protein which is expressed on the cell membranes of a transformant containing the DNA as described in (3) upon culturing said transformant, and (ii) where the compound capable of activating the G protein coupled receptor protein as described in (1) together with a compound to be tested is contacted with the G protein coupled receptor protein which is expressed on the cell membrane of the transformant containing the DNA as described in (3) upon culturing said tranformant, and comparing the cell-stimulating activities measured;

(20) the screening method as described in (18) or (19), wherein the compound capable of activating the G protein coupled receptor protein as described in (1) is one of the following: angiotensin, bombesin, cannabinoids, cholecystokinin, glutamine, serotonine, melatonin, neuropeptide Y, opioids, purine, vasopressin, oxytocin, VIPs (vasoactive intestinal and related peptides), somatostatin, dopamines, motilin, amyrin, bradykinin, CGRP (calcitonin gene related peptide), leukotriene, pancreastatin, prostanoids, thromboxane, adenosine, adrenaline, $\alpha$- and $\beta$-chemokines (e.g. IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP1$\beta$, RANTES, etc.), endothelin, enterogastrin, histamine, neurotensin, TRH, pancreatic polypeptides and galanin;

(21) the compound or a salt thereof as obtained by one of the screening methods as described in (9) and (14) to (20);

(22) a pharmaceutical composition which comprises the compound as described in (21) or a salt thereof;

(23) the kit as described in (10) which comprises a cell containing the G protein coupled receptor protein as described in (1);

(24) the kit as described in (10) which comprises a cellular membrane fraction containing the G protein coupled receptor protein as described in (1);

(25) the compound or a salt thereof as obtained by using the kit as described in (10), (23) or (24);

(26) a pharmaceutical composition comprising the compound as described in (25) or a salt thereof; and

(27) a method of assaying the G protein coupled receptor protein or a salt thereof as described in (1) or the partial peptide or a salt thereof as described in (2), which comprises contacting the antibody as described in (12) with the G protein coupled receptor protein or a salt thereof as described in (1) or the partial peptide or a salt thereof as described in (2).

[Brief Description of the Drawings]

Fig. 1 shows a partial nucleotide sequence which was analyzed from the 5' side of the novel receptor protein cDNA clone p63A2 obtained from human amygdaloid nucleus by PCR amplification and the amino acid sequence encoded thereby. The underlined part corresponds to the synthetic primer used for PCR amplification.

Fig. 2 shows a partial nucleotide sequence which was analyzed from the 3' side of the novel receptor protein cDNA clone p63A2 obtained from the human amygdaloid nucleus by PCR amplificaiton and the amino acid sequence encoded thereby. The underlined part corresponds to the synthetic primer used for PCR amplification.

Fig. 3 shows the hydrophobicity plots made based on the amino acid sequence shown in Fig. 1. From this figure, the presence of hydrophobic domains indicated by 1 to 3 is suggested.

Fig. 4 shows the hydrophobicity plots made based on the amino acid sequence shown in Fig. 2. From this figure, the presence of hydrophobic domains indicated by 4 to 6 is suggested.

Fig. 5 shows a partial amino acid sequence derived by the analysis of the p63A2-encoded novel receptor protein cDNA as compared with a partial amino acid sequence of the G protein coupled receptor protein (P30731) whose expression is induced by a murine T cell-derived glucocorticoid. The dark-shaded portions indicate agreeing amino acid residues.

Fig. 6 shows the p63A2-specific primers prepared for cloning, by RACE, the region of unknown sequence of the novel receptor protein cDNA from human hypothalamus, and the locations of the primers on $\lambda$gt11.

Fig. 7 shows the results of analysis, by 1% agarose gel electrophoresis, of the amplificaiton products obtained by RACE using various combinations of the primers shown in Fig. 6.

Fig. 8 shows the results of Southern hybridization of the plurality of amplification products shown in Fig. 7 using a p63A2-specific sequence as the probe. From the figure, the presence of amplification products having a p63A2-specific sequence as indicated by the arrows was suggested.

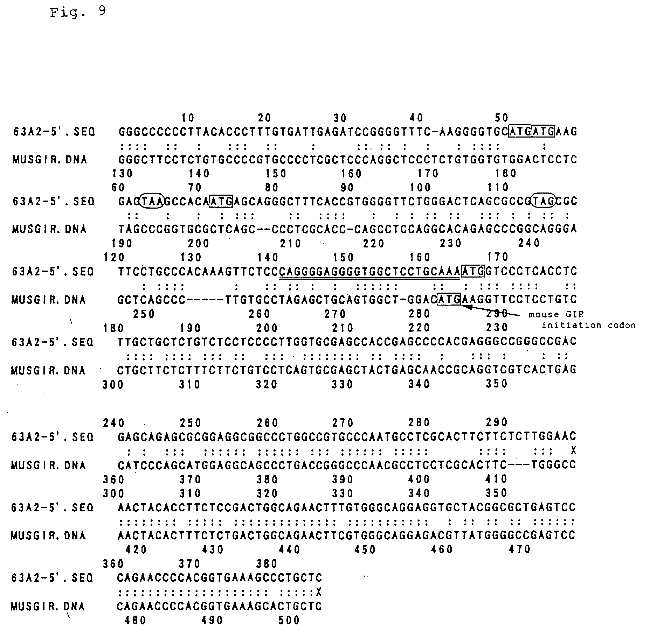

Fig. 9 shows the results of homology searching between the 5' terminal region nucleotide sequence of the cDNA obtained from human hypothalamus by RACE and the corresponding nucleotide sequence for the G protein coupled receptor protein GIR (P30731) whose expression is induced by a murine T cell-derived glucocorticoid. The portions enclosed with a square indicate sequences each possibly serving as the translation initiation codon and the portions enclosed with an ellipse indicate sequences each possibly serving as the translation termination codon. The portion doubly underlined indicates the sequence of the primer for amplifying the entire open reading frame. From this figure, the presence, in a part corresponding to the vicinity of the initiation codon for murine GIR, of a sequence possibly serving as the initiation codon in cDNA amplified by RACE method as well was suggested.

Fig. 10 shows the results of homology searching between the 3' terminal region nucleotide sequence of the cDNA obtained from human hypothalamus by RACE and the corresponding nucleotide sequence for murine GIR. From this figure, it was suggested that the 3' terminal region of the cDNA obtained, as compared with the murine GIR, failed to reach the translation termination codon.

Fig. 11 schematically shows the region of unknown sequence of open reading frame of the cDNA obtained from the human hypothalamus as estimated based on the nucleotide sequences shown in Fig. 9 and Fig. 10.

Fig. 12 shows the results of analysis, by 1% agarose gel electrophoresis, of the amplification products obtained from total human brain cDNA by 3' AmpliFINDER RACE.

Fig. 13 shows the results of Southern hybridization of the plurality of amplification products shown in Fig. 12 using a p63A2-specific sequence as the probe. From this figure, the presence of an amplification product having a p63A2-specific sequence as indicated by the arrow.

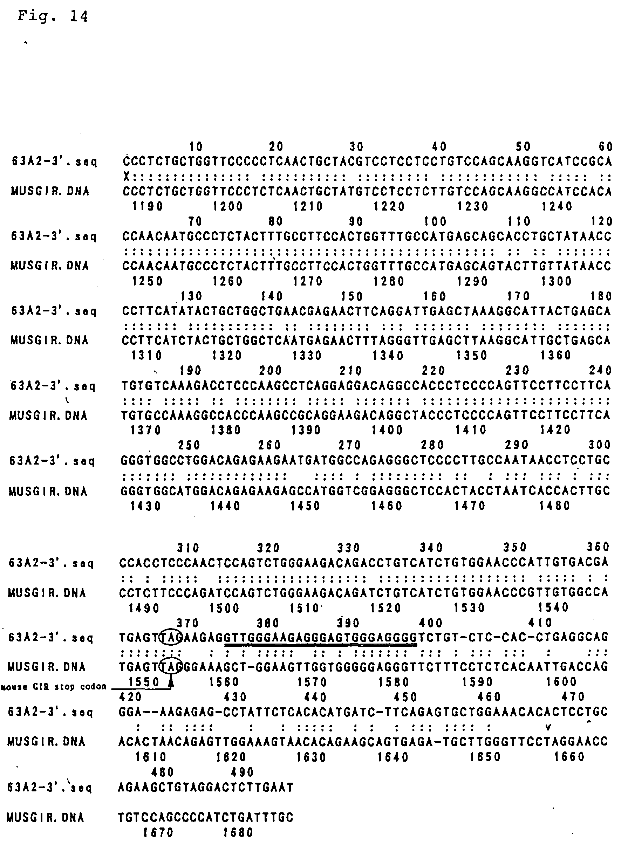

Fig. 14 shows the results of homology searching between the 3' terminal region nucleotide sequence of the cDNA obtained from total human brain poly(A)$^+$ RNA by 3' AmpliFINDER RACE and the corresponding sequence for murine GIR. The portion enclosed with an ellipse indicates a sequence possibly serving as the translation termination codon. The portion doubly underlined indicates the primer sequence for full-length open reading frame amplification. From this figure, the presence, in the vicinity of the termination codon for murine GIR, of a sequence possibly serving as the termination codon in the cDNA as well was suggested.

Fig. 15 shows the amplification product containing the full-length open reading frame of 63A2full obtained by PCR amplification using primers prepared based on the nucleotide sequences of the 5' nontranslational region shown in Fig. 9 and the 3' nontranslational region shown in Fig. 14.

Fig. 16 and Fig. 17 show the nucleotide sequence of the entire open reading frame for the p63A2full-encoded receptor protein cDNA as obtained by subcloning the PCR amplification product shown in Fig. 15 as well as the amino acid sequence encoded by said sequence.

Fig. 18 shows the hydrophobicity plots made on the basis of the amino acid sequence shown in Fig. 16 and Fig. 17. From this figure, the presence of seven hydrophobic domains as indicated by I through VII was suggested.

Fig. 19 shows a comparison between the p63A2full-encoded amino acid sequence and the murine GIR-encoded amino acid sequence. The dark-shaded portions indicate identical amino acid residues.

[Detailed Description of the Preferred Embodiment]

The G protein coupled receptor protein of the present invention may be any of G protein coupled receptor proteins derived from various tissues, e.g. amygdaloid nucleus, hypophysis, pancreas, brain, kidney, liver, gonad, thyroid gland, gall bladder, bone marrow, lung, alimentary canal, blood vessel, heart, thymus, spleen, leucocyte etc. of human and other warm-blooded animals, e.g. guinea pig, rat, mouse, swine, sheep, bovine, monkey, etc.; or cells; and comprising an amino acid sequence of SEQ ID NO:1 or substantial equivalent thereto. Thus, the G protein coupled receptor protein of the present invention includes, in addition to proteins comprising the SEQ ID NO:1, those proteins comprising amino acid sequences of more than 90%, preferably more than 95% homology to the amino acid sequence of SEQ ID NO:1 and having qualitatively substantially equivalent activity to proteins comprising the amino acid sequence of SEQ ID NO:1 in at least one biological activity. The qualitatively substantially equivalent activities which these proteins are possessed may include ligand binding activity and signal transduction activity. The term "substantially equivalent" means that the nature of the ligand binding activity and the like is equivalent. Therefore, it is allowable that even differences among grades such as the strength of ligand binding activity and the molecular weight of receptor protein are present.

Preferably, the G protein coupled receptor proteins include human amygdaloid nucleus-derived G protein coupled receptor proteins which comprises the amino acid sequence of SEQ ID NO:1 The G protein coupled receptor proteins further include mutants such as deletion proteins wherein 1 to 30 amino acid residues, preferably 1 to 10 amino acid residues, more preferably a few amino acid residues are deleted from the amino acid sequence of SEQ ID NO:1, proteins wherein 1 to 30 amino acid residues, preferably 1 to 10 amino acid residues, more preferably a few amino acid residues are added to the amino acid sequence of SEQ ID NO:1, the proteins wherein 1 to 30 amino acid residues, preferably 1 to 10 amino acid residues, more preferably a few amino acid residues in the amino acid sequence of SEQ ID NO:1 are substituted with one or more other amino acid residues and combinations thereof. In one embodiment, transmembrane regions are deleted.

Furthermore, the G protein coupled receptor protein includes the protein in which the N-terminal Met has been protected with a protective group, e.g. $C_{1-6}$ acyl such as formyl or acetyl, the protein in which the N-terminal side of Glu has been cleaved in vivo to form pyroglutamine, the protein in which the side chain of any relevant constituent amino acid has been protected, and the complex protein such as glycoproteins available upon attachment of sugar chains.

The salt of G protein coupled receptor protein includes salts with physiologically acceptable bases, e.g. alkali metals or acids such as organic or inorganic acids, and is preferably a physiologically acceptable acid addition salt. Examples of such salts are salts thereof with inorganic acids, e.g. hydrochloric acid, phosphoric acid, hydrobromic acid or sulfuric acid, etc. and salts thereof with organic acids, e.g. acetic acid, formic acid, propionic acid, fumaric acid, maleic acid, succinic acid, tartaric acid, citric acid, malic acid, oxalic acid, benzoic acid, methanesulfonic acid or benzenesulfonic acid, etc.

The G protein coupled receptor protein or a salt thereof can be produced from the tissues or cells of human or other warm-blooded animals by the per se known purification technology or, as described hereinafter, by culturing a transformant carrying a DNA coding for the G protein coupled receptor protein. It can also be produced in accordance with the procedures for peptide synthesis which are described hereinafter.

A partial peptide of G protein coupled receptor protein may include, for example, a fragment containing an extracellular portion of the G protein coupled receptor protein, i.e. the site which is exposed outside the cell membranes. Examples of the partial peptide are fragments containing a region which is an extracellular area (hydrophilic region) as analyzed in a hydrophobic plotting analysis of the G protein coupled receptor protein, such as shown in Fig. 17, e.g. peptide which comprises the amino acid sequence of from Met at the first position to Leu at the 91st position. Furthermore, a fragment which partly contains a hydrophobic region such as from Val at the 92nd position to Leu at the 300th position in SEQ ID NO:1 or from Phe at the 301st position to Ser at the 423rd position in SEQ ID NO:1 may also be used. In one embodiment peptides which contain each domain may be used too. Alternatively, peptides which contains multiple domains at the same time can be used as well. In one embodiment one can use any peptide fragment of SEQ ID NO:1 except SEQ ID NO:3 (Val-Cys-His-Val-Ile-Phe-Lys-Asn-Gln-Ang-Met-His-Ser-Ala-Thr-Ser-Leu-Phe-Ile-Val-Asn-Leu-Ala-Thr-Pro-Phe-Thr-Leu-Val-Arg-Phe-Val-Asn-Sev-Thr-Trp-Ile-Phe-Gly-Lys-Gly-Met-Cys-His-Val-Ser-Arg-Phe-Ala-Glu-Tyr-Cys-Leu-His-Val-Ser-Ala-Leu-Thr) and portions thereof. One would also not use the cDNA segment SEQ ID NO:5 (bases 274-483) or portions thereof. One would also preferably not use peptide fragments from amino acid residue 230-300 (SEQ ID NO:4) that are less than 16 amino acid residues. Similarly in the region bases (688-900) (SEQ ID NO: 6), one would not use cDNA segments that are less than 48 base pairs. In an alternative embodiment the peptide fragments would contain an amino acid residue that is unique to the human protein when compared with the corresponding murine protein. See Figure 19. In an alternative preferred embodiment, the peptide fragments are at least 16 amino acids in length preferably at least 20 amino acids, still more preferably at least 25 amino acids.

The salt of a partial peptide of G protein coupled receptor protein may be the same type of salt as mentioned for the salt of G protein coupled receptor protein.

The partial peptide of a G protein coupled receptor protein or a salt thereof can be produced by per se known procedures for peptide synthesis or by cleaving the G protein coupled receptor protein with a suitable peptidase. The process for peptide synthesis may be a solid-phase synthesis and/or a liquid-phase synthesis. Thus, the objective peptide can be produced by condensing a partial peptide or amino acid capable of constituting the protein with the residual part thereof and, when the product has a protective group, the protective group is detached whereupon a desire peptide can be manufactured. The known technology for condensation and deprotection includes the procedures described in the following literature (1)-(5).

(1) M. Bodanszky and M. A. Ondetti, Peptide Synthesis, Interscience Publishers, New York, 1966
(2) Schroeder and Luebke, The Peptide, Academic Press, New York, 1965
(3) Nobuo Izumiya et al., Fundamentals and Experiments in Peptide Synthesis, Maruzen, 1975
(4) Haruaki Yajima and Shumpei Sakakibara, Biochemical Experiment Series 1, Protein Chemistry IV, 205, 1977
(5) Haruaki Yajima (ed.), Development of Drugs-Continued, 14, Peptide Synthesis, Hirokawa Shoten

After the reaction, the protein of the present invention can be purified and isolated by a combination of conventional purification techniques such as solvent extraction, distillation, column chromatography, liquid chromatography, and recrystallization. Where the protein isolated as above is a free compound, it can be converted to a suitable salt by known methods. Conversely where the isolated product is a salt, it can be converted to the free peptide by known methods.

The DNA coding for the G protein coupled receptor protein of the present invention may be any DNA comprising a nucleotide sequence encoding the G protein coupled receptor protein which comprises an amino acid sequence of SEQ ID NO:1 or substantial equivalent thereto. It may also be any one of human genomic DNA, human genomic DNA library, human tissue- or cell-derived cDNA, human tissue- or cell-derived cDNA library, and synthetic DNA. The vector for such a library may include bacteriophage, plasmid, cosmid, and phargimide. Furthermore, using an mRNA fraction prepared from a tissue or cells, a direct amplification can be carried out by the RT-PCR method.

To be specific, the DNA encoding the human amygdaloid nucleus-derived G protein coupled receptor protein which comprises the amino acid sequence of SEQ ID NO:1 include a DNA which comprises the nucleotide sequence of SEQ ID NO:2 or its partial nucleotide sequence, e.g. the nucleotide sequence of the 1st to the 273rd position, the 274th to the 900th position, or the 901st to the 1269th position in SEQ ID NO:2.

Additionally, one can use a DNA encoding any of the aforementioned partial proteins such as a fragment of 16 amino acids, a mutant, a pingle or multiple domain peptide.

Furthermore, a DNA which can hybridize to said DNA can be used. The hybrid DNA is the DNA coding for the protein which has the similar activity to the G protein coupled receptor protein of SEQ ID NO:1.

The DNA fully encoding the G protein coupled receptor protein of the present invention can be cloned either by PCR amplification using synthetic DNA primers having a partial nucleotide sequence of the G protein coupled receptor protein or by hybridization using the DNA inserted in a suitable vector and labeled with a DNA fragment comprising a part or full region of a human-derived G protein coupled receptor protein or a synthetic DNA. The hybridization can be carried out typically by the procedure described in Molecular Cloning (2nd ed., J. Sambrook et al., Cold Spring Harbor Lab. Press, 1989). When a commercial library is used, the instructions given in the accompanying manual can be followed.

The cloned DNA coding for the G protein coupled receptor protein can be used directly or after digestion with a restriction enzyme or addition of a linker depending on purposes. This DNA has ATG as the translation initiation codon at the 5' end and may have TAA, TGA, or TAG as the termination codon at the 3' end. The translation initiation and termination codons can be added by means of suitable DNA adapters.

An expression vector for the G protein coupled receptor protein can be produced by, for example (a) cutting out a target DNA fragment from the DNA for the G protein coupled receptor protein of the present invention and (b) ligating the target DNA fragment with the downstream side of a promoter in a suitable expression vector.

The vector may include plasmids derived from Escherichia coli, e.g., pBR322, pBR325, pUC12, pUC13, etc.; plasmids derived from Bacillus subtilis, e.g., pUB110, pTP5, pC194, etc.; plasmids derived from yeasts e.g., pSH19, pSH15, etc.; bacteriophages such as λ - phage, and animal virus such as retrovirus pox virus such as a or an avipox virus, a herpes virus, vaccinia virus and baculovirus.

According to the present invention, any promoter can be used as long as it is compatible with the host cell which is used for expressing a gene. When the host for the transformation is E. coli, the promoters are preferably trp promoters, lac promoters, recA promoters, $\lambda_{PL}$ promoters, lpp promoters, etc. When the host for the transformation is Bacillus, the promoters are preferably SPO1 promoters, SPO2 promoters, penP promoters, etc. When the host is a yeast, the promoters are preferably PHO5 promoters, PGK promoters, GAP promoters, ADH promoters, etc. When the host is an animal cell, the promoters include SV40-derived promoters, retrovirus promoters, metallothionein promoters, heat shock promoters, cytomegalovirus (CMV) promoters, SRα promoters, etc. An enhancer can be effectively utilized for expression.

As required, furthermore, a host-compatible signal sequence is added to the N-terminal side of the G protein coupled receptor protein. When the host is E. coli, the utilizable signal sequences may include alkaline phosphatase signal sequences, OmpA signal sequences, etc. When the host is Bacillus, they may include α -amylase signal sequences, subtilisin signal sequences, etc. When the host is a yeast, they may include mating factor α signal sequences, invertase signal sequences, etc. When the host is an animal cell, they may include insulin signal sequences, α -interferon signal sequences, antibody molecule signal sequences, etc.

A transformant or transfectant is produced by using the vector thus constructed, which carries the G protein coupled receptor protein-encoding DNA of the present invention. The host may be, for example, Escherichia microorganisms, Bacillus microorganisms, yeasts, insect cells, animal cells, etc. Examples of the Escherichia and Bacillus microorganisms include Escherichia coli K12 • DH1 [Proc. Natl. Acad. Sci. USA, Vol. 60, 160 (1968)], JM103 [Nucleic Acids Research, Vol. 9, 309 (1981)], JA221 [Journal of Molecular Biology, Vol. 120, 517 (1978)], HB101 [Journal of molecular Biology, Vol, 41, 459 (1969)], C600 [Genetics, Vol. 39, 440 (1954)], etc. Examples of the Bacillus microorganism are, for example Bacillus subtilis MI114 [Gene, Vol. 24, 255 (1983)], 207-21 [Journal of Biochemistry, Vol. 95, 76 (1984)], etc.

The yeast may be, for example, Saccharomyces cerevisiae AH22, AH22R⁻, NA87-11A, DKD-5D, 20B-12, etc. The insect may include a silkworm (Bombyx mori larva), [Maeda et al, Nature, Vol. 315, 592 (1985)] etc. The host animal cell may be, for example, monkey-derived cell line, COS-7, Vero, Chinese hamster ovary cell line (CHO cell), DHFR gene-deficient Chinese hamster cell line (dhfr⁻ CHO cell), mouse L cell, mouse myeloma cell, human FL, etc.

Depending on the host cell used, transformation is done using standard techniques appropriate to such cells. Transformation of Escherichia microorganisms can be carried out in accordance with methods as disclosed in, for example, Proc. Natl. Acad. Sci. USA, Vol. 69, 2110 (1972), Gene, Vol. 17, 107 (1982), etc. Transformation of Bacillus microorganisms can be carried out in accordance with methods as disclosed in, for example, Molecular & General Genetics, Vol. 168, 111 (1979), etc. Transformation of the yeast can be carried out in accordance with methods as disclosed in, for example, Proc. Natl. Acad. Sci. USA, Vol. 75, 1929 (1978), etc. The insect cells can be transformed in accordance with methods as disclosed in, for example, Bio/Technology, 6, 47-55, 1988. The animal cells can be transformed by methods as disclosed in, for example, Virology, Vol. 52, 456, 1973, etc. The transformants or transfectants wherein the expression vector carrying G protein coupled receptor protein-encoding DNA harbors are produced according to the aforementioned techniques.

Cultivation of the transformant (transfectant) in which the host is Escherichia or Bacillus microorganism can be carried out suitably in a liquid culture medium. The culture medium may contains carbon sources, nitrogen sources, minerals, etc. necessary for growing the transformant. The carbon source may include glucose, dextrin, soluble starch, sucrose, etc. The nitrogen source may include organic or inorganic substances such as ammonium salts, nitrates, corn steep liquor, peptone, casein, meat extracts, bean-cakes, potato extracts, etc. Examples of the minerals may include calcium chloride, sodium dihydrogen phosphate, magnesium chloride, etc. It is further allowable to add yeasts, vitamines, growth-promoting factors, etc. It is desired that the culture medium is pH from about 5 to about 8.

The Escherichia microorganism culture medium is preferably an M9 medium containing, for example, glucose and casamino acid (Miller, Journal of Experiments in Molecular Genetics), 431-433, Cold Spring Harbor Laboratory, New York, 1972. Depending on need, the medium may be supplemented with drugs such as 3β -indolyl acrylic acid in order to improve efficiency of the promoter. In the case of an Escherichia host, the cultivation is carried out usually at about 15 to 43°C for about 3 to 24 hours. As required, aeration and stirring may be applied. In the case of Bacillus host, the cultivation is carried out usually at about 30 to 40°C for about 6 to 24 hours. As required, aeration and stirring may also be applied. In the case of the transformant in which the host is a yeast, the culture medium used may include, for example, a Burkholder minimum medium [Bostian, K.L. et al., Proc. Natl. Acad. Sci. USA, Vol. 77, 4505 (1980)], an SD medium containing 0.5% casamino acid [Bitter, G.A. et al., Proc. Natl. Acad. Sci. USA, Vol. 81, 5330 (1984)], etc. It is preferable that the pH of the culture medium is adjusted to be from about 5 to about 8. The cultivation is carried out usually at about 20 to 35°C for about 24 to 72 hours. As required, aeration and stirring may be applied. In the case of the transformant in which the host is an insect, the culture medium used may include those obtained by suitably adding additives such as passivated (or immobilized) 10% bovine serum and the like to the Grace's insect medium (Grace, T.C.C., Nature, 195, 788 (1962)). It is preferable that the pH of the culture medium is adjusted to be about 6.2 to 6.4. The cultivation is usually carried out at about 27°C for about 3 to 5 days. As desired, aeration and stirring may be applied. In the case of the transformant in which the host is an animal cell, the culture medium used may include MEM medium [Science, Vol. 122, 501 (1952)], DMEM medium [Virology, Vol. 8, 396 (1959)], RPMI 1640 medium [Journal of the American Medical Association, Vol. 199, 519 (1967)], 199 medium [Proceedings of the Society of the Biological Medicine, Vol. 73, 1 (1950)], etc. which are containing, for example, about 5 to 20% of fetal calf serum. It is preferable that the pH is from about 6 to about 8. The cultivation is usually carried out at about 30 to 40°C for about 15 to 60 hours. As required, medium exchange, aeration and stirring may be applied.

Separation and purification of the G protein coupled receptor protein from the above-mentioned cultures can be carried out according to methods described herein below.

To extract G protein coupled receptor protein from the cultured microorganisms or cells, the microorganisms or cells are collected by known methods after the cultivation, suspended in a suitable buffer solution, disrupted by ultrasonic waves, lysozyme and/or freezing and thawing, etc. and, then, a crude extract of the G protein coupled receptor protein is obtained by centrifugation or filtration. Other conventional extracting or isolating methods can be applied. The buffer solution may contain a protein-denaturing agent such as urea or guanidine hydrochloride or a surfactant such as Triton X-100 (registered trademark, hereinafter often referred to as "TM").

In the case where the G protein coupled receptor proteins are secreted into culture media, supernatant liquids are separated from the microorganisms or cells after the cultivation is finished and the resulting supernatant liquid is collected by known methods. The culture supernatant liquid and extract containing the G protein coupled receptor protein can be purified by suitable combinations of known methods for separation, isolation and purification. The known methods of separation, isolation and purification may include methods which utilizes solubility, such as salting out or sedimentation with solvents, methods which utilizes chiefly a difference in the molecular size or weight, such as dialysis, ultrafiltration, gel filtration and SDS-polyacrylamide gel electrophoresis, methods utilizing a difference in the electric charge, such as ion-exchange chromatography, methods utilizing specific affinity such as affinity chromatography, methods utilizing a difference in the hydrophobic property, such as reversed-phase high-performance liquid chromatography, and methods utilizing a difference in the isoelectric point such as isoelectric electrophoresis, etc.

In cases where the G protein coupled receptor protein thus obtained is in a free form, the free protein can be converted into a salt thereof by known methods or method analogous thereto. In case, where the G protein coupled receptor protein thus obtained is in a salt form vice versa, the protein salt can be converted into a free form or into any other salt thereof by known methods or method analogous thereto.

The G protein coupled receptor protein produced by the transformant can be arbitrarily modified or a polypeptide can be partly removed therefrom, by the action of a suitable protein-modifying enzyme before or after the purification. The protein-modifying enzyme may include trypsin, chymotrypsin, arginyl endopeptidase, protein kinase, glycosidase, etc. The activity of the G protein coupled receptor protein thus formed can be measured by experimenting the coupling (or binding) with labeled ligand or by enzyme immunoassays (enzyme linked immunoassays) using specific antibodies.

In the following, typical examples are shown with regard to the production of the G protein coupled receptor protein (63A2full) of the present invention.

(1) Construction of an expression vector for use in animal cells which contains the full-length open reading frame of 63A2full-encoding cDNA as an insert

The plasmid p63A2full obtained as mentioned later herein in Example 2 is digested with the restriction enzyme SalI and thereby the full-length open reading frame of 63A2full-encoding cDNA is excised. This is subjected to ligation reaction with the expression vector pAKKO-111 or the like for use in animal cells as digested with SalI in the same manner and further treated with BAP (bacterial alkaline phosphatase) to prevent autocyclization.

After completion of the ligation reaction, a portion of the reaction mixture is used to transform Escherichia coli DH5 or the like. Among the transformants obtained, one harboring a recombinant vector containing the 63A2full-encoding cDNA inserted in the forward direction with respect to the promoter, for example SRα, incorporated in advance in pAKKO-111 is selected by mapping by means of restriction enzyme cleavage, or by nucleotide sequence determination, and the plasmid DNA is prepared in large amounts.

(2) Preparation of CHO cells expressing the novel G protein coupled receptor 63A2full

The expression vector DNA prepared above in (1) is introduced into CHO dhfr⁻ cells using a kit for gene introduction into animal cells which is based on the calcium phosphate method or liposome method, for instance. Whereas the original CHO dhfr⁻ cells cannot grow on a nucleic acid-free medium, cells containing the expression vector introduced therein can grow even on a nucleic acid-free medium. Utilizing this, cells into which the cDNA has been introduced are selected using a selective medium prepared by adding dialized fetal calf serum to a nucleic acid-free medium. Further, from the cells selected based on their ability to grow in the selective medium, a poly(A)$^+$ RNA fraction is prepared, then cDNA is prepared using a commercially available kit, for instance, and RT-PCR is carried out using such primers as shown in Example 2. The expression of 63A2full in these cells can be verified by detecting a specific band resulting from RT-PCR. The expression CHO cells selected are cultured in $\alpha$-MEM (nucleic acid-free) medium supplemented with 10% of dialyzed fetal calf serum and an appropriate antibiotic under conditions of 37°C and 5% $CO_2$ plus 95% air, whereby the 63A2full receptor protein is produced in the CHO cells.

(3) Preparation of the novel G protein coupled receptor 63A2full receptor protein

Using CHO cells or human or animal tissues or cultured cells capable of expressing 63A2full receptor as a material, the 63A2full receptor protein is purified by the known techniques of solubilization and chromatography. On that occasion, when a ligand capable of binding to the 63A2full receptor or an antibody to the 63A2full receptor protein or a fragment peptide thereof is used, the 63A2full receptor protein contained in various chromatographic fractions can be detected with good efficiency.

More specifically, a ligand labeled by an appropriate means is bound to the 63A2full receptor and then the both are crosslinked by an appropriate method, and the 63A2full receptor protein is purified with the label as a sign for identification. Further, the 63A2full receptor protein can be obtained more efficiently by preparing an antibody column by causing binding of an anti-63A2full receptor antibody and performing immunoaffinity chromoatography using said column. In addition, by using said antibody, the 63A2full receptor protein contained in various chromatographic fractions can be quantitatively detected by EIA, RIA or Western blotting, for instance, and the quantitation results can serve as indices of purification. Furthermore, it is also possible to allow the integrations of the 63A2full receptor protein-binding G protein coupled receptor protein with various molecules of the information transmission system to remain as they are. In other words, for screening out an agonist of or an antagonist to the 63A2full receptor, for instance, the 63A2full receptor need not be obtained in an absolutely pure form but may be obtained in the form of a membrane fraction, a solubilization product or a partially purified product.

The G protein coupled receptor protein, the partial peptide thereof and the G protein coupled receptor protein-encoding DNA can be used for:

1) determining a ligand to the G protein coupled receptor protein of the present invention,
2) obtaining an antibody and an antiserum,
3) constructing a system for expressing a recombinant receptor protein,
4) developing a receptor-binding assay system using the above developing system and screening pharmaceutical candidate compounds,
5) designing drugs based upon comparison with ligands and receptors which have a similar or analogous structure,
6) preparing a probe for the analysis of genes and preparing a PCR primer,
7) gene manipulation therapy,

In particular, it is possible to screen a G protein coupled receptor agonist or antagonist specific to a warm-blooded animal such as human being by a receptor-binding assay system which uses a system for expressing a recombinant G protein coupled receptor protein. The agonist or antagonist thus screened or characterized permits various applications including prevention and/or therapy of a variety of diseases.

Described below are uses of G protein coupled receptor proteins, partial peptides thereof or G protein coupled receptor protein-encoding DNAs and their antibodies.

(1) Method for Determing a Ligand to the G Protein Coupled Receptor Protein of the Present Invention

The G protein coupled receptor protein, the partial peptide thereof or a salt thereof is useful as a reagent for investigating or determining a ligand to said G protein coupled receptor protein.

According to the present invention, methods for determining a ligand to the G protein coupled receptor protein which comprises contacting the G protein coupled receptor protein or the partial peptide thereof with the compound to be tested, and measuring the binding amount, the cell stimulating activity, etc. of the test compound to the G protein coupled receptor protein or the partial peptide thereof are provided.

The compound to be tested may include not only known ligands such as angiotensin, bombesin, canavinoid, cholecystokinin, glutamine, serotonin, melatonin, neuropeptide Y, opioids, purine, vasopressin, oxytocin, VIP (vasoactive intestinal and related peptides), somatostatin, dopamine, motilin, amylin, bradykinin, CGRP (calcitonin gene related peptides), leukotrienes, pancreastatin, prostaglandin, thromboxane, adenosine, adrenaline, α- and β-chemokines such as IL-8, GROα, GROβ, GROγ, NAP-2, ENA-78, PF4, IP10, GCP-2, MCP-1, HC14, MCP-3, I-309, MIP1α, MIP-1β, RANTES, etc.; endothelin, enterogastrin, histamine, neurotensin, TRH, pancreatic polypeptide, galanin, modified derivatives thereof, analogues thereof, family members thereof and the like but also tissue extracts, cell culture supernatants, etc. of human or warm-blooded animals such as mice, rats, swines, cattle, sheep and monkeys, etc. For example, said tissue extract, said cell culture supernatant, etc. is added to the G protein coupled receptor protein for measurement of the cell stimulating activity, etc. and fractionated by relying on the measurements whereupon a single ligand can be finally determined and obtained.

In one specific embodiment of the present invention, said method for determining the ligand includes a method for determining whether a sample (including a compound or a salt thereof) is capable of stimulating a target cell which comprises binding said compound with the G protein coupled receptor protein either in the presence of the G protein coupled receptor protein, the partial peptide thereof or a salt thereof, or in a receptor binding assay system in which the expression system for the recombinant receptor protein is constructed and used; and measuring the receptor-mediated cell stimulating activity, etc. Examples of a cell stimulating activities that can be measured include promoting or inhibiting biological responses, e.g. liberation of arachidonic acid metabolites, liberation of acetylcholine, increase of intracellular $Ca^{2+}$, production of endocellular cAMP, production of cGMP, production of inositol phosphate, changes in the cell membrane potential, phosphorylation of intracellular protein, activation of c-fos, decrease in extracellular pH, etc. Examples of said compound or a salt thereof capable of stimulating the cell via binding with the G protein coupled receptor protein include peptides, proteins, nonpeptidic compounds, synthetic compounds, fermented products, etc.

In more specific embodiments of the present invention, said methods for screening and identifying a ligand includes:

1) a method of screening for a ligand to a G protein coupled receptor protein, which comprises contacting a labeled test compound with a G protein coupled receptor protein or a salt thereof or its partial peptide or a salt thereof, and measuring the amount of the labeled test compound binding with said protein or salt thereof or with said partial peptide or salt thereof;

2) a method of screening for a ligand to a G protein coupled receptor protein, which comprises contacting a labeled test compound with cells containing the G protein coupled receptor protein or the membrane fraction of said cell, and measuring the amount of the labeled test compound binding with said cells or said membrane fraction, in such methods the DNA can be used to prepare the cells or cell membranes containing the G protein or fragment thereof;

3) a method of screening for a ligand to a G protein coupled receptor protein, which comprises contacting a labeled test compound with the G protein coupled receptor protein expressed on cell membranes by culturing transformants carrying the G protein coupled receptor protein-encoding DNA and measuring the amount of the labeled test compound binding with said G protein coupled receptor protein;

4) a method of screening for a ligand to a G protein coupled receptor protein, which comprises contacting a test compound with cells containing the G protein coupled receptor protein, and measuring the cell stimulating activity, e.g. promoting or inhibiting activity on biological responses such as liberation of arachidonic acid metabolites, liberation of acetylcholine, increase of intracellular $Ca^{2+}$, production of cAMP, production of cGMP, production of inositol phosphate, changes in the cell membrane potential, phosphorylation of intracellular protein, activation of c-fos, lowering in extracellular pH, etc. via the G protein coupled receptor protein; and

5) a method of screening for a ligand to the G protein coupled receptor protein, which comprises contacting a test compound with the G protein coupled receptor protein expressed on the cell membrane by culturing transformants carrying the G protein coupled receptor protein-encoding DNA, and measuring at least one cell stimulating activity, e.g., an activity for promoting or inhibiting physiological responses such as liberation of arachidonic acid metabolites, liberation of acetylcholine, increase of intracellular $Ca^{2+}$, production of cAMP, production of cGMP, production of inositol phosphate, changes in the cell membrane potential, phosphorylation of intracellular protein, activation of c-fos, lowering in extracellular pH etc. via the G protein coupled receptor protein.

Described below are specific illustrations of the method for screening and identifying ligands.

First, the G protein coupled receptor protein used for the method for determining the ligand may include any material so far as it contains a G protein coupled receptor protein, a partial peptide thereof or a salt thereof described above although it is preferable to express large amounts of the G protein coupled receptor proteins in animal cells.

In the manufacture of the G protein coupled receptor protein, the above-mentioned method can be used and carried out by expressing said protein encoding DNA in mammalian cells or in insect cells. With respect to the DNA fragment coding for a particular region such as an extracellular epitope, the extracellular domains, etc., complementary DNA may be used although the method of expression is not limited thereto. For example, gene fragments or synthetic DNA may be used as well.

In order to introduce the G protein coupled receptor protein-encoding DNA fragment into host animal cells and to express it efficiently, it is preferred that said DNA fragment is incorporated into the downstream side of polyhedron promoters derived from nuclear polyhedrosis virus belonging to baculovirus, promoters derived from SV40, promoters derived from retrovirus, metallothionein promoters, human heat shock promoters, cytomegalovirus promoters, SR$\alpha$ promoters, etc. Examinations of the quantity and the quality of the expressed receptor can be carried out by methods per se known to those of skill in the art or methods similar thereto based upon the present disclosure. For example, they may be conducted by methods described in publications such as Nambi, P. et al: The Journal of Biochemical Society, vol.267, pages 19555-19559 (1992).

Accordingly, with respect to the determination of the ligand, the material containing a G protein coupled receptor protein or partial peptide thereof may include products containing G protein coupled receptor proteins which are purified by methods per se known to those of skill in the art or methods similar thereto, peptide fragments of said G protein coupled receptor protein, cells containing said G protein coupled receptor protein, membrane fractions of the cell containing said protein, etc.

When the G protein coupled receptor protein-containing cell is used in the determining method of the ligand, said cell may be immobilized with binding agents including glutaraldehyde, formalin, etc. The immobilization may be carried out by methods per se known to those of skill in the art or methods similar thereto.

The G protein coupled receptor protein-containing cells are host cells which express the G protein coupled receptor protein. Examples of said host cells are microorganisms such as Escherichia coli, Bacillus subtilis, yeasts, insect cells, animal cells, etc.

The cell membrane fraction is a cell membrane-rich fraction which is prepared by methods per se known to those of skill in the art or methods similar thereto after disruption of cells. Examples of cell disruption may include a method for squeezing cells using a Potter-Elvehjem homogenizer, a disruption by a Waring blender or a Polytron homogenizer manufactured by Kinematica, a disruption by ultrasonic waves, a disruption via blowing out cells from small nozzles together with applying a pressure using a French press or the like, etc. In the fractionation of the cell membrane, a fractionation method by means of centrifugal force such as a fractional centrifugal separation and a density gradient centrifugal separation is mainly used. For example, disrupted cellular liquid is centrifuged at a low speed (500 rpm to 3,000 rpm) for a short period (usually, from about one to ten minutes), the supernatant liquid is further centrifuged at a high speed (15,000 rpm to 30,000 rpm) usually for 30 minutes to two hours and the resulting precipitate is used as a membrane fraction. Said membrane fraction contains a lot of the expressed G protein coupled receptor protein and a lot of membrane components such as phospholipids and membrane proteins derived from the cells.

The amount of the G protein coupled receptor protein in the membrane fraction cell containing said G protein coupled receptor protein is preferably $10^3$ to $10^8$ molecules per cell or, more preferably, $10^5$ to $10^7$ molecules per cell. Incidentally, the greater the expressed amount, the higher the ligand binding activity (specific activity) per membrane fraction whereby the construction of a highly sensitive screening system becomes possible and, moreover, it permits measurement of a large amount of samples within the same lot.

In conducting the above-mentioned methods 1) to 3) wherein ligands capable of binding with the G protein coupled receptor protein are determined, a suitable G protein coupled receptor fraction and a labeled test compound are necessary. The G protein coupled receptor fraction is preferably a naturally occurring (natural type) G protein coupled receptor, a recombinant G protein coupled receptor having the activity equivalent to that of the natural type. Here, the term "activity equivalent to" means the equivalent ligand binding activity, etc. as discussed above.

Suitable examples of the labeled test compound include above-mentioned compound to be tested which are labeled with [$^3$H], [$^{125}$I], [$^{14}$C], [$^{35}$S], etc.

Specifically, the determination of ligands capable of binding with G protein coupled receptor proteins is carried out as follows:

First, cells or cell membrane fractions containing the G protein coupled receptor protein are suspended in a buffer suitable for the assay to prepare the receptor sample for conducting the method of determining the ligand binding with the G protein coupled receptor protein. The buffer may include any buffer such as Tris-HCL buffer or phosphate buffer with pH 4-10, preferably, pH 6-8, etc., as long as it does not inhibit the binding of the ligand with the receptor. In addition, detergents such as CHAPS, Tween 80™ (Kao-Atlas, Japan), digitonin, deoxycholate, etc. and various proteins such as bovine serum albumin (BSA), gelatin, milk derivatives, etc. may be added to the buffer with an object of descreasing the non-specific binding. Further, a protease inhibitor such as PMSF, leupeptin, E-64 (manufactured by Peptide Laboratory), pepstatin, etc. may be added with an object of inhibiting the decomposition of the receptor and the ligand by protease. A test compound labeled with a predetermined (or certain) amount (5,000 cpm to 500,000 cpm) of [$^3$H], [$^{125}$I], [$^{14}$C], [$^{35}$S], etc. coexists in 0.01 ml to 10 ml of said receptor solution. In order to know the non-specific binding amount (NSB), a reaction tube to which a great excessive amount of the unlabeled test compound is added is prepared as well. The reaction is carried out at 0-50°C, preferably at 4-37°C for 20 minutes to 24 hours, preferably 30 minutes to three hours. After the reaction, it is filtered through a glass fiber filter or the like, washed with a suitable amount of the same buffer and the radioactivity remaining in the glass fiber filter is measured by means of a liquid scintillation counter or a gamma-counter. The test compound in which the bound (B - NSB) obtained by subtracting the non-specific binding amount (NSB) from the total binding amount (B) is more than 0 cpm is identified as a ligand to the G protein coupled receptor protein.

In conducting the above-mentioned methods 4) to 5) wherein ligands capable of binding with the G protein coupled receptor protein are determined, the cell stimulating activity, e.g. the liberation of arachidonic acid metabolites, the liberation of acetylcholine, increase of intracellular $Ca^{2+}$, production of cAMP, production of inositol phosphate, changes in the cell membrane potential, phosphorylation of intracellular protein, activation of c-fos, lowering in extracellular pH, activation of G protein, cell proliferation, etc.; mediated by the G protein coupled receptor protein may be measured by known methods or by the use of commercially available measuring kits. To be more specific, G protein coupled receptor protein-containing cells are at first cultured in a multi-well plate or the like.

In conducting the determination of ligand, it is substituted with a fresh medium or a suitable buffer which does not show toxicity to the cells in advance of the experiment, and incubated under appropriate conditions and for sufficient time after adding a test compound, etc. thereto. Then, the cells are extracted or the supernatant liquid is recovered and the resulting product is determined by each of the methods. When it is difficult to identify the production of the substance, e.g. arachidonic acid, etc. which is to be an index for the cell stimulating activity due to the degradating enzyme contained in the cell, an assay may be carried out by adding an inhibitor against said enzyme. With respect to an activity such as an inhibitory action against cAMP production, it may be detected as an inhibitory action against the production of the cells treated with forskolin or the like to increase cAMP production.

The kit used for the method of determining the ligand binding with the G protein coupled receptor protein includes a G protein coupled receptor protein or a partial peptide thereof, cells containing the G protein coupled receptor protein, a membrane fraction from the cells containing the G protein coupled receptor protein, etc.

Examples of kits for determining the ligand are as follows. The kits preferably contain the various ingredients in vials. The G protein conpled receptor protein and/or ligand may be lyophilyed to increase storage life. Instructions describing the above-assay may be included:

1. Reagent for Determining the Ligand.

1) Buffer for Measurement and Buffer for Washing.

The buffering product wherein 0.05% of bovine serum albumin (manufactured by Sigma) is added to Hanks' Balanced Salt Solution (manufactured by Gibco).

This product may be sterilized by filtration through a membrane filter with a 0.45 µm pore size, and stored at 4°C or may be formulated upon use.

2) G Protein Coupled Receptor Protein Sample.

CHO cells in which G protein coupled receptor proteins are expressed are subcultured at the rate of $5 \times 10^5$ cells/well in a 12-well plate and cultured at 37°C in a humidified 5% $CO_2$/95% air atmosphere for two days to prepare the sample.

3) Labeled Test Compound.

The compound which is labeled with commercially available [$^3$H], [$^{125}$I], [$^{14}$C], [$^{35}$S], etc. or labeled with a suitable method.

The product in a state of an aqueous solution is stored at 4°C or at -20°C and, upon use, diluted to 1 µM with a buffer for the measurement. In the case of a test compound which is barely soluble in water, it may be dissolved in an organic solvent such as dimethylformamide, DMSO, methanol and the like.

4) Unlabeled Test Compound.

The same compound as the labeled one is prepared in a concentration of 100 to 1,000-fold concentrated state.

2. Method of Measurement

1) G protein coupled receptor protein-expressing CHO cells cultured in a 12-well tissue culture plate are washed twice with 1 ml of buffer for the measurement and then 490 µl of buffer for the measurement is added to each well.
2) Five µl of the labeled test compound is added and the mixture is made to react at room temperature for one hour. For measuring the nonspecific binding amount, 5µl of the unlabeled test compound is added.
3) The reaction solution is removed from each well, which is washed with 1 ml of a buffer for the measurement three times. The labeled test compound which is binding with the cells is dissolved in 0.2N NaOH-1% SDS and mixed with 4 ml of a liquid scintillator A manufactured by WAKO Pure Chemical, Japan.

4) Radioactivity is measured using a liquid scintillation counter such as one manufactured by Beckmann.

The ligand which can bind with the G protein coupled receptor protein include substances occuring or existing, for example, in brain, pituitary gland etc. Examples of the ligand are angiotensin, bombesin, canavinoid, cholecystokinin, glutamine, serotonin, melatonin, neuropeptide Y, opioids, purine, vasopressin, oxytocin, VIP (vasoactive intestinal and related peptides), somatostatin, dopamine, motilin, amylin, bradykinin, CGRP (calcitonin gene related peptides), leukotriene, pancreastatin, prostaglandin, thromboxane, adenosine, adrenaline, $\alpha$- and $\beta$-chemokines such as IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.; endothelin, enterogastrin, histamine, neurotensins, TRH, pancreatic polypeptide, etc.

(2) Prophylactic and Therapeutic Agent for G Protein Coupled Receptor Protein Deficiency Diseases

If a ligand to the G protein coupled receptor protein is revealed via the aforementioned method (1), the G protein coupled receptor protein-encoding DNA can be used as a prophylactic and/or therapeutic agent for treating said G protein coupled receptor protein deficiency diseases depending upon the action that said ligand exerts.

For example, when there is a patient for whom the physiological action of the ligand cannot be expected because of a decrease in the G protein coupled receptor protein in vivo, the amount of the G protein coupled receptor protein in the brain cells of said patient can be increased whereby the action of the ligand can be fully achieved by:

(a) administering the G protein coupled receptor protein-encoding DNA to the patient to express it; or
(b) inserting the G protein coupled receptor protein-encoding DNA into brain cells or the like to said patient. Accordingly, the G protein coupled receptor protein-encoding DNA can be used as a safe and less toxic preventive and therapeutic agent for the G protein coupled receptor protein deficiency diseases.

When the above-mentioned DNA is used as the above-mentioned agent, said DNA may be used alone or after inserting it into a suitable vector such as retrovirus vector, adenovirus vector, adenovirus-associated virus vector, pox virus etc. followed by subjecting the product vector to a conventional means. The DNA can also be administered as "naked" DNA, with adjuvants to assist in uptake, by "gene" gun or by a catheter such as a catheter with a hydrogel. For example, it can be used orally in the form of tablets which may be sugar coated as necessary, capsules, elixirs, microcapsules etc., or non-orally in the form of injectable preparations such as aseptic solutions and suspensions in water or other pharmaceutically acceptable liquids. These preparations can be produced by mixing the DNA of the present invention with physiologically acceptable carriers, flavoring agents, excipients, vehicles, antiseptics, stabilizers, binders etc. in unit dosage forms required for generally accepted manners of pharmaceutical making. Active ingredient contents in these preparations are set so that an appropriate dose within the specified range is obtained.

Additives which can be mixed in tablets, capsules etc. include binders such as gelation, corn starch, tragacanth and gum arabic, excipients such as crystalline cellulose, swelling agents such as corn starch, gelatin and alginic acid, lubricants such as magnesium stearate, sweetening agents such as sucrose, lactose and saccharin, and flavoring agents such as peppermint, akamono oil and cherry. When the unit dosage form is the capsule, the above-mentioned materials may further incorporate liquid carriers such as oils and fats. Sterile compositions for injection can be formulated by ordinary methods of pharmaceutical making such as by dissolving or suspending active ingredients, naturally occuring vegetable oils such as sesame oil and coconut oil, etc. in vehicles such as water for injection to create pharmaceutical compositions.

Aqueous liquids for injection include physiological saline and isotonic solutions containing glucose and other auxiliary agents, e.g., D-sorbitol, D-mannitol and sodium chloride, and may be used in combination with appropriate dissolution aids such as alcohols, e.g., ethanol, polyalcohols, e.g., propylene glycol and polyethylene glycol, nonionic surfactants, e.g., polysorbate 80 (TM) and HCO-50 etc. Oily liquids include sesame oil and soybean oil, and may be used in combination with dissolution aids such as benzyl benzoate and benzyl alcohol. Furthermore the above-mentioned materials may also be formulated with buffers, e.g., phosphate buffer and sodium acetate buffer; soothing agents, e.g., benzalkonium chloride, procaine hydrochloride; stabilizers, e.g., human serum albumin, polyethylene glycol; preservatives, e.g., benzyl alcohol, phenol; antioxidants etc. The thus-prepared pharmaceutical composition such as an injectable liquid is normally filled in an appropriate ampule. Because the thus-obtained preparation is safe and of low toxicity, it can be administered to humans or warm-blooded mammals, e.g., rats, rabbits, sheep, pigs, bovines, cats, dogs, monkeys, for instance.

The dose of said DNA is normally about 0.1-100 mg, preferably 1.0-50 mg, and more preferably 1.0-20 mg per day for an adult (weighing 60 kg) in oral administration, depending on symptoms etc. In non-oral administration, it is advantageous to administer the DNA in the form of injectable preparation at a daily dose of about 0.01-30 mg, preferably about 0.1-20 mg, and more preferably about 0.1-10 mg per administration by an intravenous injection for an adult (weighing 60 kg), depending on subject of administration, target organ, symptoms, method of administration etc. For other animal species, corresponding does as converted per 60 kg weight can be administered.

If one wishes to use the G protein coupled receptor protein or fragments thereof, one would use it in a purified form, preferably at least 90% pure, more preferably at least 95% pure, still more preferably at least 98% pure and most preferably at least 99% pure. The protein preparations can be prepared in the same manner as described above for DNA. Thus, for example, a pharmaceutical composition containing an aqueous liquid such as physiological saline can be used. The protein can be administered in the same way as the DNA and the dosage ranges are the same.

(3) Quantitative Determination of the Ligand to the G Protein Coupled Receptor Protein

The G protein coupled receptor protein that has a binding property for a ligand is capable of determining quantitatively an amount of a ligand in vivo with good sensitivity.

This quantitative determination may be carried out by, for example, combining with a competitive analysis. Thus, a sample to be determined is contacted with the G protein coupled receptor protein or a partial peptide thereof so that the concentration of a ligand in said sample can be determined. In one embodiment of the quantitative determination, the protocols described in the following 1) and 2) or methods similar thereto may be used:

1) Hiroshi Irie (ed): "Radioimmunoassay" (Kodansha, Japan, 1974); and
2) Hiroshi Irie (ed): "Radioimmunoassay, Second Series" (Kodansha, Japan, 1979).

(4) Screening of Compound Changing the Binding Activity of G protein coupled receptor protein with the Ligand

G Protein coupled receptor proteins or partial peptide or salt thereof of the present invention can be used. Alternatively, expression systems for recombinant G Protein coupled receptor proteins are constructed and receptor binding assay systems using said expression system are used. In these assay systems, it is possible to screen compounds, e.g. peptides, proteins, nonpeptidic compounds, synthetic compounds, fermentation products, cell extracts, animal tissue extracts, etc.; or salts thereof which changes the binding activity of a ligand with the G protein coupled receptor protein. Such a compound includes a compound exhibiting a G protein coupled receptor-mediated cell stimulating activity, e.g. activity of promoting or activity of inhibiting physiological reactions including liberation of arachidonic acid metabolites, liberation of acetylchloline, increase of intracellular $Ca^{2+}$, production of cAMP, production of cGMP, production of inositol phosphate, changes in cell membrane potential, phosphorylation of intracellular protein, activation of c-fos, lowering in extracellular pH, etc.; so-called "G protein coupled receptor-agonist", a compound free from such a cell stimulating activity, so-called "G protein coupled receotor-antagonist", etc. The term of "change the binding activity of a ligand" includes both the case in which the binding of ligand is inhibited and the case in which the binding of ligand is promoted.

Thus, the present invention provides a method of screening for a compound which changes the binding activity of a ligand with a G protein coupled receptor protein or a salt thereof, characterized by comparing the following two cases:

(i) the case wherein the ligand is contacted with the G protein coupled receptor protein or salt thereof, or a partial peptide thereof or a salt thereof; and
(ii) the case wherein the ligand is contacted with a mixture of the G protein coupled receptor protein or salt thereof or the partial peptide or salt thereof and said test compound.

In said screening method, one characteristic feature of the present invention resides in that the amount of the ligand bound with said G protein coupled receptor protein or the partial peptide thereof, the cell stimulating activity of the ligand, etc. are measured in both the case where (i) the ligand is contacted with G protein coupled receptor proteins or partial peptide thereof and in the case where (ii) the ligand and the test compound are contacted with the G protein coupled receptor protein or the partial peptide thereof, respectively and then compared therebetween.

In one more specific embodiment of the present invention, the following is provided:

1) a method of screening for a compound or a salt thereof which changes the binding activity of a ligand with a G protein coupled receptor protein, characterized in that, when a labeled ligand is contacted with a G protein coupled receptor protein or a partial peptide thereof and when a labeled ligand and a test compound are contacted with a G protein coupled receptor protein or a partial peptide thereof, the amounts of the labeled ligand bound with said protein or a partial peptide thereof or a salt thereof are measured and compared;
2) a method of screening for a compound or a salt thereof which changes the binding activity of a ligand with a G protein coupled receptor protein, characterized in that, when a labeled ligand is contacted with cells containing G protein coupled receptor proteins or a membrane fraction of said cells and when a labeled ligand and a test compound are contacted with cells containing G protein coupled receptor proteins or a membrane fraction of said cells, the amounts of the labeled ligand binding with said protein or a partial peptide thereof or a salt thereof are measued and compared;

3) a method of screening for a compound or a salt thereof which changes the binding activity of a ligand with a G protein coupled receptor protein, characterized in that, when a labeled ligand is contacted with G protein coupled receptor proteins expressed on the cell memberane by culturing a transformant carrying a G protein coupled receptor protein-encoding DNA and when a labeled ligand and a test compound are contacted with G protein coupled receptor proteins expressed on the cell membrane by culturing a transformant carrying a G protein coupled receptor protein-encoding DNA, the amounts of the labeled ligand binding with said G protein coupled receptor protein are measured and compared;

4) a method of screening for a compound or a salt thereof which changes the binding activity of a ligand with a G protein coupled receptor protein, characterized in that, when a G protein coupled receptor protein-activating compound is contacted with cells containing G protein coupled receptor proteins and when the G protein coupled receptor protein-activating compound and a test compound are contacted with cells containing G protein coupled receptor proteins, the resulting G protein coupled receptor protein-mediated cell stimulting activities, e.g. activities of promoting or activities of inhibiting physiological responses including liberation of arachidonic acid metabolites, liberation of acetylcholine, increase of intracellular $Ca^{2+}$, production of cAMP, production of cGMP, production of inositol phosphate, changes in the cell membrane potential, phosphorylation of intracellular protein, activation of c-fos, lowering in extracellular pH etc.; are measured and compared; and

5) a method of screening for a compound or a salt thereof which changes the binding activity of a ligand with a G protein coupled receptor protein, characterized in that, when a G protein coupled receptor protein-activating compound is contacted with G protein coupled receptor proteins expressed on cell membranes by culturing transformants carrying G protein coupled receptor protein-encoding DNA and when a G protein coupled receptor protein-activating compound and a test compound are contacted with the G protein coupled receptor protein expressed on the cell membrane by culturing the transformant carrying the G protein coupled receptor protein-encoding DNA, the resulting G protein coupled receptor protein-mediated cell stimulating activities, e.g. activities of promoting or activities of inhibiting physiological responses such as liberation of arachidonic acid metabolites, liberation of acetylcholine, increase of intracellular $Ca^{2+}$, production of cAMP, production of cGMP, production of inositol phosphate, changes in the cell membrane potential, phosphorylation of intracellular protein, activation of c-fos, lowering in extracellular pH etc.; are measured and compared.

The G protein coupled receptor agonist or antagonist have to be screened by, first, obtaining a candidate compound by using G protein coupled receptor protein-containing cells, tissues or cell membrane fractions derived from rat or mouse or the like (primary screening), then, making sure whether the candidate compound really inhibitis the binding between human G protein coupled receptor proteins and ligands (secondary screening). Other receptor proteins inevitably exist and when the cells, the tissues or the cell membrane fractions were used, they intrinsically make it difficult to screen agonists or antagonists to the desired receptor proteins. By using the human-derived G protein coupled receptor protein, however, there is no need of effecting the primary screening, whereby it is possible to efficiently screen a compound that changes the binding activity between a ligand and a G protein coupled receptor. Additionally, it is possible to evaluate whether the compound that is screened is a G protein coupled receptor agonist or a G protein coupled receptor antagonist. When the G protein coupled receptor protein agonist or the G protein coupled receptor protein antagonist is researched and developed as a medicament for humans, it is necessary to be screened out by using human-derived receptor proteins. Because the different effects of the compounds which is obtained by the screening are caused by species differences of the receptor theirselves.

A compound which has strong agonist or antagonist activity may obtain by studying the correlation between the chemical structure of the compound and the activity, and designing or modifying the compound. In such the case, if a human-derived receptor can be used, designing and evaluation of a suitable compound which is effective as a medicament for human can be carried out easily. However, if an animal derived receptor is used for screening of the agonist or antagonist, the compound having strong activity for human can not be obtained generally.

Specific explanations of the screening method will be given as hereunder.

First, with respect to the G protein coupled receptor protein used for the screening method of the present invention, any product may be used so long as it contains G protein coupled receptor proteins including the substituted, deleted or added proteins or partial peptides thereof although the use of a membrane fraction of mammalian organs is preferable. However, human organs can be extremely scarce and, accordingly, G protein coupled receptor proteins which are expressed in a large amount using a recombinant technique are preferable for the screening.

In the manufacture of the G protein coupled receptor protein, the above-mentioned method can be used.

In the manufacture of the G protein coupled receptor protein, the above-mentioned method can be used and carried out by expressing said protein encoding DNA in mammalian cells or in insect cells. With respect to the DNA fragment coding for a particular region such as an extracellular epitope, the extracellular domains, etc., complementary DNA may be used although the method of expression is not limited thereto. For example, gene fragments or synthetic DNA may be used as well.

In order to introduce the G protein coupled receptor protein-encoding DNA fragment into host animal cells and to express it efficiently, it is preferred that said DNA fragment is incorporated into the downstream side of polyhedron promoters derived from nuclear polyhedrosis virus belonging to baculovirus, promoters derived from SV40, promoters derived from retrovirus, metallothionein promoters, human heat shock promoters, cytomegalovirus promoters, SRα promoters, etc. Examinations of the quantity and the quality of the expressed receptor can be carried out by methods per se known to those of skill in the art or methods similar thereto based upon the present disclosure. For example, they may be conducted by methods described in publications such as Nambi, P. et al: The Journal of Biochemical Society, vol.267, pages 19555-19559 (1992).

Accordingly, with respect to the determination of the ligand, the material containing a G protein coupled receptor protein or partial peptide thereof may include products containing G protein coupled receptor proteins which are purified by methods per se known to those of skill in the art or methods similar thereto, peptide fragments of said G protein coupled receptor protein, cells containing said G protein coupled receptor protein, membrane fractions of the cell containing said protein, etc.

When the G protein coupled receptor protein-containing cell is used in the determining method of the ligand, said cell may be immobilized with the agents including glutaraldehyde, formalin, etc. The immobilisation may be carried out by methods per se known to those of skill in the art or methods similar thereto.

The G protein coupled receptor protein-containing cells are host cells which express the G protein coupled receptor protein. Examples of said host cells are microorganisms such as Escherichia coli, Bacillus subtilis, yeasts, insect cells, animal cells, etc.

The cell membrane fraction is a cell membrane-rich fraction which is prepared by methods per se known to those of skill in the art or methods similar thereto after disruption of cells. Examples of cell disruption may include a method for squeezing cells using a Potter-Elvehjem homogenizer, a disruption by a Waring blender or a Polytron manufactured by Kinematica, a disruption by ultrasonic waves, a disruption via blowing out cells from small nozzles together with applying a pressure using a French press or the like, etc. In the fractionation of the cell membrane, a fractionation method by means of centrifugal force such as a fractional centrifugal separation and a density gradient centrifugal separation is mainly used. For example, disrupted cellular liquid is centrifuged at a low speed (500 rpm to 3,000 rpm) for a short period (usually, from about one to ten minutes), the supernatant liquid is further centrifuged at a high speed (15,000 rpm to 30,000 rpm) usually for 30 minutes to two hours and the resulting precipitate is used as a membrane fraction. Said membrane fraction contains a lot of the expressed G protein coupled receptor protein and a lot of membrane components such as phospholipids and membrane proteins derived from the cells.

The amount of the G protein coupled receptor protein in the membrane fraction cell containing said G protein coupled receptor protein is preferably $10^3$ to $10^8$ molecules per cell or, more preferably, $10^5$ to $10^7$ molecules per cell. Incidentally, the greater the expressed amount, the higher the ligand binding activity (specific activity) per membrane fraction whereby the construction of a highly sensitive screening system becomes possible and, moreover, it permits measurement of a large amount of samples within the same lot.

In conducting the above-mentioned methods 1) to 3) for screening the compound capable of changing the binding activity of the ligand with the G protein coupled receptor protein, a suitable G protein coupled receptor fraction and a labeled ligand are necessary. With respect to the G protein coupled receptor fraction, it is preferred to use naturally occurring G protein coupled receptors (natural type G protein coupled receptors) or recombinant type G protein coupled receptor fractions with the activity equivalent to that of the natural type G protein coupled. Here the term "activity equivalent to" means the same ligand binding activity, or the substantially equivalent ligand binding activity.

With respect to the labeled ligand, it is possible to use labeled ligands, labeled ligand analogized compounds, etc. For example, ligands labeled with [$^3$H], [$^{125}$I], [$^{14}$C], [$^{35}$S], etc. and other labeled substances may be utilized.

Specifically, G protein coupled receptor protein-containing cells or cell membrane fractions are first suspended in a buffer which is suitable for the selected method to prepare the receptor sample in conducting the screening for a compound which changes the binding activity of the ligand with the G protein coupled receptor protein. With respect to the buffer, any buffer such as Tris-HCl buffer or phosphate buffer of pH 4-10, preferably, pH 6-8 which does not inhibit the binding of the ligand with the receptor may be used.

In addition, a surface-active agent such as CHAPS, Tween 80™ (Kao-Atlas, Japan), digitonin, deoxycholate, etc. may be added to the buffer with an object of decreasing the nonspecific binding. Further, a protease inhibitor such as PMSF, leupeptin, E-64 manufactured by Peptide Laboratory, Japan, pepstatin, etc. may be added with the object of inhibiting the decomposition of the receptor and the ligand by protease. A labeled ligand in a certain amount (5,000 cpm to 500,000 cpm) is added to 0.01 ml to 10 ml of said receptor solution and, at the same time, $10^{-4}$M to $10^{-10}$M of a test compound coexists. In order to determine the nonspecific binding amount (NSB), a reaction tube to which a large excessive amount of unlabeled test compounds is added is prepared as well.

The reaction is carried out at 0-50°C, preferably at 4-37°C for 20 minutes to 24 hours, preferably 30 minutes to three hours. After the reaction, it is filtered through a glass fiber filter, a filter paper, or the like, washed with a suitable amount of the same buffer and the radioactivity retained in the glass fiber filter, etc. is measured by means of a liquid scintillation counter or a gamma-counter. Supposing that the count ($B_0$ - NSB) obtained by subtracting the nonspecific binding amount (NSB) from the total binding amount ($B_0$) wherein an antagonizing substance is not present is set at 100%, a test compound in which the specific binding amount (B - NSB) obtained by subtracting the nonspecific binding amount (NSB) from the total binding amount (B) is, for example, less than 50% may be selected as a candidate ligand to the G protein coupled receptor protein of the present invention.

In conducting the above-mentioned methods 4) to 5) for screening the compound which changes the binding activity of the ligand with the G protein coupled receptor protein, the G protein coupled receptor protein-mediated cell stimulating activity, e.g. activities of promoting or activities of inhibiting physiological responses such as liberation of arachidonic acid metabolites, liberation of acetylcholine, increase of intracellular $Ca^{2+}$, production of cAMP, production of inositol phosphate, changes in the cell membrane potential, phosphorylation of intracellular protein, activation of c-fos, lowering in extracellular pH etc.; may be measured by known methods or by the use of commercially available measuring kits. To be more specific, G protein coupled receptor protein-containing cells are at first cultured in a multi-well plate or the like.

In conducting the screening, it is substituted with a suitable buffer which does not show toxicity to fresh media or cells in advance, incubated under appropriate conditions and for a specified time after adding a test compound, etc. thereto. The resultant cells are extracted or the supernatant liquid is recovered and the resulting product is determined, preferably quantitatively, by each of the methods. When it is difficult to identify the production of the index substance, e.g. arachidonic acid, etc. which is to be an index for the cell stimulating activity due to the presence of decomposing enzymes contained in the cell, an assay may be carried out by adding an inhibitor against said decomposing enzyme. With respect to activities such as an inhibitory action against cAMP production, it may be detected as an inhibitory action against the cAMP production in the cells whose fundamental production has been increased by forskolin or the like.

In conducting screening by measuring the cell stimulating activity, cells in which a suitable G protein coupled receptor protein is expressed are necessary. Preferred G protein coupled receptor protein-expressing cells are naturally occurring G protein coupled receptor protein (natural type G protein coupled receptor protein)-containing cell lines or strains, e.g. mouse T cell WEHI-7TG, etc., the above-mentioned recombinant type G protein coupled receptor protein-expressing cell lines or strains, etc.

Examples of the test compound includes peptide, proteins, non-peptidic compounds, synthesized compounds, fermentation products, cell extracts, plant extracts, animal tissue extracts, serum, blood, body fluid, etc. Those compounds may be novel or known.

A kit for screening a compound which changes the binding activity of the ligand with the G protein coupled receptor protein or a salt thereof comprises a G protein coupled receptor protein or a partial peptide thereof, or G protein coupled receptor protein-containing cells or cell membrane fraction thereof.

Examples of the screening kit include as follows:

1. Reagent for Screening.

1) Buffer for Measurement and Buffer for Washing.

The product wherein 0.05% of bovine serum albumin (manufactured by Sigma) is added to Hanks' Balanced Salt Solution (manufactured by Gibco).

This may be sterilized by filtration through a membrane filter with a 0.45 μm pore size, and stored at 4°C or may be prepared upon use.

2) Sample of G Protein Coupled Receptor Protein.

CHO cells in which a G protein coupled receptor protein is expressed are subcultured at the rate of $5 \times 10^5$ cells/well in a 12-well plate and cultured at 37°C with a 5% $CO_2$ and 95% air atmosphere for two days to prepare the sample.

3) Labeled Ligand.

The ligand which is labeled with commercially available [³H], [¹²⁵I], [¹⁴C], [³⁵S], etc.

The product in a state of an aqueous solution is stored at 4°C or at -20°C and, upon use, diluted to 1 μM with a buffer for the measurement.

4) Standard Ligand Solution.

Ligand is dissolved in PBS containing 0.1% of bovine serum albumin (manufactured by Sigma) to make 1 mM and stored at -20°C.

2. Method of Measurement.

1) CHO cells are cultured in a 12-well tissue culture plate to express G protein coupled receptor proteins. The G protein coupled receptor protein-expressing CHO cells are washed with 1 ml of buffer for the measurement twice. Then 490 μl of buffer for the measurement is added to each well.

2) Five μl of a test compound solution of $10^{-3}$ to $10^{-10}$ M is added, then 5 μl of labeled ligand is added and is made to react at room temperature for one hour. For knowing the non-specific binding amount, 5 μl of the ligand of $10^{-3}$ M is added instead of the test compound.

3) The reaction solution is removed from the well, which is washed with 1 ml of buffer for the measurement three times. The labeled ligand binding with the cells is dissolved in 0.2N NaOH-1% SDS and mixed with 4 ml of a liquid scintillator A (such as manufactured by Wako Pure Chemical, Japan).

4) Radioactivity is measured using a liquid scintillation counter (e.g., one manufactured by Beckmann) and PMB (percent maximum binding) is calculated by the following equation:

$$PMB = [(B - NSB)/(B_0 - NSB)] \times 100$$

PMB:   Percent maximum binding
B:      Value when a sample is added
NSB:   Nonspecific binding
$B_0$:     Maximum binding

The compound or a salt thereof obtained by the screening method or by the screening kit is a compound which changes the binding activity of a ligand with a G protein coupled receptor protein, wherein the compound inhibits or promotes the binding, and, more particularly, it is a compound having a cell stimulating activity mediated via a G protein coupled receptor or a salt thereof, so-called "G protein coupled receptor agonist" or a compound having no said stimulating activity, so-called "G protein coupled receptor antagonist". Examples of said compound are peptides, proteins, non-peptidic compounds, synthesized compounds, fermented products, etc. and the compound may be novel or known.

Said G protein coupled receptor agonist has the same physiological action as the ligand to the G protein coupled receptor protein has and, therefore, it is useful as a safe and less toxic pharmaceutical composition depending upon said ligand activity.

On the other hand, said G protein coupled receptor antagonist is capable of inhibiting the physiological activity of the ligand to the G protein coupled receptor protein and, therefore, it is useful as a safe and less toxic pharmaceutical composition for inhibiting said ligand activity.

The above-mentioned G protein coupled receptor agonist or antagonist can be used as a prophylactic and therapeutic agent for such diseases as spinal injury, Alzheimer's disease, asthma, hyperphagia, neuropathy, dementia, pain, cerebral thrombosis, encephalitis, cerebral infarction, cerebrovascular spasm, spondylitis, angina pectoris, neurosis, opiate dependence, opiate withdrawal syndrome, schizophrenia, phobia, cerebral ischemic stroke, cocaine dependence, cocaine withdrawal syndrome, anxiety disorders, depression, respiratory distress syndrome, alcohol withdrawal syndrome, vomiting, epilepsy, etc.

When the compound or the salt thereof obtained by the screening method or by the using screening kit is used as a pharmaceutical composition as described above, it can be used by conventional methods. For example, it can be used orally in the form of tablets which may be sugar coated as necessary, capsules, elixirs, microcapsules etc., or non-orally in the form of injectable preparations such as aseptic solutions and suspensions in water or other pharmaceutically acceptable liquids. These preparations can be produced by mixing the compound or a salt thereof with physiologically acceptable carriers, flavoring agents, excipients, vehicles, antiseptics, stabilizers, binders etc. in unit dosage forms required for generally accepted manners of pharmaceutical making. Active ingredient contents in these preparations are set so that an appropriate dose within the specified range is obtained.

Additives which can be mixed in tablets, capsules etc. include binders such as gelation, corn starch, tragacanth and gum arabic, excipients such as crystalline cellulose, swelling agents such as corn starch, gelatin and alginic acid, lubricants such as magnesium stearate, sweetening agents such as sucrose, lactose and saccharin, and flavoring agents such as peppermint, akamono oil and cherry. When the unit dosage form is the capsule, the above-mentioned materials may further incorporate liquid carriers such as oils and fats. Sterile compositions for injection can be formulated by ordinary methods of pharmaceutical making such as by dissolving or suspending active ingredients, naturally occuring vegetable oils such as sesame oil and coconut oil, etc. in vehicles such as water for injection.

Aqueous liquids for injection include physiological saline and isotonic solutions containing glucose and other auxiliary agents, e.g., D-sorbitol, D-mannitol and sodium chloride, and may be used in combination with appropriate dissolution aids such as alcohols, e.g., ethanol, polyalcohols, e.g., propylene glycol and polyethylene glycol, nonionic surfactants, e.g., polysorbate 80 (TM) and HCO-50 etc. Oily liquids include sesame oil and soybean oil, and may be used in combination with dissolution aids such as benzyl benzoate and benzyl alcohol. Furthermore the above-mentioned materials may also be formulated with buffers, e.g., phosphate buffer and sodium acetate buffer; soothing agents, e.g., benzalkonium chloride, procaine hydrochloride; stabilizers, e.g., human serum albumin, polyethylene glycol; preservatives, e.g., benzyl alcohol, phenol; antioxidants etc. The thus-prepared injectable liquid is normally filled in an appropriate ampule. Because the thus-obtained preparation is safe and of low toxicity, it can be administered to humans or warm-blooded mammals, e.g., rats, rabbits, sheep, pigs, bovines, cats, dogs, monkeys for instance.

The dose of said compound or a salt thereof is normally about 0.1-100 mg, preferably 1.0-50 mg, and more preferably 1.0-20 mg per day for an adult (weighing 60 kg) in oral administration, depending on symptoms etc. In non-oral administration, it is advantageous to administer the compound or a salt thereof in the form of injectable preparation at a daily dose of about 0.01-30 mg, preferably about 0.1-20 mg, and more preferably about 0.1-10 mg per administration by an intravenous injection for an adult (weighing 60 kg), depending on subject of administration, target organ, symptoms, method of administration etc. For other animal species, corresponding dose as converted per 60 kg weight can be administered.

(5) Manufacture of Antibody or Antiserum against the G Protein Coupled Receptor Protein.

Antibodies, e.g. polyclonal antibody, monoclonal antibody, and antisera against the G protein coupled receptor protein may be manufactured by antibody- or antiserum-manufacturing methods per se known to those of skill in the art or methods similar thereto, using the G protein coupled receptor protein as antigen. For example, polyclonal antibodies can be manufactured by the method as given below.

[Preparation of a polyclonal antibody]

The above-mentioned polypeptide or protein as the antigen is coupled to a carrier protein. The carrier protein may for example be bovine thyroglobulin, bovine serum albumin, bovine gamma-globulin, hemocyanine, or Freund's complete adjuvant (Difco).

The coupling reaction between the antigen protein and the carrier protein can be carried out by the known procedure. The reagent for use in the coupling reaction includes but is not limited to glutaraldehyde and water-soluble carbodiimide. The suitable ratio of the antigen protein to the carrier protein is about 1:1 through about 1:10 and as to the reaction pH, satisfactory results are obtained in many cases when the reaction is carried out around neutral, particularly in the range of pH about 6-8. The reaction time is preferably about 1 to 12 hours in many cases and more preferably about 2 to 6 hours. The conjugate thus obtained is dialyzed against water at about 0 to 18°C in the routine manner and stored frozen or optionally lyophilized and stored.

For the production of a polyclonal antibody, a warm-blooded animal is inoculated with the immunogen produced in the manner described hereinbefore. The warm-blooded animal that can be used for this purpose includes mammalian warm-blooded animals, e.g. rabbit, sheep, goat, rat, mouse, guinea pig, bovine, equine, swine, etc.; and avian species, e.g. chicken, dove, duck, goose, quail, etc. Regarding the methodology for inoculating a warm-blooded animal with the immunogen, the inoculum size of the immunogen may be just sufficient for antibody production. For example, the desired antibody can be produced in many instances by emulsifying 1 mg of the immunogen in 1 ml of saline with Freund's complete adjuvant and injecting the emulsion subcutaneously at the back and hind-limb footpad of rabbits 5 times at 4-week intervals. For harvesting the antibody produced in the warm-blooded animal, for example a rabbit, the blood is withdrawn from the auricular vein usually during day 7 through day 12 after the last inoculation dose and centrifuged to recover an antiserum. For purification, the antiserum is generally subjected to affinity chromatography using a carrier to which each antigen peptide has been conjugated and the adsorbed fraction is recovered to provide a polyclonal antibody.

The monoclonal antibody can be produced by the following method.

[Preparation of Monoclonal Antibody]

(a) Preparation of Monoclonal Antibody-Producing Cells.

The G protein coupled receptor protein is aministered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and two to ten times in total. Examples of the applicable warm-blooded animals are monkeys, rabbits, dogs, guinea pigs, mice, rats, sheep, goats and chickens and the use of mice and rats is preferred.

In the preparation of the cells which produce monoclonal antibodies, an animal wherein the antibody titer is noted is selected from warm-blooded animals (e.g. mice) immunized with antigens, then spleen or lymph node is collected after two to five days from the final immunization and antibody-producing cells contained therein are fused with myeloma cells to give monoclonal antibody-producing hybridomas. Measurement of the antibody titer in antisera may, for example, be carried out by reacting a labeled G protein coupled receptor protein (which will be mentioned later) with the antiserum followed by measuring the binding activity of the labeling agent with the antibody. The operation for fusing may be carried out, for example, by a method of Koehler and Milstein (Nature, 256, 495, 1975), Examples of the fusion accelerator are polyethylene glycol (PEG), Sendai virus, etc. and the use of PEG is preferred.

Examples of the myeloma cells are NS-1, P3U1, SP2/0, AP-1, etc. and the use of P3U1 is preferred. The preferred fusion ratio of the numbers of antibody-producing cells used (spleen cells) to the numbers of myeloma cells is within a range of about 1:1 to 20:1. When PEG (preferably, PEG 1000 to PEG 6000) is added in a concentration of about 10-80% followed by incubating at 20-40°C (preferably, at 30-37°C) for one to ten minutes, an efficient cell fusion can be carried out.

Various methods may be applied for screening a hybridoma which produces anti-G protein coupled receptor antibody. For example, a supernatant liquid of hybridoma culture is added to a solid phase (e.g. microplate) to which the G protein coupled receptor protein antigen is adsorbed either directly or with a carrier, then anti-immunoglobulin antibody (anti-mouse immunoglobulin antibody is used when the cells used for the cell fusion are those of mouse) which is labeled with a radioactive substance, an enzyme or the like, or protein A is added thereto and then anti-G protein coupled receptor monoclonal antibodies bound on the solid phase are detected; or a supernatant liquid of the hybridoma culture is added to the solid phase to which anti-immunoglobulin or protein A is adsorbed, then the G protein coupled receptor labeled with a radioactive substance or an enzyme is added and anti-G protein coupled receptor monoclonal antibodies bonded with the solid phase is detected.

Selection and cloning of the anti-G protein coupled receptor monoclonal antibody-producing hybridoma may be carried out by methods per se known to those of skill in the art or methods similar thereto. Usually, it is carried out in a medium for animal cells, containing HAT (hypoxanthine, aminopterin and thymidine). With respect to a medium for the selection, for the cloning and for the growth, any medium may be used so far as hybridoma is able to grow therein. Examples of the medium are an RPMI 1640 medium (Dainippon Pharmaceutical Co., Ltd., Japan) containing 1-20% (preferably 10-20%) of fetal calf serum (FCS), a GIT medium (Wako Pure Chemical, Japan) containing 1-20% of fetal calf serum and a serum-free medium for hybridoma culturing (SFM-101; Nissui Seiyaku, Japan). The culturing temperature is usually 20-40°C and, preferably, about 37°C. The culturing time is usually from five days to three weeks and, preferably, one to two weeks. The culturing is usually carried out in 5% carbon dioxide gas. The antibody titer of the supernatant liquid of the hybridoma culture may be measured by the same manner as in the above-mentioned measurement of the antibody titer of the anti-G protein coupled receptor in the antiserum.

(b) Purification of the Monoclonal Antibody.

Like in the separation/purification of conventional polyclonal antibodies, the separation/purification of the anti-G protein coupled receptor monoclonal antibody may be carried out by methods for separating/purifying immunoglobulin such as salting-out, precipitation with an alcohol, isoelectric precipitation, electrophoresis, adsorption/deadsorption using ion exchangers such as DEAE, ultracentrifugation, gel filtration, specific purifying methods in which only an antibody is collected by treatment with an active adsorbent such as an antigen-binding solid phase, protein A or protein G and the bond is dissociated whereupon the antibody is obtained.

The G protein coupled receptor antibody which is manufactured by the aforementioned method (a) or (b) is capable of specifically recognizing G protein coupled receptors and, accordingly, it can be used for a quantitative determination of the G protein coupled receptor in test liquid samples and particularly for a quantitative determination by sandwich immunoassays.

Thus, the present invention provides, for example, the following methods:

(i) a quantitative determination of a G protein coupled receptor in a test liquid sample, which comprises

    (a) competitively reacting the test liquid sample and a labeled G protein coupled receptor with an antibody which reacts with the G protein coupled receptor, and
    (b) measuring the ratio of the labeled G protein coupled receptor binding with said antibody; and

(ii) a quantitative determination of a G protein coupled receptor in a test liquid sample, which comprises

    (a) reacting the test liquid sample with an antibody immobilized on an insoluble carrier and a labeled antibody simultaneously or continuously, and
    (b) measuring the activity of the labeling agent on the insoluble carrier

wherein one antibody is capable of recognizing the N-terminal region of the G protein coupled receptor while another antibody is capable of recognizing the C-terminal region of the G protein coupled receptor.

When the monoclonal antibody of the present invention recognizing a G protein coupled receptor (hereinafter, may be reffered to as "anti-G protein coupled receptor antibody") is used, G protein coupled receptors can be measured and, moreover, can be detected by means of a tissue staining, etc. as well. For such an object, antibody molecules per se may be used or F(ab')$_2$, Fab' or Fab fractions of the antibody molecule may be used too. There is no particular limitation for the measuring method using the antibody of the present invention and any measuring method may be used so far as it relates to a method in which the amount of antibody, antigen or antibody-antigen complex, depending on or corresponding to the amount of antigen, e.g. the amount of G protein coupled receptor, etc. in the liquid sample to be measured, is detected by a chemical or a physical means and then calculated using a standard curve prepared by a standard solution containing the known amount of antigen. For exmaple, nephrometry, competitive method, immunometric method and sanwich method are suitably used and, in terms of sensitivity and specificity, the sandwich method which will be described herein later is particularly preferred.

Examples of the labeling agent used in the measuring method using the labeling substance are radioisotopes, enzymes, fluorescent substances, luminescent substances, colloids, magnetic substances, etc. Examples of the radioisotope are [$^{125}$I], [$^{131}$I], [$^3$H] and [$^{14}$C]; preferred examples of the enzyme are those which are stable and with big specific activity, such as β-galactosidase, β-glucosidase, alkali phosphatase, peroxidase and malate dehydrogenase; examples of the fluorescent substance are fluorescamine, fluorescein isothiocyanate, etc.; and examples of the luminescent substance are luminol, luminol derivatives, luciferin, lucigenin, etc. Further, a biotin-avidin system may also be used for binding an antibody or antigen with a labeling agent.

In an insolubilization (immobilization) of antigens or antibodies, a physical adsorption may be used or a chemical binding which is usually used for insolubilization or immobilization of proteins or enzymes may be used as well. Examples of the carrier are insoluble polysaccharides such as agarose, dextran and cellulose; synthetic resins such as polystyrene, polyacrylamide and silicone; glass; etc.

In a sandwich (or two-site) method, the test liquid is made to react with an insolubilized anti-G protein coupled receptor antibody (the first reaction), then it is made to react with a labeled anti-G protein coupled receptor antibody (the second reaction) and the activity of the labeling agent on the insoluble carrier is measued whereupon the amount of the G protein coupled receptor in the test liquid can be determined. The first reaction and the second reaction may be conducted reversely or simultaneously or they may be conducted with an interval. The type of the labeling agent and the method of insolubilization (immobilization) may be the same as those mentioned already herein. In the immunoassay by means of a sandwich method, it is not always necessary that the antibody used for the labeled antibody and the antibody for the solid phase is one type or one species but, with an object of improving the measuring sensitivity, etc., a mixture of two or more antibodies may be used too.

In the method of measuring G protein coupled receptors by the sandwich method of the present invention, the preferred anti-G protein coupled receptor antibodies used for the first and the second reactions are antibodies wherein their sites binding to the G protein coupled receptors are different from each other. Thus, the antibodies used in the first and the second reactions are those wherein, when the antibody used in the second reaction recognizes the C-terminal region of the G protein coupled receptor, then the antibody recognizing the site other than C-terminal regions, e.g. recognizing the N-terminal region, is preferably used in the first reaction.

The anti-G protein coupled receptor antibody of the present invention may be used in a measuring system other than the sandwich method such as a competitive method, an immunometric method and a naphrometry. In a competitive method, an antigen in the test solution and a labeled antigen are made to react with an antibody in a competitive manner, then an unreacted labeled antigen (F) and a labeled antigen binding with an antibody (B) are separated (i.e. B/F separation) and the labeled amount of any of B and F is measured whereupon the amount of the antigen in the test solution is determined. With respect to a method for such a reaction, there are a liquid phase method in which a soluble antibody is used as the antibody and the B/F separation is conducted by polyethylene glycol, a second antibody to the above-mentioned antibody, etc.; and a solid phase method in which an immobilized antibody is used as the first antibody or a soluble antibody is used as the first antibody while an immobilized antibody is used as the second antibody.

In an immunometric method, an antigen in the test solution and an immobilized antigen are subjected to a competitive reaction with a certain amount of a labeled antibody followed by separating into solid and liquid phases; or the antigen in the test solution and an excess amount of labeled antibody are made to react, then a immobilized antigen is added to bind an unreacted labeled antibody with the solid phase and separated into solid and liquid phases. After that, the labeled amount of any of the phases is measured to determine the antigen amount in the test solution.

In a nephrometry, the amount of insoluble sediment which is produced as a result of the antigen-antibody reaction in a gel or in a solution is measured. Even when the antigen amount in the test solution is small and only a small amount of the sediment is obtained, a laser nephrometry wherein scattering of laser is utilized can be suitably used.

In applying each of those immunological measuring methods (immunoassays) to the measuring method of the present invention, it is not necessary to set up any special condition, operation, etc. therefor. A measuring system (assay system) for G protein coupled receptor may be constructed taking the technical consideration of the persons skilled in the art into consideration in the conventional conditions and operations for each of the methods. With details of those conventional technical means, a variety of reviews, reference books, etc. may be referred to. They are, for example, Hiroshi Irie (ed): "Radioimmunoassay" (Kodansha, Japan, 1974); Hiroshi Irie (ed): "Radioimmunoassay; Second Series" (Kodansha, Japan, 1979); Eiji Ishikawa et al. (ed): "Enzyme Immunoassay" (Igaku Shoin, Japan, 1978); Eiji Ishikawa et al. (ed): "Enzyme Immunoassay" (Second Edition) (Igaku Shoin, Japan, 1982); Eiji Ishikawa et al. (ed): "Enzyme Immunoassay" (Third Edition) (Igaku Shoin, Japan, 1987); "Methods in Enzymology" Vol. 70 (Immunochemical Techniques (Part A)); ibid. Vo. 73 (Immunochemical Techniques (Part B)); ibid. Vo. 74 (Immunochemical Techniques (Part C)); ibid. Vo. 84 (Immunochemical Techniques (Part D: Selected Immunoassays)); ibid. Vol. 92 (Immunochemical Techniques (Part E: Monoclonal Antibodies and General Immunoassay Methods)); ibid. Vol. 121 (Immunochemical Techniques (Part I: Hybridoma Technology and Monoclonal Antibodies)) (Academic Press); etc.

As such, the amount of G protein coupled receptor proteins can now be determined with a high precision using the anti-G protein coupled receptor antibody of the present invention.

In the specification and drawings of the present application, the abbreviations used for bases (nucleotides), amino acids and so forth are those recommended by the IUPAC-IUB Commission on Biochemical Nomenclature or those conventionally used in the art. Examples thereof are given below. Amino acids for which optical isomerism is possible are, unless otherwise specified, in the L form.

| | |
|---|---|
| DNA : | Deoxyribonucleic acid |
| cDNA : | Complementary deoxyribonucleic acid |
| A : | Adenine |
| T : | Thymine |
| G : | Guanine |
| C : | Cytosine |
| RNA : | Ribonucleic acid |
| mRNA : | Messenger ribonucleic acid |
| dATP : | Deoxyadenosine triphosphate |
| dTTP : | Deoxythymidine triphosphate |
| dGTP : | Deoxyguanosine triphosphate |
| dCTP : | Deoxycytidine triphosphate |
| ATP : | Adenosine triphosphate |
| EDTA : | Ethylenediamine tetraacetic acid |
| SDS : | Sodium dodecyl sulfate |
| EIA : | Enzyme Immunoassay |
| G, Gly: | Glycine (or Glycyl) |
| A, Ala: | Alanine (or Alanyl) |
| V, Val: | Valine (or Valyl) |
| L, Leu: | Leucine (or Leucyl) |
| I, Ile: | Isoleucine (or Isoleucyl) |
| S, Ser: | Serine (or Seryl) |
| T, Thr: | Threonine (or Threonyl) |
| C, Cys: | Cysteine (or Cysteinyl) |
| M, Met: | Methionine (or Methionyl) |
| E, Glu: | Glutamic acid (or Glutamyl) |
| D, Asp: | Aspartic acid (or Aspartyl) |
| K, Lys: | Lysine (or Lysyl) |
| R, Arg: | Arginine (or Arginyl) |
| H, His: | Histidine (or Histidyl) |
| F, Phe: | Phenylalamine (or Phenylalanyl) |
| Y, Tyr: | Tyrossine (or Tyrosyl) |
| W, Trp: | Tryptophan (or Tryptophanyl) |
| P, Pro: | Proline (or Prolyl) |
| N, Asn: | Asparagine (or Asparaginyl) |
| Q, Gin: | Glutamine (or Glutaminyl) |

Each SEQ ID NO set forth in the SEQUENCE LISTING of the specification refers to the following sequence:

[SEQ ID NO:1] is a ful-length amino acid sequence encoded by the human amygdaloid nucleus-derived G protein coupled receptor protein cDNA included in p63A2full,

[SEQ ID NO:2] is a nuleotide sequence of human mygdaloid nucleus-derived G protein coupled receptor protein cDNA included in p63A2full,

[SEQ ID NO:3] is a partial amino acid sequence of human amygdaloid nucleus-derived G protein coupled receptor protein which is induced by the nucleotide sequencing of 5'-terminal portion of cDNA included in p63A2,

[SEQ ID NO:4] is a partial amino acid sequence of human amygdaloid nucleus-derived G protein coupled receptor protein which is induced by the nucleotide sequencing of 3'-terminal portion of cDNA included in p63A2,

[SEQ ID NO:5] is a partial nucleotide sequence obtained by sequencing from 5'-terminal of human amygdaloid nucleus-derived G protein coupled receptor protein cDNA fragment included in p63A2,

[SEQ ID NO:6] is a partial nucleotide sequence obtained by sequencing from 3'-terminal of human amygdaloid nucleus-derived G protein coupled receptor protein cDNA fragment included in p63A2,

[SEQ ID NO:7] is a synthetic DNA primer for screening of cDNA coding for the G protein coupled receptor protein of the present invention,

[SEQ ID NO:8] is a synthetic DNA primer for screening of cDNA coding for the G protein coupled receptor protein of the present invention,

[SEQ ID NO:9] is a synthetic DNA primer for screening of cDNA coding for the G protein coupled receptor protein of the present invention,

[SEQ ID NO:10] is a synthetic DNA primer for screening of cDNA coding for the G protein coupled receptor protein of the present invention,

(SEQ ID NO:11] is a synthetic DNA primer for screening of cDNA coding for the G protein coupled receptor protein of the present invention,

[SEQ ID NO:12] is a synthetic DNA primer for screening of cDNA coding for the G protein coupled receptor protein of the present invention,

[SEQ ID NO:13] is a synthetic DNA primer for screening of cDNA coding for the G protein coupled receptor protein of the present invention,

[SEQ ID NO:14] is a synthetic DNA primer for screening of cDNA coding for the G protein coupled receptor protein of the present invention,

[SEQ ID NO:15] is a synthetic DNA primer for screening of cDNA coding for the G protein coupled receptor protein of the present invention,

[SEQ ID NO:16] is a synthetic DNA primer for screening of cDNA coding for the G protein coupled receptor protein of the present invention,

[SEQ ID NO:17] is a synthetic DNA primer for screening of cDNA coding for the G protein coupled receptor protein of the present invention,

[SEQ ID NO:18] is a synthetic DNA primer for screening of cDNA coding for the G protein coupled receptor protein of the present invention, and

[SEQ ID NO:19] is a synthetic DNA primer for screening of cDNA coding for the G protein coupled receptor protein of the present invention.

The transformant Escherichia coli, designated INVαF'/p63A2, which is obtained in the Reference Example 3 mentioned hereinafter, is on deposit under the terms of the Budapest Treaty from August 9, 1994, with the National Institute of Bioscience and Human-Technology (NIBH), Agency of Industrial Science and Technology, Ministry of International Trade and Industry, Japan and has been assigned the Accession Number FERM BP-4777. It is also on deposit from August 22, 1994 with the Institute for Fermentation, Osaka, Japan (IFO) and has been assigned the Accession Number IFO 15738.

The transformant Escherichia coli, designated p63A2full, which is obtained in the Example 2 mentioned hereinafter, is on deposit under the terms of the Budapest Treaty from February 2, 1996, with NIBH and has been assigned the Accession Number FERM BP-5380. It is also on deposit from February 13, 1996 with IFO and has been assigned the Accession Number IFO 15924.

[Examples]

Described below are working examples of the present invention which are provided only for illustrative purposes, and not to limit the scope of the present invention.

[Reference Example 1]

Preparation of Synthetic DNA Primer for Amplifying DNA Coding for G Protein Coupled Receptor Protein

A compariton of deoxyribonucleotide sequences coding for the known amino acid sequences corresponding to or near the first membrane-spanning domain each of human-derived TRH receptor protein (HTRHR), human-derived RANTES receptor protein (HUMRANTES), human Burkitt's lymphoma-derived unknown ligand receptor protein (HSBLR1A), human-derived somatostatin receptor protein (HUMSOMAT), rat-derived $\mu$-opioid receptor protein (RNU02083), rat-derived $\kappa$-opioid receptor protein (U00442), human-derived neuromedin B receptor protein (HUMNMBR), human-derived muscarinic acetylcholine receptor protein (HSHM4), rat-derived adrenaline $\alpha_1$B receptor protein (RATAADRE01), human-derived somatostatin 3 receptor protein (HUMSSTR3X), human-derived $C_5$a receptor protein (HUMC5AAR), human-derived unknown ligand receptor protein (HUMRDC1A), human-derived unknown ligand receptor protein (HUMOPIODRE) and rat-derived adrenaline $\alpha_2$B receptor protein (RATA2BAR) was made. As a result, highly homologous regions or parts were found.

Further, a comparison of deoxynucleotide sequences coding for the known amino acid sequences corresponding to or near the sixth membrane-spanning domain each of mouse-derived unknown ligand receptor protein (HUMGIR), human-derived bombesin receptor protein (HUMBOMB3S), human-derived adenosine A2 receptor protein (S46950), mouse-derived unknown ligand receptor protein (MUSGPCR), mouse-derived TRH receptor protein (S43387), rat-derived neuromedin K receptor protein (RATNEURA), rat-derived adenosine A1 receptor protein (RATA1ARA), human-derived neurokinin A receptor protein (HUMNEKAR), rat-derived adenosine A3 receptor protein (RATADENREC), human-derived somatostatin 1 receptor protein (HUMSRI1A), human-derived neurokinin 3 receptor protein (S86371S4), rat-derived unknown ligand receptor protein (RNCGPCR), human-derived somatostatin 4 receptor protein (HUMSSTR4Z) and rat-derived GnRH receptor protein (RATGNRHA) was made. As a result, highly homologous regions or parts were found.

The aforementioned abbreviations in the parentheses are identifiers (reference numbers) which are indicated when Genganik/EMBL Data Bank is retrieved by using DNASIS Gene/Protein Sequencing Data Base (CD019, Hitachi Software Engineering, Japan) and are usually called "Accession Numbers" or "Entry Names". HTRHR is, however, the sequence as disclosed in Japanese Patent Publication No. 304797/1993 (EPA 638645).

Specifically, it was planned to incorporate mixed bases relying upon the base regions that were in agreement with cDNAs coding for a large number of receptor proteins in order to enhance base agreement of sequences with as many receptor cDNAs as possible even in other regions. Based upon these sequences, the degenerate synthetic DNA having a nucleotide sequence represented by SEQ ID NO:7 or SEQ ID NO:8 which is complementary to the homologous nucleotide sequence were produced.

[Synthetic DNAs]

5'-CGTGG (G or C) C (A or C) T (G or C) (G or C)
TGGGCAAC (A, G, C or T) (C or T) CCTG-3' (SEQ ID NO:7)
5'-GT (A, G, C or T) G (A or T) (A or G) (A or G) GGCA (A, G, C or T) CCAGCAGA (G or T) GGCAAA-3' (SEQ ID NO:8)

The parentheses indicate the incorporation of a plurality of bases, leading to multiple oligonucleotides in the primer preparation. In other words, nucleotide resides in parentheses of the aforementioned DNAs were incorporated in the presence of a mixture of plural bases at the time of synthesis.

[Reference Example 2]

Isolation of Human Amygdaloid Nucleus-Derived G Protein Coupled Receptor Protein-Encoding DNA

(1) Amplification of Receptor cDNA by PCR Using Human Amygdaloid Nucleus-Derived cDNA

By using a human amygdala-derived cDNA (QuickClone, CLONTECH Laboratories, Inc.) as a template, PCR amplification using the DNA primers synthesized in Example 1 was carried out. The composition of the reaction solution consisted of the synthetic DNA primers (SEQ: 5' primer sequence and 3' primer sequence) each in an amount of 1 μM, 1 ng of the template cDNA, 0.25 mM dNTPs, 1 μl of Taq DNA polymerase and a buffer attached to the enzyme kit, and the total amount of the reaction solution was made to be 100 μl. The cycle for amplification including 95°C for 1 min., 55°C for 1 min. and 72°C for 1 min. was repeated 30 times by using a Thermal Cycler (Perkin-Elmer Co.). Prior to adding Taq DNA polymerase, the remaining reaction solution was mixed and was heated at 95°C for 5 minutes and at 65°C for 5 minutes. The amplified produce were confirmed relying upon 1.2% agarose gel electrophoresis and ethidium bromide staining.

(2) Subcloning of PCR Product into Plasmid Vector and Selection of Novel Receptor Candidate Clone via analyzing Nucleotide Sequence of Inserted cDNA Region

The PCR products were separated by using a 0.8% low-melting temperature agarose gel, the band parts were excised from the gel with a razor balde, and were heat-melted, extracted with phenol and precipitated in ethanol to recover DNAs. According to the protocol attached to a TA Cloning Kit (Invitrogen Co.), the recovered DNAs were subcloned into the plasmid vector, pCR™II. The recombinant vectors were introduced into E. Coli INVα F' competent cells (Invitrogen Co.) to produce transformants. Then, transformant clones having a cDNA-inserted fragment were selected in an LB agar culture medium containing ampicillin and X-gal. Only transformant clones exhibiting white color were picked with a sterilized toothstick to obtain transformant Escherichia coli INVα F'/p63A2.

The individual clones were cultured overnight in an LB culture medium containing ampicillin and treated with an automatic plasmid extracting machine (Kurabo Co., Japan) to prepare plasmid DNAs. An aliquot of the DNA thus prepared was cut by EcoRI to confirm the size of the cDNA fragment that was inserted. An aliquot of the remaining DNA was further processed with RNase, extracted with phenol/chloroform, and precipitated in ethanol so as to be condensed. Sequencing was carried out by using a DyeDeoxy terminator cycle sequencing kit (ABI Co.), the DNAs were sequenced by using a fluorescent automatic sequencer, and the the data of the nucleotide sequences obtained were analyzed by using DNASIS (Hitachi System Engineering Co., Japan).

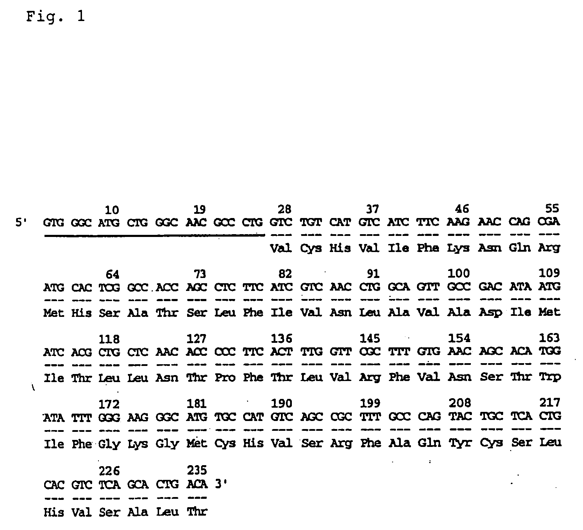

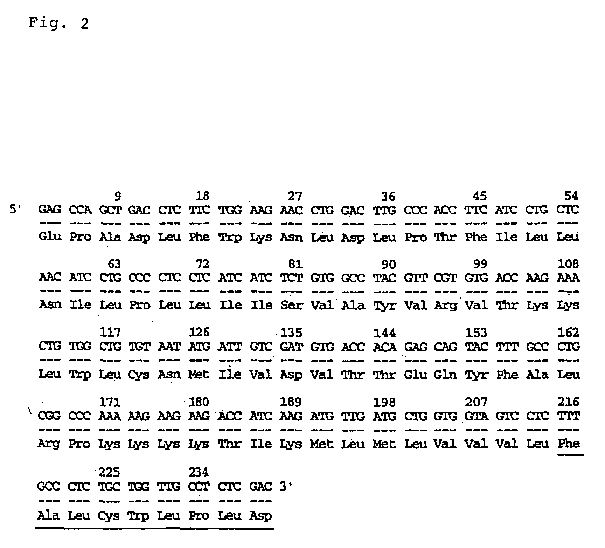

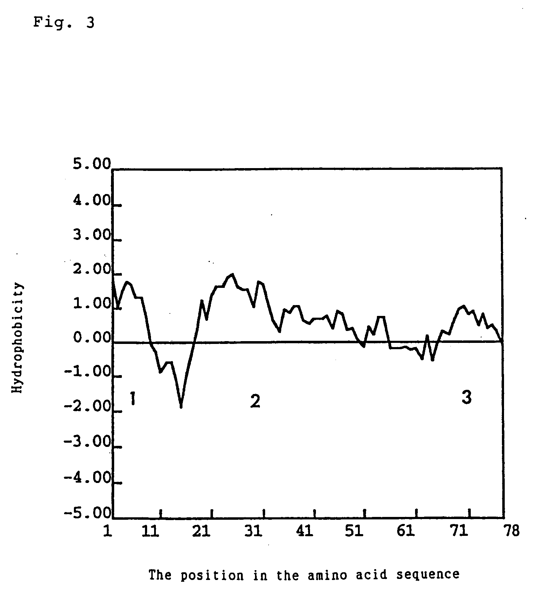

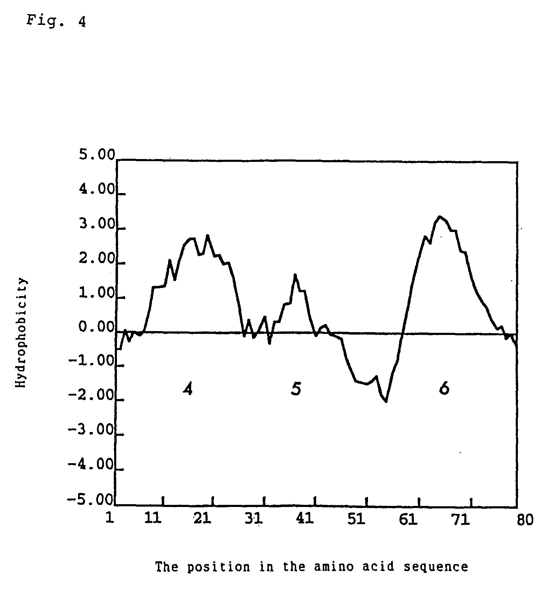

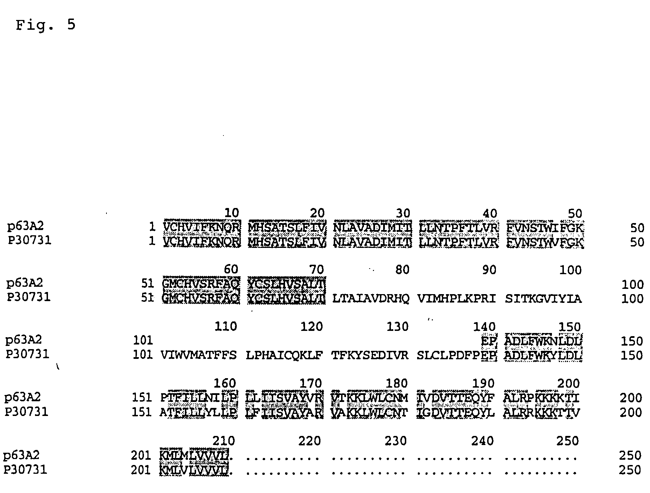

Homology search was carried out based upon the determined nucleotide sequences [Figures 1 and 2]. As a result, it was found that a novel G protein coupled receptor protein was encoded by the cDNA fragment insert in the plasmid, p63A2 possessed by the transformant Escherichia coli INVα F'/p63A2. To further confirm this fact, by using DNASIS (Hitachi System Engineering Co., Japan) the nucleotide sequences were converted into amino acid sequences [Figures 1 and 2], and homology retrieval was carried out in view of hydrophobicity plotting [Figures 3 and 4] and at the amino acid sequence level to find homology to mouse GIR [Figure 5].

Partial peptide sequences of SEQ ID Nos. 3 and 4 can be obatained by adapting the above-described methods.

[Example 1] Cloning of the unknown sequence region of cDNA encoding the novel human orphan receptor (63A2full) from a human hypothalamic cDNA library by RACE (rapid amplificaiton of cDNA ends)

As a template for the RACE, a human hypothalamic cDNA library (Clontech) was prepared. Since this cDNA library is constructed on the basis of the phage vector λgt11, the following primers were synthesized for amplifying the cDNA portion:

Primers based on the known nucleotide sequence of the left arm portion of λgt11:

U1 : sequence 5'-ATATGGGGATTGGTGGCGACGACTC-3' (25mer)      (SEQ ID NO:9)
U2 : sequence 5'-CAACATCAGCCGCTACAGTCAACAG-3' (25mer)      (SEQ ID NO:10)

Primers based on the known nucleotide sequence of the right arm:

L1 : sequence 5'-GCCCGGTTATTATTATTTTTGACAC-3' (25mer)      (SEQ ID NO:11)
L2 : sequence 5'-TTCCTTACGCGAAATACGGGCAGAC-3' (25mer)      (SEQ ID NO:12)

Further, the following were synthesized as primers specific to 63A2:

63U : sequence 5'-CGGCCACCAGCCTCTTCATCGTCAACCTGGC-3' (31mer) (SEQ ID NO:13)
63L : sequence 5'-GGCAACCAGCAGAGGGCAAAGAGGACTACC-3' (30mer) (SEQ ID NO:14)

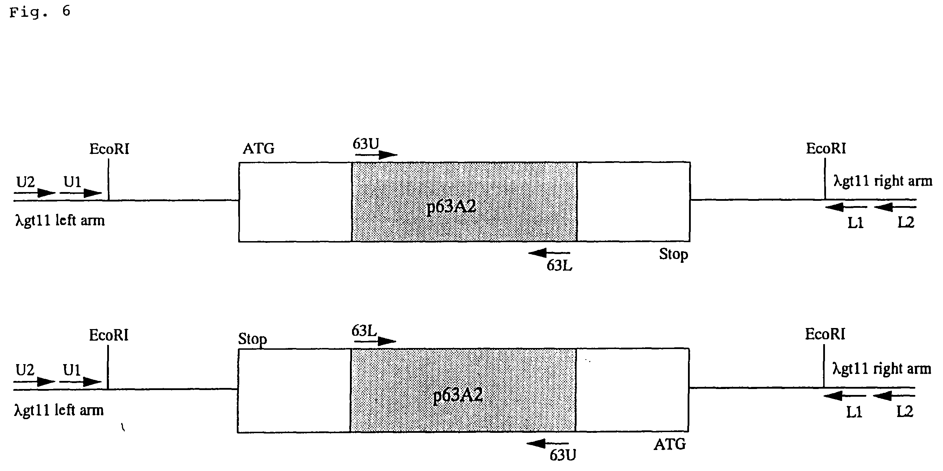

The anticipated relative locations of the respective primers, insert cDNA fragment and λgt11 are shown in Fig. 6.

The human hypothalamic cDNA λgt11 phage library solution (20 μl) was mixed with 80 μl of sterile distilled water and then heat-denatured by heating at 95°C for 10 minutes, followed by quenching in ice water for 10 minutes.

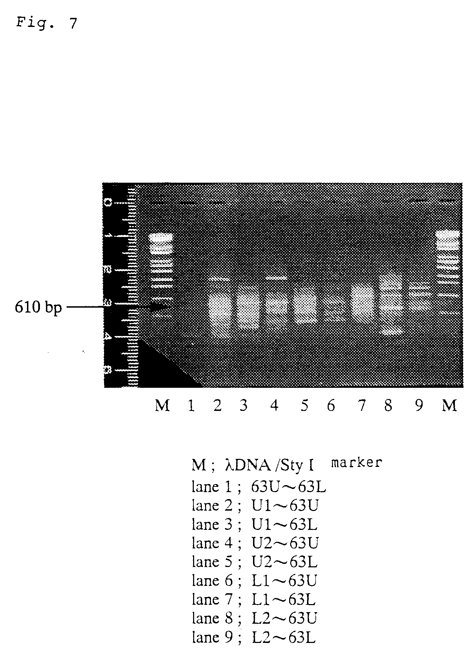

The PCR reaction was carried out by the hot start technique using AmpliWax PCR Gem 100 (Perkin Elmer). The bottom layer mix was prepared by mixing up 2 μl of 10 x EX PCR buffer (attached to the enzyme), 4 μl of 12.5 mM dNTP solution, 0.5 μl each of 50 μM primer solutions (the primer combinations being, corresponding to Fig. 6 and Fig. 7, as follows: lane 1, 63U and 63L; lane 2, U1 and 63U; lane 3, U1 and 63L; lane 4, U2 and 63U; lane 5, U2 and 63L; lane 6, L1 and 63U; lane 7, L1 and 63L; lane 8, L2 and 63U; lane 9, U2 and 63L) and 13 μl of sterile distilled water. The top layer mix was prepared by mixing up 2.5 μl of the phage solution prepared as the template in the above manner, 3 μl of 10 x EX PCR buffer (attached to the enzyme), 0.5 μl of TaKaRa EX Taq DNA polymerase (Takara Shuzo) and 24 μl of sterile distilled water. The PCR mix was prepared by adding one AmpliWax PCR Gem 100 to the thus-prepared bottom layer mix, treating the same at 70°C for 5 minutes and in ice for 5 minutes, and adding the top layer mix thereto.

The tube containing the reaction mix was set on the thermal cycler Gene ATAQ Controller (Pharmacia) and treated at 95°C for 3 minutes, at 62°C for 2 minutes and at 75°C for 2 minutes. Further, the cycle of 1 minute at 95°C, 1 minute at 62°C and 2 minutes at 75°C was repeated 30 times, followed by 8 minutes of treatment at 75°C.

After completion of the reaction, a portion (5 μl) of the reaction mixture was subjected to SeaKem GTG (FMC) 1% agarose gel electrophoresis for analyzing the amplification products. As shown in Fig. 7, a large number of amplification products were detected, so that Southern hybridization was performed for detecting PCR products specific to 63A2.

For Southern hybridization, the non-RI-labeled DIG System (Boehringer Mannheim) was used. The probe was prepared using the PCR DIG probe synthesis kit (Boehringer Mannheim) with the plasmid p63A2 as the template and 63U and 63L as the primers. Southern transfer was effected by the capillary method using 20 x SSC, 0.4 M NaOH solution. DNA fixation on the filter was performed using the UV crosslinker CL-1000 (UVP). Hybridization was carried out at 68°C for 1 hour using the Express Hyb hybridization solution (Clontech) containing 60 ng of the PCR DIG probe prepared. The first 15-minute washing was carried out two times with 2 x SSC, 0.1% SDS solution at room temperature. The second 15-minute washing was carried out two times with 0.1 x SSC, 0.1% SDS solution at 60°C. The DIG luminescent detection kit (Boehringer Mannheim) was used for detecting hybridized bands.

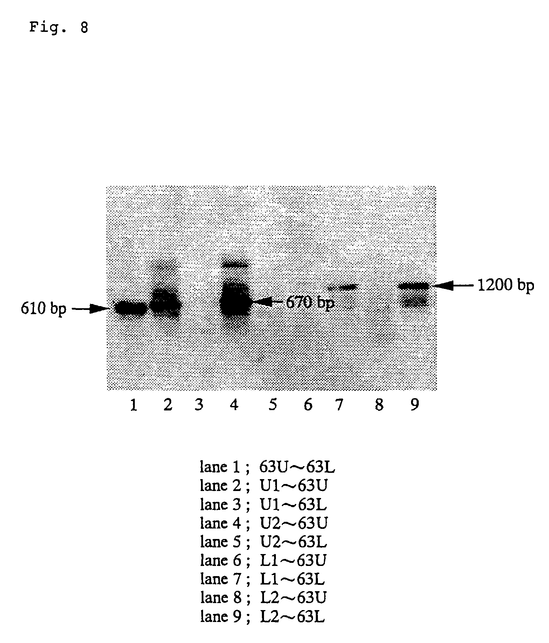

As shown in Fig. 8, the Southern hybridization detected a DNA band hybridizing with the probe at about 670 bp with the primer combinations U1 and 63U, and U2 and 63U, and such a DNA band at about 1,200 bp with the combinations L1 and 63L, and L2 and 63L. Therefore, the respective DNA fragments were recovered from the hybridized portions and subcloned into a plasmid vector using the TA cloning kit (Invitrogen), and clones containing the 670 bp or 1,200 bp PCR products were subjected to insert nucleotide sequence analysis.

The sequencing reaction was carried out on the R.O.B. DNA processor (Pharmacia) using the BcaBEST™ dideoxy sequencing kit (Takara Shuzo), and the nucleotide sequence determination was performed using the ALF DNA sequencer II (Pharmacia).

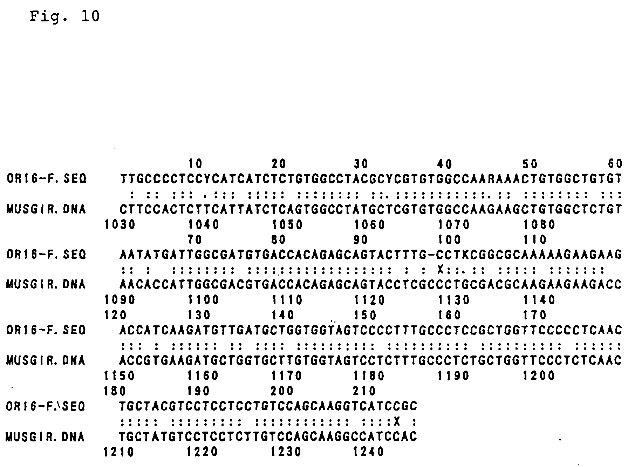

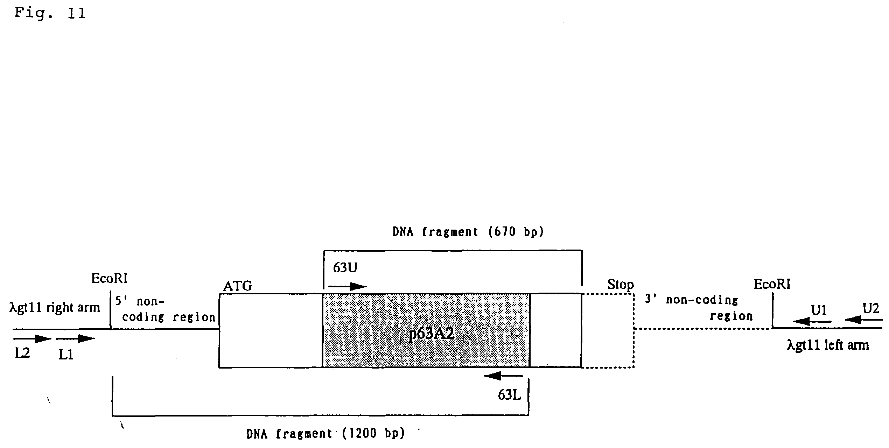

Comparison of the thus-obtained nucleotide sequence with the nucleotide sequence of GIR, which is the murine counterpart of 63A2, verified the presence of portions very similar in sequence, as shown in Fig. 9 and Fig. 10. However, as shown in Fig. 10, estimation from the structure of GIR revealed that, with regard to the 3' side of the open reading frame of 63A2, the portion containing the termination codon for stopping translation had not been obtained. Based on the sequences shown in Fig. 9 and Fig. 10, the sequence now revealed of 63A2 and the unknown sequence region are schematically shown in Fig. 11. Then, cloning of the 3' side portion of the open reading frame was performed using the 3'-AmpliFINDER RACE kit (Clontech) with a total human brain poly(A)$^+$ RNA (Clontech) as the template.

For the 3'-AmpliFINDER RACE, the following were synthesized as primers specific to 63A2:

63-6 : sequence 5'-TTCATCCTGCTCTACATCCTGCCCCTCCTC-3' (30mer) (SEQ ID NO:15)
63-7 : sequence 5'-GCCCTGCGGCGCAAAAAGAAGAAGACCATC-3' (30mer) (SEQ ID NO:16)
63-8 : sequence 5'-GCTGGTGGTAGTCCTCTTTGCCCTCTGCTG-3' (30mer) (SEQ ID NO:17)

For the first strand cDNA synthesis, 11.5 μl of diethyl pyrocarbonate (DEPC)-treated water (attached to the kit) and 1 μl of 10 μM NN-1 oligo(dT) CDS primer (attached to the kit) were added to 1 μl of 1 μg/μl total human brain poly(A)$^+$ RNA and the mixture was treated at 65°C for 10 minutes and then in ice for 2 minutes. To this mixture were added 5 μl of 4 x reverse transcriptase buffer (attached to the kit) and 5 μl of ultrapure dNTP mix (attached to the kit). After 2 minutes of treatment at 52°C, 0.5 μl of 25 units/μl AMV reverse transcriptase (attached to the kit) was further added. After 30 minutes of treatment at 52°C, 0.5 μl of 2 units/μl E. coli RNase H (attached to the kit) was added and the whole mixture was treated at 37°C for 20 minutes and at 95°C for 5 minutes. The thus-prepared solution was stored as the first strand cDNA synthesis solution at -20°C.

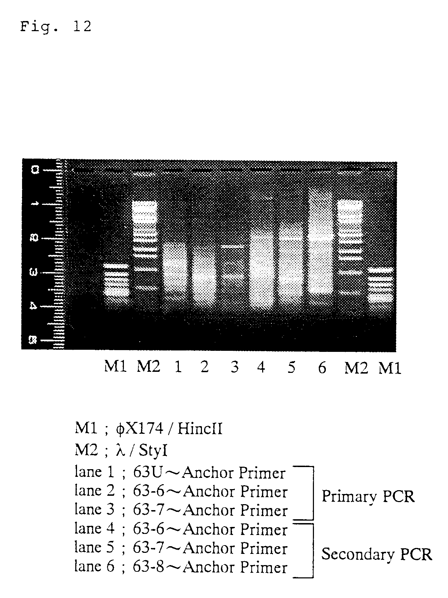

The primary PCR was performed by the hot start technique using the AmpliWax PCR Gem 100 (Perkin Elmer). The bottom layer mix was prepared by mixing up 2 µl of 10 x EX PCR buffer (attached to the enzyme), 1 µl of 10 mM dNTP solution, 1 µl each of 10 µM primer solutions (the primer combinations being, corresponding to Fig. 12, as follows: lane 1, 63U and anchor primer (attached to the kit); lane 2, 63-6 and anchor primer; lane 3, 63-7 and anchor primer) and 15 µl of sterile distilled water. The top layer mix was prepared by mixing up 1 µl of the first strand cDNA synthesis solution prepared in advance as the template, 3 µl of 10 x EX PCR buffer (attached to the enzyme), 0.5 µl of TaKaRa EX Taq DNA polymerase (Takara Shuzo) and 25.5 µl of sterile distilled water. The PCR mix was prepared by adding one Ampli-Wax PCR Gem 100 to the thus-prepared bottom layer mix, treating the same at 70°C for 5 minutes and in ice for 5 minutes, and adding the top layer mix thereto.

The tube containing the reaction mix was set on the thermal cycler PJ2000 (Perkin Elmer) and treated at 95°C for 3 minutes, at 60°C for 2 minutes and at 75°C for 2 minutes. Further, the cycle of 1 minute at 95°C, 1 minute at 60°C and 2 minutes at 75°C was repeated 30 times, followed by 8 minutes of treatment at 75°C.

The secondary PCR was also carried out by the hot start technique using the AmpliWax PCR Gem 100 (Perkin Elmer). The bottom layer mix was prepared by mixing up 2 µl of 10 x EX PCR buffer (attached to the enzyme), 1 µl of 10 mM dNTP solution, 1 µl each of 10 µM primer solutions (the primer combinations being, corresponding to Fig. 12, as follows: lane 4, 63-6 and anchor primer; lane 5, 63-7 and anchor primer; lane 6, 63-8 and anchor primer) and 15 µl of sterile distilled water. The top layer mix was prepared by mixing up 1 µl of the primary reaction PCR solution as the template (the PCR solution of the primary reaction using 63U and anchor primer being used for the reaction using 63-6 and anchor primer; the PCR solution of the primary reaction using 63-6 and anchor primer for the reaction using 63-7 and anchor primer; and the PCR solution of the primary reaction using 63-7 and anchor primer for the reaction using 63-8 and anchor primer), 3 µl of 10 x EX PCR buffer (attached to the enzyme), 0.5 µl of TaKaRa EX Taq DNA polymerase (Takara Shuzo) and 25.5 µl of sterile distilled water. The PCR mix was prepared by adding one AmpliWax PCR Gem 100 to the thus-prepared bottom layer mix, treating the same at 70°C for 5 minutes and in ice for 5 minutes, and adding the top layer mix thereto.

The tube containing the reaction mix was set on the thermal cycler PJ2000 (Perkin Elmer) and treated at 95°C for 3 minutes, at 60°C for 2 minutes and at 75°C for 2 minutes. Further, the cycle of 1 minute at 95°C, 1 minute at 60°C and 2 minutes at 75°C was repeated 30 times, followed by 8 minutes of treatment at 75°C.

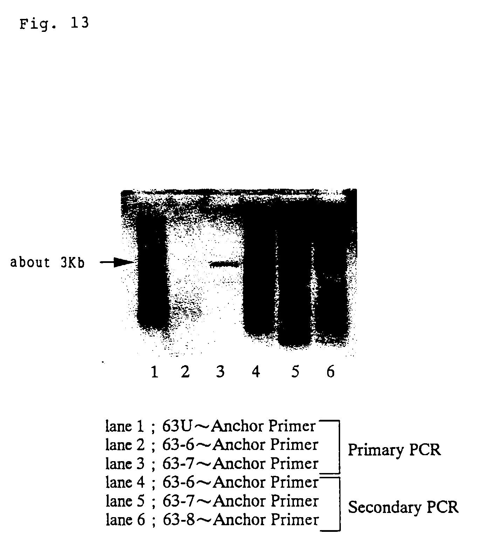

As shown in Fig. 12, a plurality of bands appeared in the primary PCR as well as in the secondary PCR using the primary PCR products as templates. Therefore, analyses were performed by means of Southern hybridization in the same manner as mentioned above. A a result, a band of about 3 kb was detected, as shown in Fig. 13. The DNA fragments were recovered from the respective hybridized portions and subcloned into a plasmid vector using the TA cloning kit (Invitrogen), and the insert nucleotide sequence was analyzed for a clone containing the 3 kb PCR product.

The sequencing reaction was carried out using the BcaBEST dideoxy sequencing kit (Takara Shuzo) and R.O.B. DNA processor (Pharmacia), and the nucleotide sequence analysis was performed using the ALF DNA sequencer II (Pharmacia).

The thus-obtained nucleotide sequence was compared with GIR, which is the murine counterpart of 63A2, whereby the presence of a portion very close in sequence to the 3' terminal side of the open reading frame of GIR was confirmed, as shown in Fig. 14. (Example 2] Cloning of the full-length open reading frame of 63A2full-encoding cDNA

Based on the sequences of the 5'- and 3'-nontranslational region portions as determined in Example 1, the following pair of primers with an added restriction enzyme SalI site were synthesized:

F63U(-30) : sequence 5'-GGAGTCGACCAGGGGAGGGGTGGCTCCTGCAAA-3' (33mer) (SEQ ID NO:18)
F63L(1465) : sequence 5'-CCCGTCGACCCCCTCCCACTCCCTCTTCCCAAC-3' (33mer) (SEQ ID NO:19)

Using these, PCR was carried out with the total human brain QUICK-Clone cDNA (Clontech) as the template for amplifying the full-length open reading frame of 63A2full.

The PCR reaction was carried out by the hot start technique Using the AmpliWax PCR Gem 100 (Perkin Elmer). The bottom layer mix was prepared by mixing up 2 µl of 10 x EX PCR buffer (attached to the enzyme), 4 µl of 2.5 mM dNTP solution, 0.5 µl each of 50 µM primer solutions (the primer combination being F63U and F63L) and 13 µl of sterile distilled water. The top layer mix was prepared by mixing up 1 µl of the total human brain QUICK-Clone cDNA as the template, 3 µl of 10 x EX PCR buffer (attached to the enzyme), 0.5 µl of TaKaRa EX Taq DNA polymerase (Takara Shuzo) and 25.5 µl of sterile distilled water. The reaction mix was prepared by adding one AmpliWax PCR Gem 100 to the thus-prepared bottom layer mix, treating the resulting mixture at 70°C for 5 minutes and then in ice water for 5 minutes, and adding the top layer mix thereto.

The tube containing the reaction mix was set on the thermal cycler PJ2000 (Perkin Elmer) and treated at 95°C for 3 minutes, at 63°C for 2 minute and at 75°C for 2 minutes. Further, the cycle of 1 minute at 95°C, 1 minute at 63°C and 2 minutes at 75°C was repeated 30 times, followed by 8 minutes of treatment at 75°C.

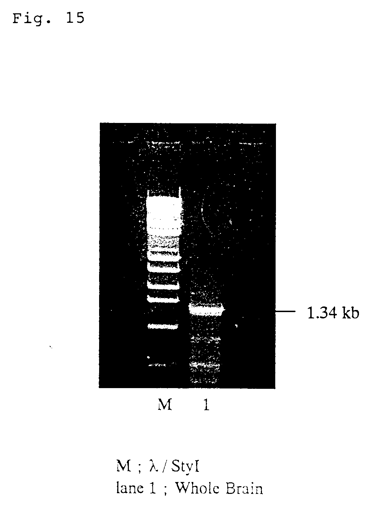

After completion of the reaction, a portion (5 µl) of the reaction mixture was subjected to SeaKem GTG (FMC) 1% agarose gel electrophoresis for analyzing amplification products. As shown in Fig. 15, a band, 1.34 kb in size, was detected. This PCR product was subcloned into a plasmid vector using the TA cloning kit (Invitrogen) and, for the transformant strain obtained, the whole nucleotide sequence of the insert portion was determined. Said strain was named Escherichia coli/p63A2full.

The full-length nucleotide sequence of the open reading frame of 63A2full and the amino acid sequence deduced therefrom are shown in Fig. 16 and Fig. 17. As shown in Fig. 18, the presence of 7 hydrophobic domains characteristic of G protein coupled receptor proteins was confirmed also from the results of hydrophobicity plotting. Furthermore, as shown in Fig. 19, the amino acid sequence of the human total brain-derived 63A2full, when compared with that of murine GIR, showed a homology as high as 85.8%.

The G protein coupled receptor protein and the DNA encoding said protein as provided by the present invention can be used in (1) identifying the ligands to the G protein coupled receptor protein of the present invention, (2) acquiring antibodies and antisera, (3) constructing expression systems for recombinant receptor proteins, (4) developing receptor binding assay systems and screening out candidate drug compound using said expression systems, (5) drug designing based on the comparison with structurally similar ligand and receptors, (6) preparation of probes and PCR primers for gene diagnosis, and (7) gene therapy, among others. In particular, to elucidate the structures and properties of G protein coupled receptor proteins may lead to development of unique drugs acting on these systems.

**Claims**

1. A G protein coupled receptor protein which comprises an amino acid sequence represented by SEQ ID NO:1 or its substantial equivalent thereto, or a salt thereof.

2. A partial peptide of the G protein coupled receptor protein as claimed in Claim 1, or a salt thereof.

3. A DNA which comprises a nucleotide sequence coding for the G protein coupled receptor protein as claimed in Claim 1 or the partial peptide as claimed in Claim 2.

4. The DNA as claimed in Claim 3, which comprises the nucleotide sequence represented by SEQ ID NO:2.

5. A recombinant vector comprising the DNA as claimed in Claim 3.

6. A transformant carrying the DNA as claimed in Claim 3 or the recombinant vector as claimed in Claim 5.

7. A process for producing the G protein coupled receptor protein or a salt thereof as claimed in Claim 1, which comprises culturing the transformant as claimed in Claim 6.

8. A method for determining a ligand to the G protein coupled receptor protein as claimed in Claim 1, which comprises contacting (i) the G protein coupled receptor protein or a salt thereof as claimed in Claim 1 or the partial peptide or a salt thereof as claimed in Claim 2, with (ii) a sample to be tested.

9. A screening method for a compound capable of changing the binding activity of the G protein coupled receptor protein as claimed in Claim 1 with a ligand, or a salt thereof, which comprises making a comparison between: (i) at least one case where said ligand is contacted with the G protein coupled receptor protein or a salt thereof as claimed in Claim 1, or the partial peptide or a salt thereof as claimed in Claim 2, and (ii) at least one case where said ligand together with a sample to be tested is contacted with the G protein coupled receptor protein or a salt thereof as claimed in Claim 1 or the partial peptide or a salt thereof as claimed in Claim 2.

10. A kit for the screening of a compound capable of changing the binding activity of the G protein coupled receptor protein as claimed in Claim 1 with a ligand, or a salt thereof, which comprises the G protein coupled receptor protein or a salt thereof as claimed in Claim 1, or the partial peptide or a salt thereof as claimed in Claim 2.

11. A compound capable of changing the binding activity of the G protein coupled receptor protein as claimed in claim 1 with a ligand, or a salt thereof, which is obtained by the screening method as claimed in claim 9 or by using the kit for the screening as claimed in claim 10.

12. An antibody against the G protein coupled receptor protein or a salt thereof as claimed in Claim 1 or the partial peptide or a salt thereof as claimed in Claim 2.

Fig. 1

```
              10              19              28              37              46              55
5'  GTG GGC ATG CTG GGC AAC GCC CTG GTC TGT CAT GTC ATC TTC AAG AAC CAG CGA
    ―――――――――――――――――――――――――――――  --- --- --- --- --- --- --- --- --- ---
                                     Val Cys His Val Ile Phe Lys Asn Gln Arg


              64              73              82              91              100             109
    ATG CAC TCG GCC ACC AGC CTC TTC ATC GTC AAC CTG GCA GTT GCC GAC ATA ATG
    --- --- --- --- --- --- --- --- --- --- --- --- --- --- --- --- --- ---
    Met His Ser Ala Thr Ser Leu Phe Ile Val Asn Leu Ala Val Ala Asp Ile Met


              118             127             136             145             154             163
    ATC ACG CTG CTC AAC ACC CCC TTC ACT TTG GTT CGC TTT GTG AAC AGC ACA TGG
    --- --- --- --- --- --- --- --- --- --- --- --- --- --- --- --- --- ---
    Ile Thr Leu Leu Asn Thr Pro Phe Thr Leu Val Arg Phe Val Asn Ser Thr Trp


              172             181             190             199             208             217
    ATA TTT GGG AAG GGC ATG TGC CAT GTC AGC CGC TTT GCC CAG TAC TGC TCA CTG
    --- --- --- --- --- --- --- --- --- --- --- --- --- --- --- --- --- ---
    Ile Phe Gly Lys Gly Met Cys His Val Ser Arg Phe Ala Gln Tyr Cys Ser Leu


              226             235
    CAC GTC TCA GCA CTG ACA 3'
    --- --- --- --- --- ---
    His Val Ser Ala Leu Thr
```

40

Fig. 2

```
              9             18            27            36            45            54
5'  GAG CCA GCT GAC CTC TTC TGG AAG AAC CTG GAC TTG CCC ACC TTC ATC CTG CTC
    --- --- --- --- --- --- --- --- --- --- --- --- --- --- --- --- --- ---
    Glu Pro Ala Asp Leu Phe Trp Lys Asn Leu Asp Leu Pro Thr Phe Ile Leu Leu

              63            72            81            90            99           108
    AAC ATC CTG CCC CTC CTC ATC ATC TCT GTG GCC TAC GTT CGT GTG ACC AAG AAA
    --- --- --- --- --- --- --- --- --- --- --- --- --- --- --- --- --- ---
    Asn Ile Leu Pro Leu Leu Ile Ile Ser Val Ala Tyr Val Arg Val Thr Lys Lys

             117           126           135           144           153           162
    CTG TGG CTG TGT AAT ATG ATT GTC GAT GTG ACC ACA GAG CAG TAC TTT GCC CTG
    --- --- --- --- --- --- --- --- --- --- --- --- --- --- --- --- --- ---
    Leu Trp Leu Cys Asn Met Ile Val Asp Val Thr Thr Glu Gln Tyr Phe Ala Leu

             171           180           189           198           207           216
 \  CGG CCC AAA AAG AAG AAG ACC ATC AAG ATG TTG ATG CTG GTG GTA GTC CTC TTT
    --- --- --- --- --- --- --- --- --- --- --- --- --- --- --- --- --- ---
    Arg Pro Lys Lys Lys Lys Thr Ile Lys Met Leu Met Leu Val Val Val Leu Phe
                                                                           ___

             225           234
    GCC CTC TGC TGG TTG CCT CTC GAC 3'
    --- --- --- --- --- --- --- ---
    Ala Leu Cys Trp Leu Pro Leu Asp
    _____
```

Fig. 3

The position in the amino acid sequence

Fig. 4

The position in the amino acid sequence

Fig. 5

```
                        10          20          30          40          50
p63A2    1  VCHVIFKNQR  MHSATSLFIV  NLAVADIMII  LLNTPFTLVR  FVNSTWIFGK   50
P30731   1  VCHVIFKNQR  MHSATSLFIV  NLAVADIMII  LLNTPFTLVR  FVNSTWVFGK   50

                        60          70          80          90         100
p63A2   51  GMCHVSRFAQ  YCSLHVSAYI                                       100
P30731  51  GMCHVSRFAQ  YCSLHVSALI  LTAIAVDRHQ  VIMHPLKPRI  SITKGVIYIA   100

                       110         120         130         140         150
p63A2  101                                                  EP  ADLFWKNLDL   150
P30731 101  VIWVMATFFS  LPHAICQKLF  TFKYSEDIVR  SLCLPDFPEP  ADLFWKYLDL   150

                       160         170         180         190         200
p63A2  151  PTFILLNILP  LLIISVAYVR  VTKKLWLCNM  IVDVTTEQYF  ALRPKKKKTI   200
P30731 151  ATFILLYLLP  LFIISVAYAR  VAKKLWLCNT  IGDVTTEQYL  ALRRKKKTTV   200

                       210         220         230         240         250
p63A2  201  KMIMLVVVII  ..........  ..........  ..........  ..........   250
P30731 201  KMLVLVVVII  ..........  ..........  ..........  ..........   250
```

Fig. 6

Fig. 7

610 bp —

M   1   2   3   4   5   6   7   8   9   M

M ; λDNA /Sty I  marker
lane 1 ; 63U~63L
lane 2 ; U1~63U
lane 3 ; U1~63L
lane 4 ; U2~63U
lane 5 ; U2~63L
lane 6 ; L1~63U
lane 7 ; L1~63L
lane 8 ; L2~63U
lane 9 ; L2~63L

Fig. 8

610 bp →

← 670 bp

← 1200 bp

1  2  3  4  5  6  7  8  9

lane 1 ; 63U~63L
lane 2 ; U1~63U
lane 3 ; U1~63L
lane 4 ; U2~63U
lane 5 ; U2~63L
lane 6 ; L1~63U
lane 7 ; L1~63L
lane 8 ; L2~63U
lane 9 ; L2~63L

Fig. 9

```
                  10        20        30        40        50
63A2-5'.SEQ   GGGCCCCCCTTACACCCTTTGTGATTGAGATCCGGGGTTTC-AAGGGGTGCATGATGAAG
              :::::  ::  :    :::   ::    :      ::. ::  :   :   : ::::     :
MUSGIR.DNA    GGGCTTCCTCTGTGCCCCGTGCCCCTCGCTCCCAGGCTCCCTCTGTGGTGTGGACTCCTC
              130       140       150       160       170       180
              60        70        80        90        100       110
63A2-5'.SEQ   GAGTAAGCCACAATGAGCAGGGCTTTCACCGTGGGGTTCTGGGACTCAGCGCCGTAGCGC
              ::   :   :   :  :::    ::   ::::    :   :  :  :: ::   ::: :  :  ::  :
MUSGIR.DNA    TAGCCCGGTGCGCTCAGC--CCCTCGCACC-CAGCCTCCAGGCACAGAGCCCGGCAGGGA
              190       200       210       220       230       240
              120       130       140       150       160       170
63A2-5'.SEQ   TTCCTGCCCACAAAGTTCTCCCAGGGGAGGGGTGGCTCCTGCAAAATGGTCCCTCACCTC
              :  ::::        :     :: :: ::  :   :::::::      ::   : :::  ::::      ::
MUSGIR.DNA    GCTCAGCCC-----TTGTGCCTAGAGCTGCAGTGGCT-GGACATGAAGGTTCCTCCTGTC
                  250       260       270       280        290 ── mouse GIR
                                                                  initiation codon
              180       190       200       210       220       230
63A2-5'.SEQ   TTGCTGCTCTGTCTCCTCCCCTTGGTGCGAGCCACCGAGCCCCACGAGGGCCGGGCCGAC
              ::::  ::::  :::  ::    :::   ::::::::: ::  ::::   :     :::  :       :  ::
MUSGIR.DNA    CTGCTTCTCTTTCTTCTGTCCTCAGTGCGAGCTACTGAGCAACCGCAGGTCGTCACTGAG
              300       310       320       330       340       350

              240       250       260       270       280       290
63A2-5'.SEQ   GAGCAGAGCGCGGAGGCGGCCCTGGCCGTGCCCAATGCCTCGCACTTCTTCTCTTGGAAC
              :  :   :::     :::::::  :::::::  :::  ::::::  :::::      ::::       ::: X
MUSGIR.DNA    CATCCCAGCATGGAGGCAGCCCTGACCGGGCCCAACGCCTCCTCGCACTTC---TGGGCC
              360       370       380       390       400       410
              300       310       320       330       340       350
63A2-5'.SEQ   AACTACACCTTCTCCGACTGGCAGAACTTTGTGGGCAGGAGGTGCTACGGCGCTGAGTCC
              :::::::  ::::::::::::::::  :::::::::::::   :  :: :: ::  :::::::
MUSGIR.DNA    AACTACACTTTCTCTGACTGGCAGAACTTCGTGGGCAGGAGACGTTATGGGGCCGAGTCC
                  420       430       440       450       460       470
              360       370       380
63A2-5'.SEQ   CAGAACCCCACGGTGAAAGCCCTGCTC
              :::::::::::::::::::::::: :::::X
MUSGIR.DNA    CAGAACCCCACGGTGAAAGCACTGCTC
                  480       490       500
```

48

Fig. 10

```
                 10        20        30        40        50        60
OR16-F.SEQ    TTGCCCCTCCYCATCATCTCTGTGGCCTACGCYCGTGTGGCCAARAAACTGTGGCTGTGT
              : ::  :::  . :::  :::::  ::::::::::  ::. :::::::::::::. ::  ::::::::: :::
MUSGIR.DNA    CTTCCACTCTTCATTATCTCAGTGGCCTATGCTCGTGTGGCCAAGAAGCTGTGGCTCTGT
              1030      1040      1050      1060      1070      1080
                 70        80        90       100       110
OR16-F.SEQ    AATATGATTGGCGATGTGACCACAGAGCAGTACTTTG-CCTKCGGCGCAAAAAGAAGAAG
              ::  :   :::::::::  :::::.:::::::::::::::::  :  : X::.. ::  ::::: :::::::
MUSGIR.DNA    AACACCATTGGCGACGTGACCACAGAGCAGTACCTCGCCCTGCGACGCAAGAAGAAGACC
              1090      1100      1110      1120      1130      1140
              120       130       140       150       160       170
OR16-F.SEQ    ACCATCAAGATGTTGATGCTGGTGGTAGTCCCCTTTGCCCTCCGCTGGTTCCCCCTCAAC
              ::: :  ::::::  ::  ::::  :::::::::::  ::::::::::: ::::::::::::  ::::::
MUSGIR.DNA    ACCGTGAAGATGCTGGTGCTTGTGGTAGTCCTCTTTGCCCTCTGCTGGTTCCCTCTCAAC
              1150      1160      1170      1180      1190      1200
              180       190       200       210
OR16-F.\SEQ   TGCTACGTCCTCCTCCTGTCCAGCAAGGTCATCCGC
              ::::: :::::::::: ::::::::::::: ::::X :
MUSGIR.DNA    TGCTATGTCCTCCTCTTGTCCAGCAAGGCCATCCAC
              1210      1220      1230      1240
```

EP 0 789 076 A2

Fig. 11

DNA fragment (670 bp)

63U

EcoRI

ATG                                                    Stop          EcoRI

λgt11 right arm | 5' non-      p63A2          3' non-coding          U1      U2
                  coding region                region
L2    L1                                                          λgt11 left arm

63L

DNA fragment (1200 bp)

Fig. 12

M1 M2 1 2 3 4 5 6 M2 M1

M1 ; φX174 / HincII
M2 ; λ / StyI
lane 1 ; 63U~Anchor Primer ⎤
lane 2 ; 63-6~Anchor Primer ⎬ Primary PCR
lane 3 ; 63-7~Anchor Primer ⎦
lane 4 ; 63-6~Anchor Primer ⎤
lane 5 ; 63-7~Anchor Primer ⎬ Secondary PCR
lane 6 ; 63-8~Anchor Primer ⎦

Fig. 13

about 3Kb →

1   2   3   4   5   6

lane 1 ; 63U~Anchor Primer ⎤
lane 2 ; 63-6~Anchor Primer ⎬ Primary PCR
lane 3 ; 63-7~Anchor Primer ⎦
lane 4 ; 63-6~Anchor Primer ⎤
lane 5 ; 63-7~Anchor Primer ⎬ Secondary PCR
lane 6 ; 63-8~Anchor Primer ⎦

Fig. 14

```
                  10        20        30        40        50        60
63A2-3'.seq    CCCTCTGCTGGTTCCCCCTCAACTGCTACGTCCTCCTCCTGTCCAGCAAGGTCATCCGCA
               X::::::::::::::: ::::::::::: ::::::::: :::::::::::: ::::: ::
MUSGIR.DNA     CCCTCTGCTGGTTCCCTCTCAACTGCTATGTCCTCCTCTTGTCCAGCAAGGCCATCCACA
                1190      1200      1210      1220      1230      1240
                  70        80        90       100       110       120
63A2-3'.seq    CCAACAATGCCCTCTACTTTGCCTTCCACTGGTTTGCCATGAGCAGCACCTGCTATAACC
               :::::::::::::::::::::::::::::::::::::::::::::: :: :: :::::::
MUSGIR.DNA     CCAACAATGCCCTCTACTTTGCCTTCCACTGGTTTGCCATGAGCAGTACTTGTTATAACC
                1250      1260      1270      1280      1290      1300
                 130       140       150       160       170       180
63A2-3'.seq    CCTTCATATACTGCTGGCTGAACGAGAACTTCAGGATTGAGCTAAAGGCATTACTGAGCA
               :::::::: ::::::::::: :: ::::::::: ::: :::::::: :::::::: ::::::::
MUSGIR.DNA     CCTTCATCTACTGCTGGCTCAATGAGAACTTTAGGGTTGAGCTTAAGGCATTGCTGAGCA
                1310      1320      1330      1340      1350      1360
                 190       200       210       220       230       240
63A2-3'.seq    TGTGTCAAAGACCTCCCAAGCCTCAGGAGGACAGGCCACCCTCCCCAGTTCCTTCCTTCA
               :::: ::::: :: ::::::::: ::::: ::::::: :::::::::::::::::::::::::
MUSGIR.DNA     TGTGCCAAAGGCCACCCAAGCCGCAGGAAGACAGGCTACCCTCCCCAGTTCCTTCCTTCA
                1370      1380      1390      1400      1410      1420
                 250       260       270       280       290       300
63A2-3'.seq    GGGTGGCCTGGACAGAGAAGAATGATGGCCAGAGGGCTCCCCTTGCCAATAACCTCCTGC
               ::::::: ::::::::::::::   :::: : ::::::::::: ::  : ::: ::: : :::
MUSGIR.DNA     GGGTGGCATGGACAGAGAAGAGCCATGGTCGGAGGGCTCCACTACCTAATCACCACTTGC
                1430      1440      1450      1460      1470      1480


                 310       320       330       340       350       360
63A2-3'.seq    CCACCTCCCAACTCCAGTCTGGGAAGACAGACCTGTCATCTGTGGAACCCATTGTGACGA
               :: : ::::: ::::::::::::::::::: ::::::::::::: :::::: : ::::: ::
MUSGIR.DNA     CCTCTTCCCAGATCCAGTCTGGGAAGACAGATCTGTCATCTGTGGAACCCGTTGTGGCCA
                1490      1500      1510·     1520      1530      1540
                 370       380       390       400       410
63A2-3'.seq    TGAGTCTAGAAGAGGGTTGGGAAGAGGGAGTGGGAGGGGTCTGT-CTC-CAC-CTGAGGCAG
               :::::::: :     : : :::::  :: : ::::::: ::: : ::: ::: : :  :::
MUSGIR.DNA     TGAGTCAGGGAAAGCT-GGAAGTTGGTGGGGGAGGGTTCTTTCCTCTCACAATTGACCAG
mouse GIR stop codon    1550 ▲     1560      1570      1580      1590      1600
                 420       430       440       450       460       470
63A2-3'.seq    GGA--AAGAGAG-CCTATTCTCACACATGATC-TTCAGAGTGCTGGAAACACACTCCTGC
               : :: ::::: :      :  : :::::  : :  : ::: :::::  :          v
MUSGIR.DNA     ACACTAACAGAGTTGGAAAGTAACACAGAAGCAGTGAGA-TGCTTGGGTTCCTAGGAACC
                1610      1620      1630      1640      1650      1660
                 480       490
63A2-3'.seq    AGAAGCTGTAGGACTCTTGAAT

MUSGIR.DNA     TGTCCAGCCCCATCTGATTTGC
                1670      1680
```

Fig. 15

1.34 kb

M ; λ / StyI
lane 1 ; Whole Brain

Fig. 16

```
          9           18           27           36           45           54
5'  ATG GTC CCT CAC CTC TTG CTG CTC TGT CTC CTC CCC TTG GTG CGA GCC ACC GAG
    --- --- --- --- --- --- --- --- --- --- --- --- --- --- --- --- --- ---
    Met Val Pro His Leu Leu Leu Leu Cys Leu Leu Pro Leu Val Arg Ala Thr Glu


          63           72           81           90           99          108
    CCC CAC GAG GGC CGG GCC GAC GAG CAG AGC GCG GAG GCG GCC CTG GCC GTG CCC
    --- --- --- --- --- --- --- --- --- --- --- --- --- --- --- --- --- ---
    Pro His Glu Gly Arg Ala Asp Glu Gln Ser Ala Glu Ala Ala Leu Ala Val Pro


         117          126          135          144          153          162
    AAT GCC TCG CAC TTC TTC TCT TGG AAC AAC TAC ACC TTC TCC GAC TGG CAG AAC
    --- --- --- --- --- --- --- --- --- --- --- --- --- --- --- --- --- ---
    Asn Ala Ser His Phe Phe Ser Trp Asn Asn Tyr Thr Phe Ser Asp Trp Gln Asn


         171          180          189          198          207          216
    TTT GTG GGC AGG AGG CGC TAC GGC GCT GAG TCC CAG AAC CCC ACG GTG AAA GCC
    --- --- --- --- --- --- --- --- --- --- --- --- --- --- --- --- --- ---
    Phe Val Gly Arg Arg Arg Tyr Gly Ala Glu Ser Gln Asn Pro Thr Val Lys Ala


         225          234          243          252          261          270
    CTG CTC ATT GTG GCT TAC TCC TTC ATC ATT GTC TTC TCA CTC TTT GGC AAC GTC
    --- --- --- --- --- --- --- --- --- --- --- --- --- --- --- --- --- ---
    Leu Leu Ile Val Ala Tyr Ser Phe Ile Ile Val Phe Ser Leu Phe Gly Asn Val


         279          288          297          306          315          324
    CTG GTC TGT CAT GTC ATC TTC AAG AAC CAG CGA ATG CAC TCG GCC ACC AGC CTC
    --- --- --- --- --- --- --- --- --- --- --- --- --- --- --- --- --- ---
    Leu Val Cys His Val Ile Phe Lys Asn Gln Arg Met His Ser Ala Thr Ser Leu


         333          342          351          360          369          378
    TTC ATC GTC AAC CTG GCA GTT GCC GAC ATA ATG ATC ACG CTG CTC AAC ACC CCC
    --- --- --- --- --- --- --- --- --- --- --- --- --- --- --- --- --- ---
    Phe Ile Val Asn Leu Ala Val Ala Asp Ile Met Ile Thr Leu Leu Asn Thr Pro


         387          396          405          414          423          432
    TTC ACT TTG GTT CGC TTT GTG AAC AGC ACA TGG ATA TTT GGG AAG GGC ATG TGC
    --- --- --- --- --- --- --- --- --- --- --- --- --- --- --- --- --- ---
    Phe Thr Leu Val Arg Phe Val Asn Ser Thr Trp Ile Phe Gly Lys Gly Met Cys


         441          450          459          468          477          486
    CAT GTC AGC CGC TTT GCC CAG TAC TGC TCA CTG CAC GTC TCA GCA CTG ACA CTG
    --- --- --- --- --- --- --- --- --- --- --- --- --- --- --- --- --- ---
    His Val Ser Arg Phe Ala Gln Tyr Cys Ser Leu His Val Ser Ala Leu Thr Leu


         495          504          513          522          531          540
    ACA GCC ATT GCG GTG GAT CGC CAC CAG GTC ATC ATG CAC CCC TTG AAA CCC CGG
    --- --- --- --- --- --- --- --- --- --- --- --- --- --- --- --- --- ---
    Thr Ala Ile Ala Val Asp Arg His Gln Val Ile Met His Pro Leu Lys Pro Arg


         549          558          567          576          585          594
    ATC TCA ATC ACA AAG GGT GTC ATC TAC ATC GCT GTC ATC TGG ACC ATG GCT ACG
    --- --- --- --- --- --- --- --- --- --- --- --- --- --- --- --- --- ---
    Ile Ser Ile Thr Lys Gly Val Ile Tyr Ile Ala Val Ile Trp Thr Met Ala Thr


         603          612          621          630          639          648
    TTC TTT TCA CTC CCA CAT GCT ATC TGC CAG AAA TTA TTT ACC TTC AAA TAC AGT
    --- --- --- --- --- --- --- --- --- --- --- --- --- --- --- --- --- ---
    Phe Phe Ser Leu Pro His Ala Ile Cys Gln Lys Leu Phe Thr Phe Lys Tyr Ser


         657          666          675          684          693          702
    GAG GAC ATT GTG CGC TCC CTC TGC CTG CCA GAC TTC CCT GAG CCA GCT GAC CTC
    --- --- --- --- --- --- --- --- --- --- --- --- --- --- --- --- --- ---
    Glu Asp Ile Val Arg Ser Leu Cys Leu Pro Asp Phe Pro Glu Pro Ala Asp Leu
```

Fig. 17

```
        711             720             729             738             747             756
TTC TGG AAG TAC CTG GAC TTG GCC ACC TTC ATC CTG CTC TAC ATC CTG CCC CTC
--- --- --- --- --- --- --- --- --- --- --- --- --- --- --- --- --- ---
Phe Trp Lys Tyr Leu Asp Leu Ala Thr Phe Ile Leu Leu Tyr Ile Leu Pro Leu

        765             774             783             792             801             810
CTC ATC ATC TCT GTG GCC TAC GCT CGT GTG GCC AAG AAA CTG TGG CTG TGT AAT
--- --- --- --- --- --- --- --- --- --- --- --- --- --- --- --- --- ---
Leu Ile Ile Ser Val Ala Tyr Ala Arg Val Ala Lys Lys Leu Trp Leu Cys Asn

        819             828             837             846             855             864
ATG ATT GGC GAT GTG ACC ACA GAG CAG TAC TTT GCC CTG CGG CGC AAA AAG AAG
--- --- --- --- --- --- --- --- --- --- --- --- --- --- --- --- --- ---
Met Ile Gly Asp Val Thr Thr Glu Gln Tyr Phe Ala Leu Arg Arg Lys Lys Lys

        873             882             891             900             909             918
AAG ACC ATC AAG ATG TTG ATG CTG GTG GTA GTC CTC TTT GCC CTC TGC TGG TTC
--- --- --- --- --- --- --- --- --- --- --- --- --- --- --- --- --- ---
Lys Thr Ile Lys Met Leu Met Leu Val Val Val Leu Phe Ala Leu Cys Trp Phe

        927             936             945             954             963             972
CCC CTC AAC TGC TAC GTC CTC CTC CTG TCC AGC AAG GTC ATC CGC ACC AAC AAT
--- --- --- --- --- --- --- --- --- --- --- --- --- --- --- --- --- ---
Pro Leu Asn Cys Tyr Val Leu Leu Leu Ser Ser Lys Val Ile Arg Thr Asn Asn

        981             990             999            1008            1017            1026
GCC CTC TAC TTT GCC TTC CAC TGG TTT GCC ATG AGC AGC ACC TGC TAT AAC CCC
--- --- --- --- --- --- --- --- --- --- --- --- --- --- --- --- --- ---
Ala Leu Tyr Phe Ala Phe His Trp Phe Ala Met Ser Ser Thr Cys Tyr Asn Pro

       1035            1044            1053            1062            1071            1080
TTC ATA TAC TGC TGG CTG AAC GAG AAC TTC AGG ATT GAG CTA AAG GCA TTA CTG
--- --- --- --- --- --- --- --- --- --- --- --- --- --- --- --- --- ---
Phe Ile Tyr Cys Trp Leu Asn Glu Asn Phe Arg Ile Glu Leu Lys Ala Leu Leu

       1089            1098            1107            1116            1125            1134
AGC ATG TGT CAA AGA CCT CCC AAG CCT CAG GAG GAC AGG CCA CCC TCC CCA GTT
--- --- --- --- --- --- --- --- --- --- --- --- --- --- --- --- --- ---
Ser Met Cys Gln Arg Pro Pro Lys Pro Gln Glu Asp Arg Pro Pro Ser Pro Val

       1143            1152            1161            1170            1179            1188
CCT TCC TTC AGG GTG GCC TGG ACA GAG AAG AAT GAT GGC CAG AGG GCT CCC CTT
--- --- --- --- --- --- --- --- --- --- --- --- --- --- --- --- --- ---
Pro Ser Phe Arg Val Ala Trp Thr Glu Lys Asn Asp Gly Gln Arg Ala Pro Leu

       1197            1206            1215            1224            1233            1242
GCC AAT AAC CTC CTG CCC ACC TCC CAA CTC CAG TCT GGG AAG ACA GAC CTG TCA
--- --- --- --- --- --- --- --- --- --- --- --- --- --- --- --- --- ---
Ala Asn Asn Leu Leu Pro Thr Ser Gln Leu Gln Ser Gly Lys Thr Asp Leu Ser

       1251            1260            1269
TCT GTG GAA CCC ATT GTG ACG ATG AGT TAG 3'
--- --- --- --- --- --- --- --- --- ---
Ser Val Glu Pro Ile Val Thr Met Ser ***
```

Fig. 18

The position in the amino acid sequence

Fig. 19

```
                        10        20        30        40        50
63A2.AMI        1  M-VPHLLLLC LLPLVRATEP HEGRADEQSA EAALAVPNAS HFFSWNNYTF    50
MUSGIR.AMI      1  MKVPPVLLLF LLSSVRATEQ PQVVTEHPSM EAALTGPNAS SHF-WANYTF    50

                        60        70        80        90        100
63A2.AMI       51  SDWQNFVGRR RYGAESQNPT VKALLIVAYS FIIVFSLFGN VLVCHVIFKN   100
MUSGIR.AMI     51  SDWQNFVGRR RYGAESQNPT VKALLIVAYS FTIVFSLFGN VLVCHVIFKN   100

                        110       120       130       140       150
63A2.AMI      101  QRMHSATSLF IVNLAVADIM ITLLNTPFTL VRFVNSTWIF GKGMCHVSRF   150
MUSGIR.AMI    101  QRMHSATSLF IVNLAVADIM ITLLNTPFTL VRFVNSTWVF GKGMCHVSRF   150

                        160       170       180       190       200
63A2.AMI      151  AQYCSLHVSA LTLTAIAVDR HQVIMHPLKP RISITKGVIY IAVIWTMATF   200
MUSGIR.AMI    151  AQYCSLHVSA LTLTAIAVDR HQVIMHPLKP RISITKGVIY IAVIWVMATF   200

                        210       220       230       240       250
63A2.AMI      201  FSLPHAICQK LFTFKYSEDI VRSLCLPDFP EPADLFWKYL DLATFILLYI   250
MUSGIR.AMI    201  FSLPHAICQK LFTFKYSEDI VRSLCLPDFP EPADLFWKYL DLATFILLYL   250

                        260       270       280       290       300
63A2.AMI      251  LPLLIISVAY ARVAKKLWLC NMIGDVTTEQ YFALRRKKKK TIKMLMLVVV   300
MUSGIR.AMI    251  LPLFIISVAY ARVAKKLWLC NTIGDVTTEQ YIALRRKKKT TVKMLVLVVV   300

                        310       320       330       340       350
63A2.AMI      301  LFALCWFPLN CYVLLLSSKV IRTNNALYFA FHWFAMSSTC YNPFIYCWLN   350
MUSGIR.AMI    301  LFALCWFPLN CYVLLLSSKA IHTNNALYFA FHWFAMSSTC YNPFIYCWLN   350

                        360       370       380       390       400
63A2.AMI      351  ENFRIELKAL LSMCQRPPKP QEDRPPSPVP SFRVAWTEKN DGDRAPLANN   400
MUSGIR.AMI    351  ENFRVELKAL LSMCQRPPKP QEDRIPSPVP SFRVAWTEKS HGRRAPLPNH   400

                        410       420       430       440       450
63A2.AMI      401  LLPTSQLQSG KTDLSSVEPI VTVS*..... .......... ..........   450
MUSGIR.AMI    401  HLPSSQIQSG KTDLSSVEPV VAVS*..... .......... ..........   450
```